# EUROPEAN PATENT APPLICATION

(11) **EP 4 696 690 A1**
(43) Date of publication of application: **18.02.2026**
(21) Application number: 24195055.9
(22) Date of filing: 16.08.2024
(51) Int. Cl.: C07D 487/10, C07D 498/10, A61P 3/00, A61P 25/00, A61P 29/00, A61P 35/00, A61K 31/496

(54) **SPIROCYCLIC MAGL INHIBITORS**

(71) Applicant: Universiteit Leiden, 2311 EZ Leiden (NL)
(72) Inventor: VAN DER STELT, Marcelis, 2311 EZ Leiden (NL); HUIZENGA, Mirjam Cornelia Wellemina, 2311 EZ Leiden (NL); JANSSEN, Antonius Petrus Arnoldus, 2311 EZ Leiden (NL); VAN DEN BERG, Richard Jan Baptist Hendrikus Nouel, 2311 EZ Leiden (NL)
(74) Representative: HGF

(57) **Abstract**

This invention relates to compound of formula I that are useful as inhibitors, in particular as inhibitors of monoacylglycerol lipase (MAGL). The invention also relates to uses of said compounds.

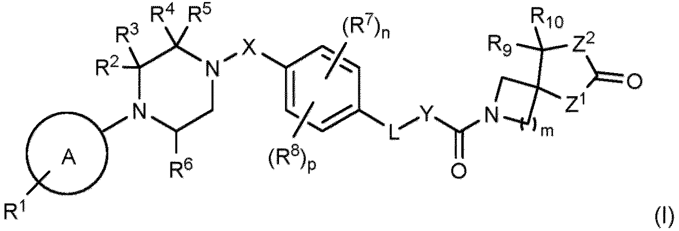

## Description

This invention relates to compounds that are useful as inhibitors, in particular as inhibitors of monoacylglycerol lipase (MAGL).

### BACKGROUND

Cancers represent the second leading cause of death internationally. The rewiring of energy metabolism is of crucial importance to cancer cells. Deregulated metabolic pathways, resulting in a lipogenic phenotype, enable cancer cells to promote their survival, migration and invasion. Not only the de novo fatty acid synthesis, but also the hydrolytic generation of free fatty acids from stored (phospho)lipid precursors constitutes an important pathway of lipogenesis. Next to a metabolic role, these liberated fatty acids and their secondary metabolites (e.g. prostaglandins) serve also as oncogenic signalling purposes.

Monoacylglycerol lipase (MAGL) represents a key lipolytic enzyme in cancer cells that produces free fatty acids from lipid precursors. It has been suggested that inhibition of MAGL constitutes a novel therapeutic avenue to treat cancer via interfering with a fatty acid network that promotes cancer pathogenesis (DK Nomura et al. Monoacylglycerol Lipase Regulates a Fatty Acid Network that Promotes Cancer Pathogenesis. Cell. 2010, 140(1): 49 - 61; Pagano et al., Pharmacological inhibition of MAGL attenuates experimental colon carcinogenesis. Pharmacol Res., 2017, 5(119): 227-236). MAGL, a serine hydrolase, converts monoacylglycerols to free fatty acids, including arachidonic acid. MAGL is highly expressed in aggressive cancer cells and primary human tumors and its overexpression in nonaggressive tumor cells increases their pathogenicity. MAGL activity is co-opted by aggressive cancer cells and its elevated activity is associated with the translation of the lipogenic state of cancer cells into an array of protumorigenic signals, including LPA, LPC and PGE₂, that promote cancer cell migration, invasion and *in vivo* tumor growth (Nomura *et al.,* 2010, *supra*)*.* Inhibition of MAGL activity, by RNA interference technology or pharmacological inhibition, resulted in a reduction of free fatty acid levels and impaired cancer pathogenicity in a multitude of *in vitro* and *in vivo* models (Nomura *et al.,* 2010, *supra*)*.* MAGL may also be implicated in other conditions, for example, in Parkinson's disease, Alzheimer's diseases, multiple sclerosis, inflammatory and neuropathic pain, acute liver injury, anxiety and depression.

WO 2021/175913, Jiang et al Nature Comm, 2023 and Jiang et al. J. Med. Chem. 2024, relate to MAGL inhibitors in the treatment of cancer and other indications, such as chemotherapy-induce neuropathy.

Therapeutic candidate compounds should have high cellular target engagement as well as high lipophilic efficiency (LipE), which is defined as the pIC₅₀ or pKᵢ value minus the logP value. A high LipE is associated with favourable adsorption, distribution, metabolism and excretion (ADME) drug properties.

Accordingly, there is a general need to develop improved MAGL inhibitors. For example, there is a need to develop MAGL inhibitors having improved physicochemical properties, e.g. improved cellular activity and/or improved lipophilic efficiency (LipE).

An object of the invention is therefore to provide compounds that are useful as improved MAGL inhibitors. Another object of the invention is to provide compounds that are useful in the treatment of cancer. A further object of the invention is to provide compounds that are useful in the treatment of pathologies that are amenable to treatment with MAGL inhibitors.

### SUMMARY OF THE DISCLOSURE

The invention provides compounds that are useful as inhibitors of monoacylglycerol lipase (MAGL).

In a first aspect of the present invention, there is provided a compound of formula (I), or a pharmaceutically acceptable salt or solvate thereof: 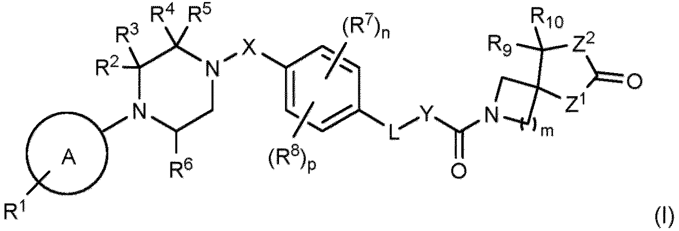 wherein
A is a 6-membered aromatic ring, or a 5- or 6-membered heteroaromatic ring optionally comprising at least one N atom, wherein the 6-membered aromatic ring of the 5- or 6-membered heteroaromatic ring is optionally fused to a 5- or -6 membered heterocycloalkyl ring or a C₅ or C₆ cycloalkyl;
X is selected from -C(O)- and -C(R^{a}R^{b})-;
L is selected from S(=O)-, -SO₂-, -S-, -O-, -C(O)-, -CH₂-, -C(R^{c}R^{d})-;
Y is -CH₂-, -CF₂-, -C(R^{c}R^{d})-;
Z¹ is -NR^{e}-;
Z² is selected from -CH₂-, -O-CH₂-, and -O-;
R¹ is selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl;
each R² and R⁴ is independently selected from H and C₁₋₄ alkyl;
each R³, R⁵ and R⁶ is independently selected from H and C₁₋₄ alkyl; or R³ and R⁶ together form an alkyl or heteroalkyl bridge and R⁵ is selected from H and C₁₋₄ alkyl;
each R⁷ and R⁸ is independently selected from H, halo, C₁₋₄ alkyl, C₁₋₄ haloalkyl, C₁₋₄ alkoxy, CN and NO₂;
R⁹ is selected from H, C₁₋₆ haloalkyl, OR¹¹, C₁₋₆ alkyl, and C₂₋₈ alkyl interrupted by 1, 2 or 3 ether linkages, -(C₂₋₄ haloalkyl)-OR¹¹, and -(C₁₋₆ haloalkyl)-R¹¹;
R¹⁰ is selected from H and C₁₋₆ alkyl;
R¹¹ is selected from C₁₋₆ alkyl, C₂₋₈ alkyl interrupted by 1, 2 or 3 ether linkages, C₃₋₈ cycloalkyl, C₆ aryl, or heterocyclyl;
wherein each R⁹, R¹⁰ and R¹¹ is optionally independently substituted where chemically possible with one, two or three groups independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl;
R^{a} and R^{b} are independently selected from H, and C₁₋₄ alkyl; or wherein R^{a} and R^{b} together form a 3- to 6-membered cycloalkyl or heterocycloalkyl ring system;
R^{c} and R^{d} are independently selected at each occurrence from H, F, C₁₋₄ alkyl, and C₁₋₄ haloalkyl; or R^{c} and R^{d} together form a 3- to 6-membered cycloalkyl or heterocycloalkyl ring system;
R^{e} is selected from H and C₁₋₄ alkyl;
m is selected from 1, or 2;
n is selected from 0, 1, or 2; and
p is selected from 0, 1, or 2.

In a second aspect of the present invention, there is provided a pharmaceutical composition comprising a compound of the first aspect.

In a third aspect of the present invention, there is provided the compound of the first aspect or the pharmaceutical composition of the second aspect for use as a medicament.

In a fourth aspect of the present invention, there is provided the compound of the first aspect or the pharmaceutical composition of the second aspect for use in the treatment of a condition which is modulated by monoacylglycerol lipase (MAGL).

In a fifth aspect of the present invention, there is provided the compound of the first aspect or the pharmaceutical composition of the second aspect for use in the treatment of a condition selected from acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

In a sixth aspect of the present invention, there is provided a use of the compound of the first aspect or the pharmaceutical composition of the second aspect for the inhibition of monoacylglycerol lipase (MAGL).

In a seventh aspect of the present invention, there is provided a use of the compound of the first aspect in the manufacture of a medicament for use in the treatment of a condition selected from: acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

### BRIEF DESCRIPTION OF THE DRAWINGS

Embodiments of the invention are further described hereinafter with reference to the accompanying drawings, in which:

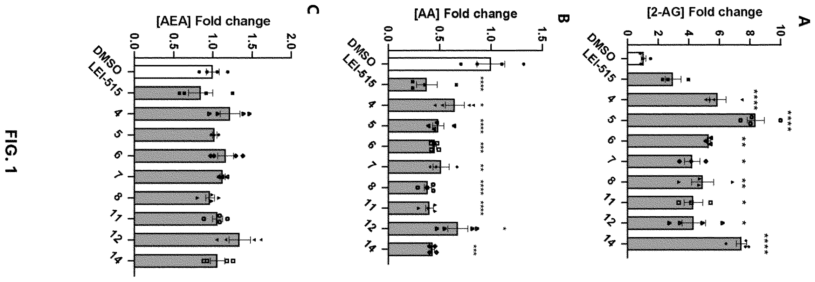

Figure 1 shows the effect of compounds of invention on the levels of (A) 2-AG, (B) arachidonic acid (AA), and (c) anandamide (AEA) in U-87 MG cells (1 µM, 1 h). Statistical analysis: one-way ANOVA with Tukey's multiple comparison test. **** = p < 0.0001, *** = p < 0.001, ** = p < 0.01, * = p < 0.05 vs DMSO, n = 4.

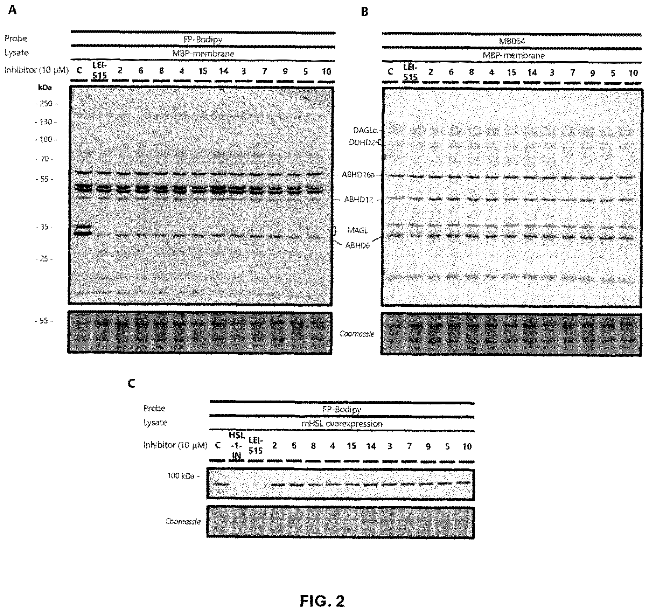

Figure 2 shows gel based competitive ABPP of compounds of the invention in mouse brain membrane with serine hydrolase ABP FP-Bodipy (A) or MB064 (B). (C) Gel based competitive ABPP in mHSL overexpression lysate using FP-Bodipy. C = DMSO control, HSL-1-IN = HSL inhibitor.

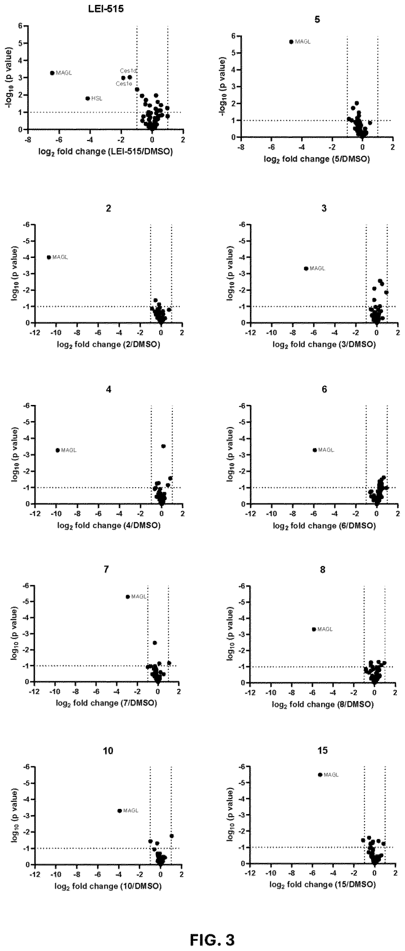

Figure 3 shows the chemical proteomics selectivity profiles of LEI-515 and compounds of the invention (1 µM, 30 min, 37°C) on mouse liver proteome using a probe cocktail of MB108 and FP-Biotin (2.5 µM and 5 µM respectively, 30 minutes, 37 °C). Data represented as a volcano plot with cut-off values: unique peptides ≥2; -1≤ log2(fold change) ≥1; p <0.05 using multiple unpaired t-test with FOR 10% (n=3).

### DETAILED DESCRIPTION

The abbreviations used herein have their conventional meaning within the chemical and biological arts.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

For the avoidance of doubt, it is hereby stated that the information disclosed earlier in this specification under the heading "Background" is relevant to the invention and is to be read as part of the disclosure of the invention.

All publications, patent applications, patents, and other references mentioned herein are incorporated by reference in their entirety. In case of conflict, the present specification, including definitions, will control.

### DEFINITIONS

The following explanations of terms and methods are provided to better describe the present disclosure and to guide those of ordinary skill in the art in the practice of the present disclosure.

The invention concerns amongst other things the treatment of a disease. The term "treatment", and the therapies encompassed by this invention, include the following and combinations thereof: (1) hindering, e.g. delaying initiation and/or progression of, an event, state, disorder or condition, for example arresting, reducing or delaying the development of the event, state, disorder or condition, or a relapse thereof in case of maintenance treatment or secondary prophylaxis, or of at least one clinical or subclinical symptom thereof; (2) preventing or delaying the appearance of clinical symptoms of an event, state, disorder or condition developing in an animal (e.g. human) that may be afflicted with or predisposed to the state, disorder or condition but does not yet experience or display clinical or subclinical symptoms of the state, disorder or condition; and/or (3) relieving and/or curing an event, state, disorder or condition (e.g., causing regression of the event, state, disorder or condition or at least one of its clinical or subclinical symptoms, curing a patient or putting a patient into remission). The benefit to a patient to be treated may be either statistically significant or at least perceptible to the patient or to the physician. It will be understood that a medicament will not necessarily produce a clinical effect in each patient to whom it is administered; thus, in any individual patient or even in a particular patient population, a treatment may fail or be successful only in part, and the meanings of the terms "treatment", "prophylaxis" and "inhibitor" and of cognate terms are to be understood accordingly. The compositions and methods described herein are of use for therapy and/or prophylaxis of the mentioned conditions.

The term "prophylaxis" includes reference to treatment therapies for the purpose of preserving health or inhibiting or delaying the initiation and/or progression of an event, state, disorder or condition, for example for the purpose of reducing the chance of an event, state, disorder or condition occurring. The outcome of the prophylaxis may be, for example, preservation of health or delaying the initiation and/or progression of an event, state, disorder or condition. It will be recalled that, in any individual patient or even in a particular patient population, a treatment may fail, and this paragraph is to be understood accordingly.

The term "inhibit" (and "inhibiting") includes reference to delaying, stopping, reducing the incidence of, reducing the risk of and/or reducing the severity of an event, state, disorder or condition. Inhibiting an event, state, disorder or condition may therefore include delaying or stopping initiation and/or progression of such, and reducing the risk of such occurring. For example, the compounds of the invention may inhibit MAGL and therefore may inhibit an event, state, disorder or condition for which MAGL is a therapeutic target.

An "inhibitor" is a molecule that binds to an enzyme and decreases its activity. An "irreversible inhibitor" is an inhibitor where the binding involves a chemical reaction, e.g. formation of a covalent bond between the molecule and enzyme. A "reversible inhibitor" is an inhibitor where binding involves non-covalent interactions such as ionic bonds, hydrogen bonds, and hydrophobic interactions. The compounds of the invention may act as inhibitors of MAGL.

The term "alkyl" as used herein include reference to a straight or branched chain alkyl moiety having 1, 2, 3, 4, 5, 6, 7, 8, 9 or 10 carbon atoms. The term includes reference to, for example, methyl, ethyl, propyl (n-propyl or isopropyl), butyl (n-butyl, sec-butyl or tert-butyl), pentyl, hexyl and the like. In particular, alkyl may be a "C₁-C₄ alkyl", i.e. an alkyl having 1, 2, 3 or 4 carbon atoms; or a "C₁-C₆ alkyl", i.e. an alkyl having 1, 2, 3, 4, 5 or 6 carbon atoms; or a "C₁-C₃ alkyl", i.e. an alkyl having 1, 2 or 3 carbon atoms. The term "lower alkyl" includes reference to alkyl groups having 1, 2, 3 or 4 carbon atoms.

The term "cycloalkyl" as used herein includes reference to an alicyclic moiety having 3, 4, 5 or 6 carbon atoms. The group may be a bridged or polycyclic ring system. More often cycloalkyl groups are monocyclic. This term includes reference to groups such as cyclopropyl, cyclobutyl, cyclopentyl, cyclohexyl and the like.

The term "heterocycloalkyl" as used herein includes reference to a saturated heterocyclic moiety having 3, 4, 5, 6 or 7 ring carbon atoms and 1, 2, 3, 4 or 5 ring heteroatoms selected from nitrogen, oxygen, phosphorus and sulphur. For example, a heterocycolalkyl may comprise 3, 4, or 5 ring carbon atoms and 1 or 2 ring heteroatoms selected from nitrogen and oxygen. The group may be a polycyclic ring system but more often is monocyclic. This term includes reference to groups such as azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolizidinyl and the like.

The terms "halo" or "halogen" as used herein includes reference to F, Cl, Br or I, for example F, Cl or Br. In a particular class of embodiments, halogen is F or CI, of which F is more common.

The term "haloalkyl" refers to an alkyl group where one or more hydrogen atoms are substituted by a corresponding number of halogens. An exemplary haloalkyl group is trifluoromethyl. Another exemplary haloalkyl group is 2-fluoro-2-methylpropyl.

The term "alkoxy" as used herein include reference to -O-alkyl, wherein alkyl is straight or branched chain and comprises 1, 2, 3, 4, 5 or 6 carbon atoms. In one class of embodiments, alkoxy has 1, 2, 3 or 4 carbon atoms, e.g. 1, 2 or 3 carbon atoms. This term includes reference to, for example, methoxy, ethoxy, propoxy, isopropoxy, butoxy, tert-butoxy, pentoxy, hexoxy and the like. The term "lower alkoxy" includes reference to alkoxy groups having 1, 2, 3 or 4 carbon atoms.

The term "substituted" as used herein in reference to a moiety means that one or more, especially up to 5, more especially 1, 2 or 3, of the hydrogen atoms in said moiety are replaced independently of each other by the corresponding number of the described substituents. Unless otherwise specified, exemplary substituents include -OH, -CN, -NH₂, =O, -halo, -C₁-C₆ alkyl, -C₂-C₆ alkenyl, -C₁-C₆ haloalkyl, -C₁-C₆ haloalkoxy and-C₂-C₆ haloalkenyl, -C₁-C₆ alkylcarboxylic acid (e.g. -CH₃COOH or -COOH). Where the substituent is a -C₁-C₆ alkyl or -C₁-C₆ haloalkyl, the C₁-C₆ chain is optionally interrupted by an ether linkage (-O-) or an ester linkage (-C(O)O-). Exemplary substituents for a substituted alkyl may include -OH, -CN, -NH₂, =O, -halo, -CO₂H, -C₁-C₆ haloalkyl, -C₁-C₆ haloalkoxy and-C₂-C₆haloalkenyl, -C₁-C₆ alkylcarboxylic acid (e.g. -CH₃COOH or -COOH). For example, exemplary substituents for an alkyl may include -OH, -CN, -NH₂, =O, -halo.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

Where steric issues determine placement of substituents on a group, the isomer having the lowest conformational energy may be preferred.

Where a compound, moiety, process or product is described as "optionally" having a feature, the disclosure includes such a compound, moiety, process or product having that feature and also such a compound, moiety, process or product not having that feature. Thus, when a moiety is described as "optionally substituted", the disclosure comprises the unsubstituted moiety and the substituted moiety.

Where two or more moieties are described as being "independently" or "each independently" selected from a list of atoms or groups, this means that the moieties may be the same or different. The identity of each moiety is therefore independent of the identities of the one or more other moieties.

The term "pharmaceutically acceptable" as used herein includes reference to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio. This term includes acceptability for both human and veterinary purposes.

The term "pharmaceutical composition" as used herein includes reference to a composition or formulation comprising at least one active compound and optionally one or more additional pharmaceutically acceptable ingredients, for example a pharmaceutically acceptable carrier. Unless the context indicates otherwise, all references to a "composition" herein are references to a pharmaceutical composition.

The term "product" or "product of the invention" as used herein includes reference to any product containing a compound of the present invention. In particular, the term product relates to compositions and formulations containing a compound of the present invention, such as a pharmaceutical composition, for example.

The term "therapeutically effective amount" as used herein refers to an amount of a drug, or pharmaceutical agent that, within the scope of sound pharmacological judgment, is calculated to (or will) provide a desired therapeutic response in a mammal (animal or human). The therapeutic response may for example serve to cure, delay the progression of or prevent a disease, disorder or condition.

The term "pharmaceutically acceptable salts" is meant to include salts of the active compounds which are prepared with relatively nontoxic acids or bases, depending on the particular substituents found on the compounds described herein. When compounds contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

Thus, the compounds of the present disclosure may exist as salts with pharmaceutically acceptable acids. The present invention includes such salts. Examples of such salts include hydrochlorides, hydrobromides, sulfates, methanesulfonates, nitrates, maleates, acetates, citrates, fumarates, tartrates (e.g. (+)-tartrates, (-)-tartrates or mixtures thereof including racemic mixtures, succinates, benzoates and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centres) or double bonds; the racemates, enantiomeric mixtures, diastereomers, diastereomeric mixtures, tautomers, geometric isomers and individual isomers are encompassed within the scope of the present invention. The compounds of the present invention do not include those which are known in the art to be too unstable to synthesize and/or isolate.

An enantiomer can be characterised by the absolute configuration of its asymmetric centre and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture". Where a compound of the invention has two or more stereo centres, any combination of (R) and (S) stereoisomers is contemplated. The combination of (R) and (S) stereoisomers may result in a diastereomeric mixture or a single diastereoisomer. The compounds of the invention may be present as a single stereoisomer or may be mixtures of stereoisomers, for example racemic mixtures and other enantiomeric mixtures, and diastereomeric mixtures.

Reference to an "enantiomeric excess" or "e.e." of a compound of the invention refers to an excess of a cis- or trans- enantiomer of in a mixture of the enantiomers. Where the compound of the invention exist as a mixture of enantiomers, either the cis- or trans-enantiomer may be present in an enantiomeric excess. Alternatively, the cis- and trans-enantiomers may be present in equal amounts, i.e. as a racemate (with an enantiomeric excess of 0%).

Where the compound is a single stereoisomer, the compounds may still contain other diasteroisomers or enantiomers as impurities. Hence a single stereoisomer does not necessarily have an enantiomeric excess (e.e.) or diastereomeric excess (d.e.) of 100% but could have an e.e. or d.e. of about at least 85%, for example at least 90%, at least 95%, at least 99%, or at least 99.9%.

The compounds of this invention may possess one or more asymmetric centres; such compounds can therefore be produced as individual (R) or (S) stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well known in the art (see discussion in Chapter 4 of "Advanced Organic Chemistry", 4th edition J. March, John Wiley and Sons, New York, 2001), for example by synthesis from optically active starting materials or by resolution of a racemic form. It is to be understood that the present invention encompasses all optical, diastereoisomers and geometric isomers and mixtures thereof.

The compounds of the invention may have geometric isomeric centres (cis- and trans - isomers). Thus, the compounds of the invention may be represented with relative cis- or trans- conformation, e.g. where two stereo centres are present on a ring. For example, where a compound has two stereo centres, one or more *straight, unwedged* solid or hashed bonds may be depicted at each stereo centre to illustrate relative cis- or trans-conformation. This is illustrated in the exemplary trans- compound of the invention below: 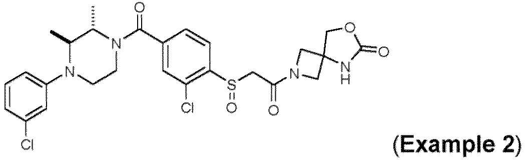 wherein the compound comprises a mixture (e.g. a racemic mixture, or other enantiomeric mixture) of the (R,R) and (S,S) trans-enantiomers. Accordingly, it is to be understood that the above trans- compound encompasses the individual trans- enantiomers. Similarly, in cases where the compound is a cis-compound, the compound comprises a mixture (e.g. a racemic mixture, or other enantiomeric mixture) of the (R,S) and (S,R) cis-enantiomers. Accordingly, the structure of such a cis- compound encompasses the individual cis-enantiomers. Alternatively, a "(±)" may also be used to indicate that a compound is represented by the above described relative cis- or trans- conformation.

Also disclosed herein and in the Examples are compounds in the absolute cis- or trans- conformation. Such compounds are shown with one or more *wedged* solid or hashed bonds at each stereo centre (and not *straight, unwedged* bonds), or are shown without a "(±)". For example, the structure of Example 2 above refers to a compound with relative trans- conformation (± 2-(2-((2-Chloro-4-(trans-4-(3-chlorophenyl)-2,3-dimethylpiperazine-1-carbonyl)phenyl)sulfinyl)acetyl)-7-oxa-2,5-diazaspiro[3.4]octan-6-one). However, the below compound refers to the (S,S)-trans enantiomer (2-(2-((2-chloro-4-((2S,3S)-4-(3-chlorophenyl)-2,3-dimethylpiperazine-1-carbonyl)phenyl)sulfinyl)acetyl)-7-oxa-2,5-diazaspiro[3.4]octan-6-one):

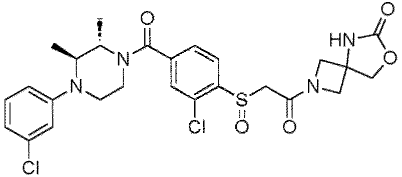

Z/E and cis/trans isomers may be separated by, for example, chromatography and fractional crystallisation.

Known techniques for the preparation/isolation of individual enantiomers when necessary include chiral synthesis from a suitable optically pure precursor or resolution of the racemate (e.g. the separation of the cis-enantiomers of the compound of formula (II), or the trans-enantiomers of the trans-compound of formula (III)) using, for example, chiral high-pressure liquid chromatography (HPLC).

Alternatively, the racemate (or a racemic precursor) may be reacted with a suitable optically active compound, for example, an alcohol, or, in the case where the compound of the invention contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

When any racemate crystallises, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

While both of the crystal forms present in a racemic mixture have identical physical properties, they may have different physical properties compared to the true racemate. Racemic mixtures may be separated by conventional techniques known to those skilled in the art - see, for example, "Stereochemistry of Organic Compounds" by E. L. Eliel and S. H. Wilen (Wiley, 1994).

The compounds of the present invention may also contain unnatural proportions of atomic isotopes at one or more of the atoms that constitute such compounds. For example, the compounds may be radiolabelled with radioactive isotopes, such as for example tritium (³H), iodine-125 (¹²⁵I) or carbon-14 (¹⁴C). All isotopic variations of the compounds of the present invention, whether radioactive or not, are encompassed within the scope of the present invention.

### COMPOUNDS

In one aspect, the invention provides compounds of formula (I) as previously described or a pharmaceutically acceptable salt, or solvate thereof.

In embodiments, the compound is a compound of formula (II) or a pharmaceutically acceptable salt or solvate thereof: 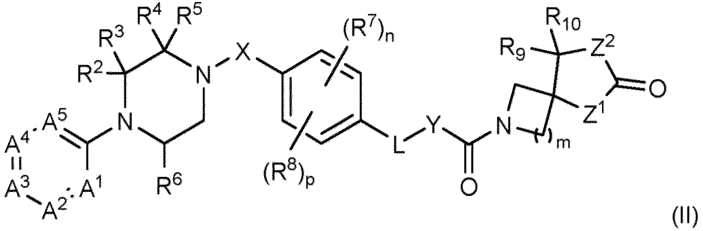 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, X, Y, Z¹, Z², R¹, R², R³, R⁴, R⁵, R⁶, R⁷, R⁸, R⁹, R¹⁰, m, n and p are as defined herein.

In embodiments, the compound is a compound of formula (III) or a pharmaceutically acceptable salt or solvate thereof: 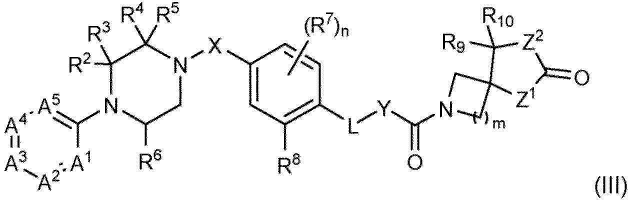 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, X, Y, Z¹, Z², R¹, R², R³, R⁴, R⁵, R⁶, R⁷, R⁸, R⁹, R¹⁰, m and n are as defined herein.

In embodiments, the compound is a compound of formula (IV) or a pharmaceutically acceptable salt or solvate thereof: 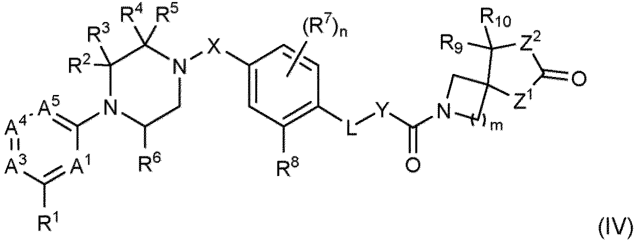 wherein A¹, A³, A⁴ and A⁵ are each independently selected from CH, CR¹² and N; wherein R¹² is selected from H, halo, CN, OCF₃, C₁₋₄ alkyl, C₁₋₄ haloalkyl, and C₁₋₄ alkoxy; and wherein L, X, Y, Z¹, Z², R¹, R², R³, R⁴, R⁵, R⁶, R⁷, R⁸, R⁹, R¹⁰, m and n are as defined herein.

In embodiments, the compound is a compound of formula (V) or a pharmaceutically acceptable salt or solvate thereof: 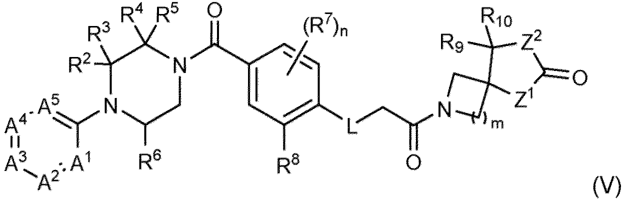 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, Z¹, Z², R¹, R², R³, R⁴, R⁵, R⁶, R⁷, R⁸, R⁹, R¹⁰, m and n are as defined herein.

In embodiments, the compound is a compound of formula (VI) or a pharmaceutically acceptable salt or solvate thereof: 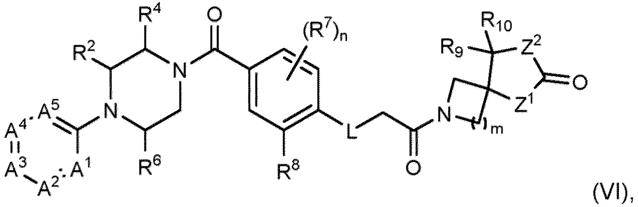 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, Z¹, Z², R¹, R², R⁴, R⁶, R⁷, R⁸, R⁹, R¹⁰, m and n are as defined herein. R² and R⁴ may be arranged *trans* to one another.

In embodiments, the compound is a compound of formula (VII) or a pharmaceutically acceptable salt or solvate thereof: 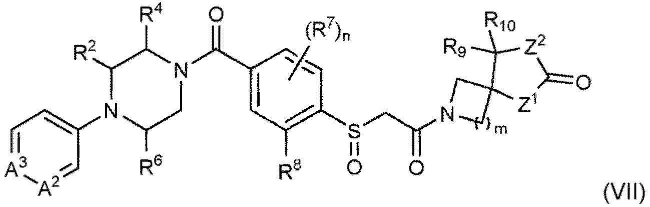 wherein A³ is selected from CH, CR¹² and N, and A² is CR¹; or A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein Z¹, Z², R¹, R², R⁴, R⁶, R⁷, R⁸, R⁹, R¹⁰, m and n are as defined herein. R² and R⁴ may be arranged *trans* to one another.

In embodiments, the compound is a compound of formula (Villa), (Vlllb) or a pharmaceutically acceptable salt or solvate thereof: 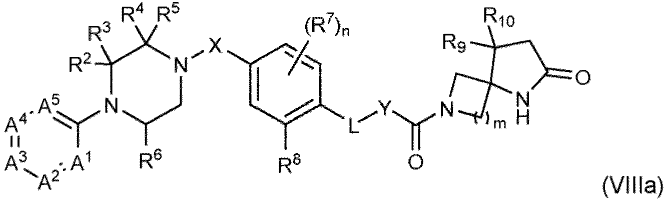 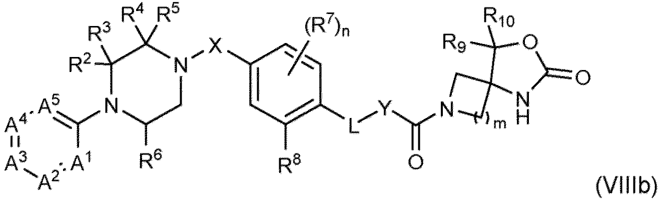 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, X, Y, R¹, R², R³, R⁴, R⁵, R⁶, R⁷, R⁸, R⁹, R¹⁰, m and n are as defined herein.

In embodiments, the compound is a compound of formula (IXa), (IXb) or a pharmaceutically acceptable salt or solvate thereof: 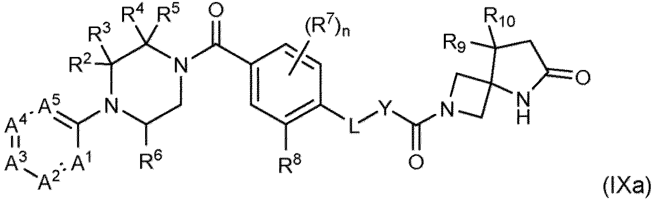 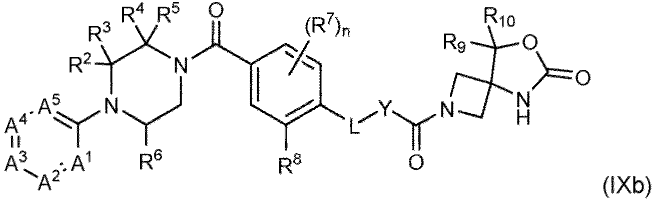 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, Y, R¹, R², R³, R⁴, R⁵, R⁶, R⁷, R⁸, R⁹, R¹⁰ and n are as defined herein.

In embodiments, the compound is a compound of formula (Xa), (Xb) or a pharmaceutically acceptable salt or solvate thereof: 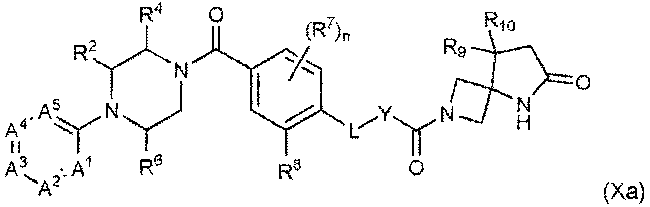 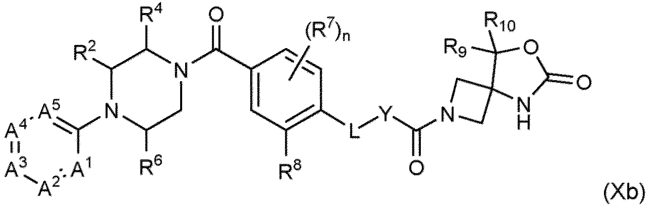 wherein A¹, A³, A⁴ and A⁵ are each independently selected from CH, CR¹² and N, and A² is CR¹; or A¹, A⁴ and A⁵ are each independently selected from CH, CR¹² and N, and A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, Y, R¹, R², R⁴, R⁶, R⁷, R⁸, R⁹, R¹⁰ and n are as defined herein.

In embodiments, the compound is a compound of formula (XIa), (Xlb) or a pharmaceutically acceptable salt or solvate thereof: 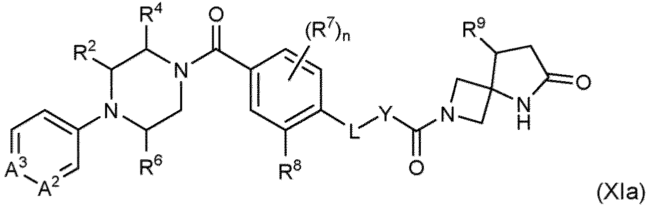 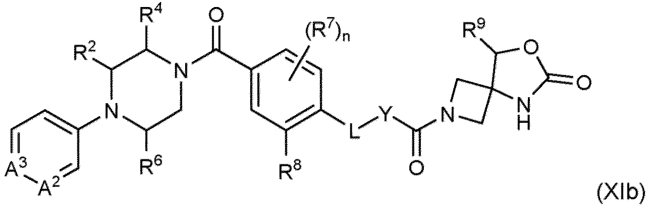 wherein A³ is selected from CH, CR¹² and N, and A² is CR¹; or A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein L, Y, R¹, R², R⁴, R⁶, R⁷, R⁸, R⁹ and n are as defined herein.

In embodiments, the compound is a compound of formula (Xlla), (Xllb) or a pharmaceutically acceptable salt or solvate thereof: 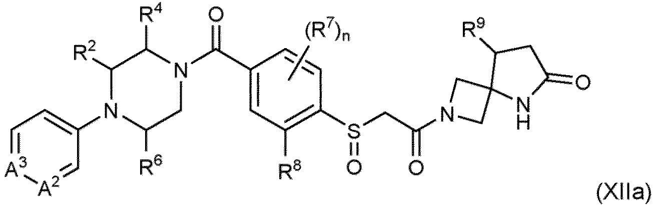 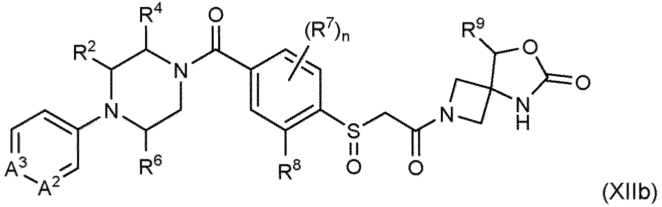 wherein A³ is selected from CH, CR¹² and N, and A² is CR¹; or A² and A³ together form a 5- or 6-membered heterocycloalkyl ring; wherein R¹² is selected from H, halo, CN, OCF₃, C₁₋₄ alkyl, C₁₋₄ haloalkyl, and C₁₋₄ alkoxy; and wherein R¹, R², R⁴, R⁶, R⁷, R⁸, R⁹ and n are as defined herein.

In embodiments, one or more of A, A¹, A², A³, A⁴, A⁵, L, X, Y, Z¹, Z², R¹, R², R³, R⁴, R⁵, R⁶, R⁷, R⁸, R⁹, R¹⁰, R¹¹, R¹², R^{a}, R^{b}, R^{c}, R^{d}, R^{e}, a, b, d, e, m, n and p are as described in the following paragraphs:

A may be a 6-membered aromatic ring or a 5- or 6-membered heteroaromatic ring comprising at least one N atom, optionally fused to a 5- or -6 membered heterocycloalkyl ring or a C₅ or C₆ cycloalkyl. A may be a 6-membered aromatic ring, optionally fused to a 5- or -6 membered heterocycloalkyl ring. A may be a 5- or 6-membered heteroaromatic ring comprising at least one N atom, optionally fused to a 5- or -6 membered heterocycloalkyl ring or a C₅ or C₆ cycloalkyl.

A may be a 6-membered aromatic ring. A may be a 5- or 6-membered heteroaromatic ring comprising at least one N atom. A may be a 5-membered heteroaromatic ring comprising at least one N atom. A may be a 6-membered heteroaromatic ring comprising at least one N atom.

A may be selected from optionally substituted phenyl, optionally substituted pyridyl, and optionally substituted benzodioxole. A may be selected from optionally substituted phenyl and optionally substituted benzodioxole. A may be optionally substituted phenyl. A may be optionally substituted benzodioxole.

It may be that, when substituted, said phenyl, pyridyl or benzodioxole is substituted with one or more groups selected from: halo, C₁₋₄ alkyl, C₁₋₄ haloalkyl, C₁₋₄ alkoxy, NO₂, CN, O-(C₁₋₄ alkyl)ₐ-aryl, or O-(C₁₋₄ alkyl)_{b}-heteroaryl. It may be that, when substituted, said phenyl, pyridyl or benzodioxole is substituted with one or more groups selected from: halo, C₁₋₄ alkyl, C₁₋₄ haloalkyl, C₁₋₄ alkoxy and O-(C₁₋₄ alkyl)ₐ-aryl. It may be that, when substituted, said phenyl, pyridyl or benzodioxole is substituted with one or more groups selected from: halo, C₁₋₄ alkyl and C₁₋₄ haloalkyl. It may be that, when substituted, said phenyl, pyridyl or benzodioxole is substituted with one or more halo groups (e.g. F, Cl or Br).

It may be that A¹, A³, A⁴ and A⁵ are each independently selected from CH, CR¹² and N, and A² is CR¹. Any one, two or three of A¹, A³, A⁴ and A⁵ may be N. Any one of A¹, A³, A⁴ and A⁵ may be N. In embodiments, each of A¹, A³, A⁴ and A⁵ is independently selected from CH and CR¹².

Alternatively, it may be that A¹, A⁴ and A⁵ are each independently selected from CH, CR¹² and N, and A² and A³ together form a 5- or 6-membered heterocycloalkyl ring. Any one, two or three of A¹, A⁴ and A⁵ may be N. Any one of A¹, A⁴ and A⁵ may be N. In embodiments, each of A¹, A⁴ and A⁵ is independently selected from CH and CR¹². In embodiments, A² and A³ together form a 5-membered heterocycloalkyl ring (e.g. a 1,3-dioxole).

A² may be CH. A² may be C-C₁₋₄ alkyl (e.g. C-CH₃). A² may be C-halo (e.g. C-Cl or C-F).

A¹, A², A³, A⁴ and A⁵ may be selected such that the to which they belong is selected from a substituted or unsubstituted phenyl, substituted or unsubstituted pyridyl, substituted or unsubstituted pyrimidyl, or substituted or unsubstituted benzodioxole. A', A², A³, A⁴ and A⁵ may be selected such that the ring to which they belong is selected from a substituted or unsubstituted phenyl and substituted or unsubstituted benzodioxole. A', A², A³, A⁴ and A⁵ may be selected such that the ring to which they belong is substituted or unsubstituted phenyl. A¹, A², A³, A⁴ and A⁵ may be selected such that the ring to which they belong is substituted or unsubstituted benzodioxole.

X may be -C(O)-. X may be -C(R^{a}R^{b})-. X may be -CH₂-.

L may be selected from -S(=O)-, -SO₂-, -O- and -C(O)-. L may be selected from - S(=O)- and -O-. L may be -S(=O)-. L may be -O-.

Y may be -CH₂-, -CF₂-, -CH(C₁₋₄ alkyl)-, -C(C₁₋₄ alkyl)₂-, 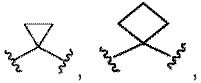 or 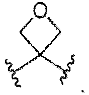 Y may be -CH₂-. Y may be -CF₂-. Y may be -CH(C₁₋₄ alkyl)-. Y may be -C(C₁₋₄ alkyl)₂-.

Z¹ may be -NH-. Z¹ may be -N(C₁₋₄alkyl)- (e.g. -N(Me)-).

Z² may be selected from -CH₂- and -O-. Z² may be -CH₂-. Z² may be -O-CH₂-. Z² may be -O-.

R' may be selected from H, halo, C₁₋₄ alkyl, C₁₋₄ haloalkyl, C₁₋₄ alkoxy, NO₂, CN, O-(C₁₋₄ alkyl)ₐ-aryl, or O-(C₁₋₄ alkyl)_{b}-heteroaryl. R¹ may be selected from H, halo, C₁₋₄ alkyl, C₁₋₄ haloalkyl, or O-(C₁₋₄ alkyl)ₐ-aryl. R¹ may be selected from halo, C₁₋₄ alkyl, C₁₋₄ haloalkyl, or O-(C₁₋₄ alkyl)ₐ-aryl. R¹ may be halo (for example F, Cl or Br; e.g. Cl or Br). R¹ may be C₁₋₄ alkyl (e.g. methyl). R¹ may be , C₁₋₄ haloalkyl (e.g. CF₃). R¹ may be O-(C₁₋₄ alkyl)ₐ-aryl (e.g. O-Bn). R¹ may be O-(C₁₋₄ alkyl)ₐ-aryl. R¹ may be H.
a may be 1 or 2. a may be 1. b may be 1 or 2. b may be 1.

Each R² and R⁴ may be independently selected from H and C₁₋₄ alkyl (e.g. CH₃). R² may be H. R² may be C₁₋₄ alkyl (e.g. CH₃). R⁴ may be H. R⁴ may be C₁₋₄ alkyl (e.g. CH₃).

It may be that R² is H and R⁴ is C₁₋₄ alkyl (e.g. CH₃). It may be that R⁴ is H and R² is C₁₋₄ alkyl (e.g. CH₃). It may be that R² and R⁴ are both H. It may be that R² and R⁴ are both C₁₋₄ alkyl (e.g. CH₃).

R² and R⁴ may be arranged *trans* to one another. For the avoidance of doubt, where appropriate, this means that R² and R⁴ are arranged on the piperazine ring as:

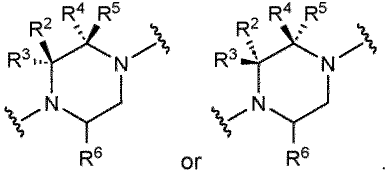

It may be that each of R³, R⁵ and R⁶ is independently selected from H and C₁₋₄ alkyl. It may be that R³ and R⁵ together form a 5- or 6-membered ring and R⁶ is selected from H and C₁₋₄ alkyl. It may be that R³ and R⁶ together form an alkyl or heteroalkyl bridge and R⁵ is selected from H and C₁₋₄ alkyl.

It may be that each of R³, R⁵ and R⁶ may be independently selected from H and CH₃. It may be that R³ and R⁵ together form a 5- or 6-membered ring and R⁶ is selected from H and CH₃. It may be that R³ and R⁶ together form an alkyl or heteroalkyl bridge and R⁵ is selected from H and CH₃.

Each R⁷ may be independently selected from H, halo, C₁₋₄ alkyl, C₁₋₄ haloalkyl, C₁₋₄ alkoxy, CN and NO₂. Each R⁷ may be independently selected from H, halo, C₁₋₄ haloalkyl, and NO₂. Each R⁷ may be independently selected from halo, C₁₋₄ haloalkyl, and NO₂. Each R⁷ may be independently selected from halo (e.g. F, Cl, or Br), C₁₋₄ haloalkyl (e.g. CF₃), and NO₂. Each R⁷ may be independently selected from H and halo. Each R⁷ may be H. n may be 0 or 1. n may be 0. n may be 1.

Each R⁸ may be independently selected from H, halo, C₁₋₄ alkyl, C₁₋₄ haloalkyl, C₁₋₄ alkoxy, CN and NO₂. Each R⁸ may be independently selected from H, halo, C₁₋₄ haloalkyl, and NO₂. Each R⁸ may be independently selected from halo, C₁₋₄ haloalkyl, and NO₂. Each R⁸ may be independently selected from halo (e.g. F, Cl, or Br), C₁₋₄ haloalkyl (e.g. CF₃), and NO₂. Each R⁸ may be halo (e.g. F, Cl, or Br). p may be 0 or 1. p may be 1 or 2. p may be 1. p may be 0. p may be 2.

It may be that n is 0 or 1 and p is 1. In these embodiments, it may be that R⁷ is H and R⁸ is halo (e.g. F, Cl, or Br).

R⁹ may be selected from H, C₁₋₆ haloalkyl, -OR¹⁰, OR¹¹, C₁₋₆ alkyl, C₂₋₈ alkyl interrupted by 1, 2 or 3 ether linkages, -(C₂₋₄ haloalkyl)-OR¹¹, and -(C₁₋₆ haloalkyl)-R¹¹. R⁹ may be selected from C₁₋₄ haloalkyl, -OR¹⁰, C₂₋₆ alkyl interrupted by 1, 2 or 3 ether linkages, -(C₂₋₄ haloalkyl)-OR¹¹, and (C₁₋₆ haloalkyl)-R¹¹.

R⁹ may be H. R⁹ may be C₁₋₆ alkyl (e.g. methyl, ethyl, or isopropyl).

R⁹ may be optionally substituted where chemically possible with one, two or three groups (e.g. one group) independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R⁹ may be substituted where chemically possible with one, two or three groups (e.g. one group) each independently selected from H, OH, =O, CN, halo, C₁₋₄ haloalkyl, C₁₋₄ alkyl, C₁₋₄ alkoxy, OCF₃, and phenyl. R⁹ may be substituted where chemically possible with one, two or three groups (e.g. one group) each independently selected from H, -OH, halo, C₁₋₄ haloalkyl, C₁₋₄ alkyl, and C₁₋₄ alkoxy. Alternatively, R⁹ may be unsubstituted.

R¹⁰ may be H. R¹⁰ may be C₁₋₆ alkyl (e.g. methyl, ethyl, or isopropyl).

R¹⁰ may be optionally substituted where chemically possible with one, two or three groups (e.g. one group) independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R¹⁰ may be substituted where chemically possible with one, two or three groups (e.g. one group) each independently selected from H, OH, =O, CN, halo, C₁₋₄ alkyl, C₁₋₄ haloalkyl, C₁₋₄ alkoxy, OCF₃, and phenyl. R¹⁰ may be substituted where chemically possible with one, two or three groups (e.g. one group) each independently selected from H, -OH, halo, C₁₋₄ haloalkyl, C₁₋₄ alkyl, and C₁₋₄ alkoxy. Alternatively, R¹⁰ may be unsubstituted.

It may be that R⁹ and R¹⁰ are both H.

R¹¹ may be selected from C₁₋₆ alkyl, C₂₋₈ alkyl interrupted by 1, 2 or 3 ether linkages, C₃₋₈ cycloalkyl, C₆ aryl, 5- or 6-membered heteroaryl, and 3- to 7-membered heterocycloalkyl. R¹¹ may be selected from C₁₋₄ alkyl, C₂₋₆ alkyl interrupted by 1, 2 or 3 ether linkages, C₃₋₆ cycloalkyl, or 3- to 7-membered heterocycloalkyl.

R¹¹ may be optionally substituted where chemically possible with one, two or three groups (e.g. one group) independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl. R¹¹ may be substituted where chemically possible with one, two or three groups (e.g. one group) each independently selected from H, OH, =O, CN, halo, C₁₋₄ alkyl, C₁₋₄ haloalkyl, C₁₋₄ alkoxy, OCF₃, and phenyl. R¹¹ may be substituted where chemically possible with one, two or three groups (e.g. one group) each independently selected from H, -OH, halo, C₁₋₄ haloalkyl, C₁₋₄ alkyl, and C₁₋₄ alkoxy. Alternatively, R¹¹ may be unsubstituted.

Each R¹² may be independently selected from: H, halo, CN, OCF₃, C₁₋₄ alkyl, C₁₋₄ haloalkyl, and C₁₋₄ alkoxy. It may be that each R¹² is H. It may be that one or more R¹² are independently selected from halo, CN, OCF₃, C₁₋₄ alkyl, C₁₋₄ haloalkyl, and C₁₋₄ alkoxy; and each remaining R¹² is H. It may be that one R¹² is selected from halo, CN, OCF₃, C₁₋₄ alkyl, C₁₋₄ haloalkyl, and C₁₋₄ alkoxy; and each remaining R¹² is H.

R^{a} and R^{b} may be independently selected from H and C₁₋₄ alkyl. It may be that R^{a} is H and R^{b} is C₁₋₄ alkyl. It may be that R^{a} and R^{b} are both H. It may be that R^{a} and R^{b} together form a 3- to 6-membered (e.g. a 3- to 4-membered) cycloalkyl or heterocycloalkyl ring system. It may be that R^{a} and R^{b} together form a 3- to 6-membered (e.g. a 3 to 4-membered) cycloalkyl ring system.

R^{c} and R^{d} may be independently selected at each occurrence from H, F, C₁₋₄ alkyl, and C₁₋₄ haloalkyl. R^{c} and R^{d} may be independently selected at each occurrence from H, F and C₁₋₄ alkyl. R^{c} and R^{d} may be independently selected at each occurrence from H and F. It may be that R^{c} and R^{d} together form a 3- to 6-membered (e.g. 3- to 4-membered) cycloalkyl or heterocycloalkyl ring system. It may be that R^{c} and R^{d} together form a 3- to 6-membered (e.g. 3- to 4-membered) cycloalkyl ring system.

R^{e} may be H. R^{e} may be C₁₋₄ alkyl.

The compound may be selected from the following compounds, or a pharmaceutically acceptable salt or solvate thereof: 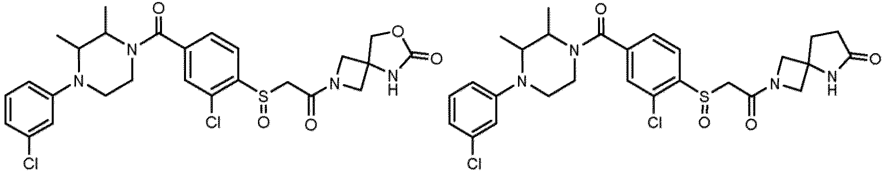 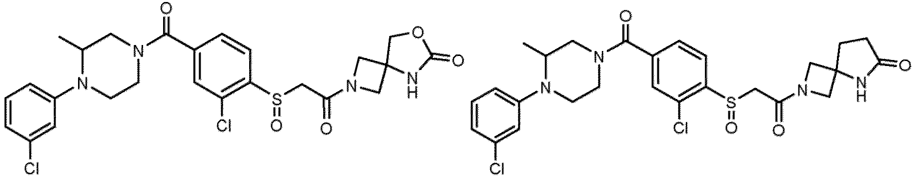 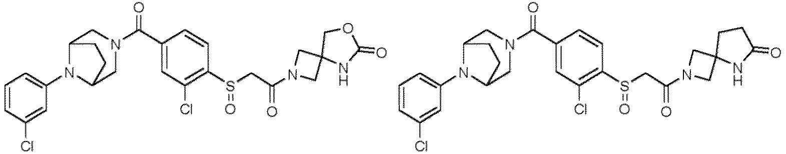 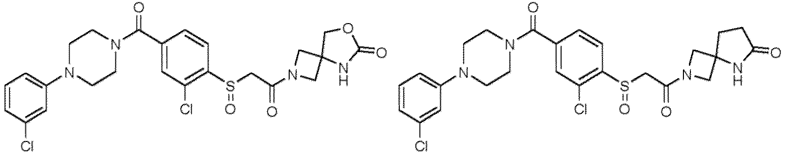 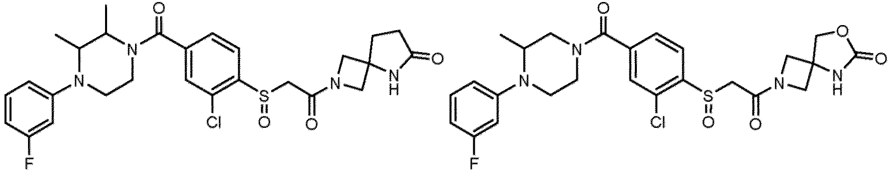 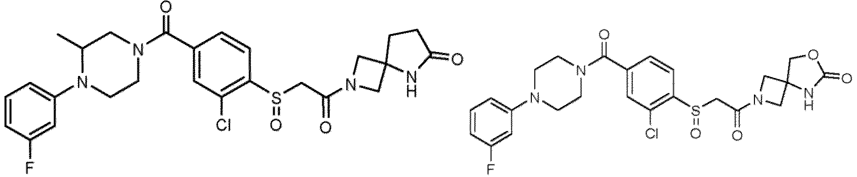 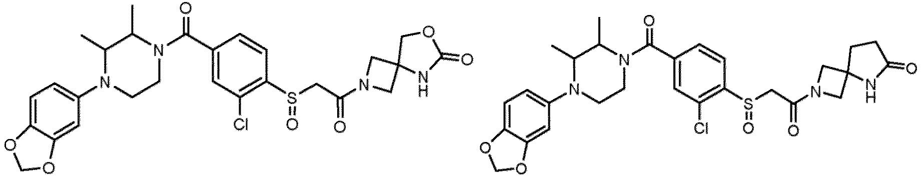 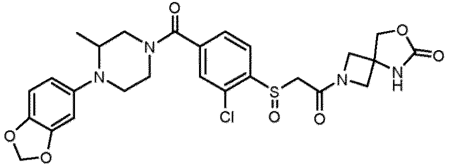 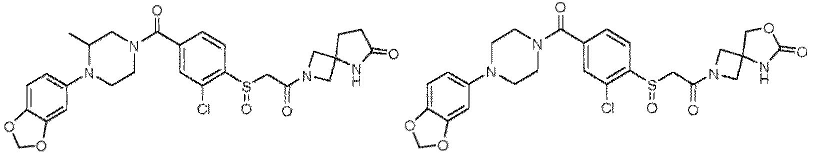 and

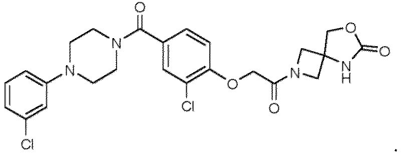

The compound may be selected from the following compounds, or a pharmaceutically acceptable salt or solvate thereof: 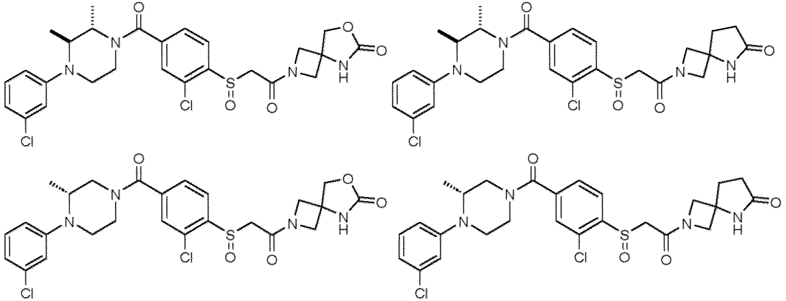 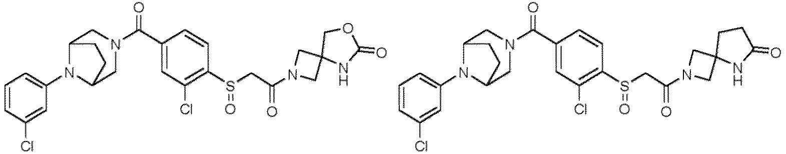 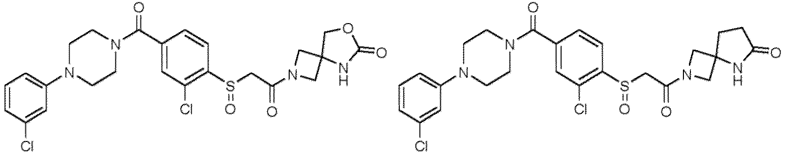 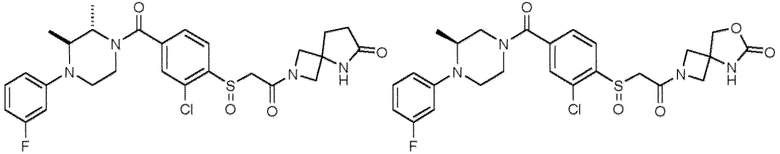 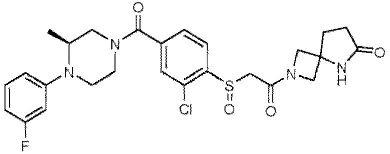 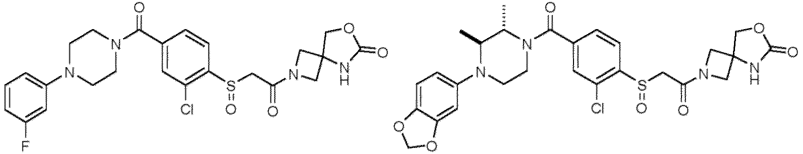 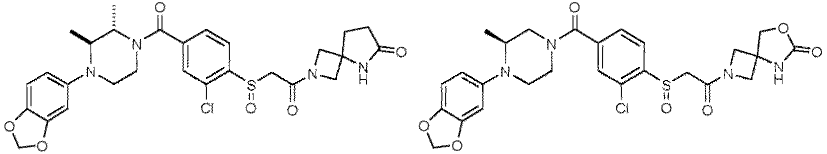 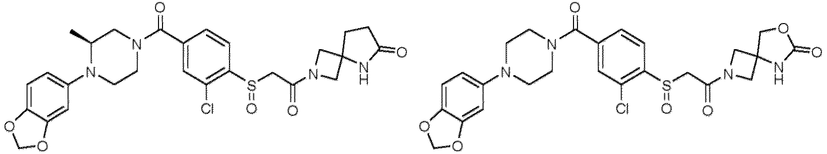 and

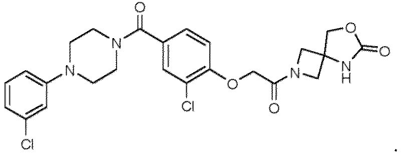

### FORMULATIONS AND ADMINISTRATION

Compounds of the invention may be administered orally, topically, intravenously, subcutaneously, buccally, rectally, dermally, nasally, tracheally, bronchially, by any other parenteral route, as an oral or nasal spray or via inhalation. The compounds may be administered in the form of pharmaceutical preparations comprising prodrug or active compound either as a free compound or, for example, a pharmaceutically acceptable nontoxic organic or inorganic acid or base addition salt, in a pharmaceutically acceptable dosage form. Depending upon the disorder and patient to be treated and the route of administration, the compositions may be administered at varying doses.

Typically, therefore, the pharmaceutical compounds of the invention may be administered orally, topically, or parenterally ("parenterally" as used herein, refers to modes of administration which include intravenous, intramuscular, intraperitoneal, intrasternal, subcutaneous and intraarticular injection and infusion) to a host to obtain a protease-inhibitory effect. In the case of larger animals, such as humans, the compounds may be administered alone or as compositions in combination with pharmaceutically acceptable diluents, excipients or carriers.

Actual dosage levels of active ingredients in the pharmaceutical formulations and pharmaceutical compositions of this invention may be varied so as to obtain an amount of the active compound(s) that is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

In the treatment, prevention, control, amelioration, or reduction of risk of conditions which require inhibition of MAGL activity, an appropriate dosage level may generally be about 0.01 to 500 mg per kg patient body weight per day which can be administered in single or multiple doses. The dosage level will be about 0.1 to about 250 mg/kg per day; more preferably about 0.5 to about 100 mg/kg per day. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0 and 1000.0 milligrams of the active ingredient for the symptomatic adjustment of the dosage to the patient to be treated. The compounds may be administered on a regimen of 1 to 4 times per day, e.g. once or twice per day. The dosage regimen may be adjusted to provide the optimal therapeutic response.

According to a further aspect of the invention there is thus provided a pharmaceutical composition or formulation including a compound of the invention. The composition may further comprise a pharmaceutically acceptable excipient, optionally a pharmaceutically acceptable adjuvant, diluent or carrier.

Pharmaceutical formulations or compositions of this invention for parenteral injection may comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters, such as ethyl oleate. Proper fluidity can be maintained, for example, by the use of coating materials, such as lecithin, by the maintenance of the required particle size in the case of dispersions and by the use of surfactants.

These compositions may also contain adjuvants such as preservative, wetting agents, emulsifying agents and dispersing agents. Inhibition of the action of microorganisms may be ensured by the inclusion of various antibacterial and antifungal agents, for example, paraben, chlorobutanol or phenol sorbic acid. It may also be desirable to include isotonic agents, such as sugars or sodium chloride, for example. Prolonged absorption of the injectable pharmaceutical form may be brought about by the inclusion of agents (for example, aluminium monostearate and gelatine) which delay absorption.

Solid dosage forms for oral administration include capsules, tablets, pills, powders and granules. In such solid dosage forms, the active compound is typically mixed with at least one inert, pharmaceutically acceptable excipient or carrier, such as sodium citrate or dicalcium phosphate and/or one or more: a) fillers or extenders, such as starches, lactose, sucrose, glucose, mannitol and silicic acid; b) binders, such as carboxymethylcellulose, alginates, gelatine, polyvinylpyrrolidone, sucrose and acacia; c) humectants, such as glycerol; d) disintegrating agents, such as agar-agar, calcium carbonate, potato or tapioca starch, alginic acid, certain silicates and sodium carbonate; e) solution retarding agents, such as paraffin; f) absorption accelerators, such as quaternary ammonium compounds; g) wetting agents, such as acetyl alcohol and glycerol monostearate; h) absorbents, such as kaolin and bentonite clay and i) lubricants, such as talc, calcium stearate, magnesium stearate, solid polyethylene glycols, sodium lauryl sulfate and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Oral formulations may contain a dissolution aid. Examples of dissolution aids include non-ionic surface active agents, such as sucrose fatty acid esters, glycerol fatty acid esters, sorbitan fatty acid esters (e.g. sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkyamine oxides; bile acid and salts thereof (eg chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsufonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract, and/or in delayed fashion. Examples of embedding compositions include polymeric substances and waxes.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavoring and perfuming agents. Suspensions, in addition to the active compounds, may contain suspending agents such as ethoxylated isostearyl alcohols, polyoxyethylene sorbitol and sorbitan esters, microcrystalline cellulose, aluminium metahydroxide, bentonite, agar-agar, and traganacanth and mixtures thereof.

Compositions for rectal or vaginal administration may be in the form of suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, sprays, creams, foams, gels, ointments and inhalants. The active compound is mixed under sterile conditions with a pharmaceutically acceptable carrier and any needed preservatives, buffers or propellants which may be required. Ophthalmic formulations, eye ointments, powders and solutions are also contemplated as being within the scope of this invention.

Insofar as they do not interfere with the activity of the compounds, the compositions and formulations according to the present subject matter may contain other active agents intended, in particular, for use in treating a condition selected from acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression. The other active ingredients may be intended for use in treating cancer, for example breast cancer, ovarian cancer, melanoma, hepatocellular carcinoma, colorectal cancer, prostate cancer, or nasopharyngeal carcinoma.

The formulations according to the present subject matter may also contain inactive components. Suitable inactive components are well known in the art and are described in standard textbooks, such as Goodman and Gillman's: The Pharmacological Bases of Therapeutics, 8thEd., Gilman et al, Eds. Pergamon Press (1990), and Remington's Pharmaceutical Sciences, 17th Ed., Mack Publishing Co., Easton, Pa. (1990), both of which are incorporated by reference herein in their entirety.

The formulations and compositions may be used in combination with an additional pharmaceutical dosage form to enhance their effectiveness in treating any of the disorders described herein. In this regard, the present formulations and compositions may be administered as part of a regimen additionally including any other pharmaceutical and/or pharmaceutical dosage form known in the art as effective for the treatment of any of these disorders.

### USES

The compounds of the invention are inhibitors of monoacylglycerol lipase (MAGL). MAGL is a serine hydrolase that plays a crucial role catalysing the hydrolysis of monoglycerides into glycerol and fatty acids. It links the endocannabinoid and eicosanoid systems together by degradation of the abundant endocannabinoid 2-arachidaoylglycerol into arachidonic acid, the precursor of prostaglandins and other inflammatory mediators.

MAGL is highly expressed in many cancer cells and is involved in metabolism more generally. In view of this, MAGL inhibitors are useful for treatments in many therapeutic fields, including as anti-nociceptive, anxiolytic, anti-inflammatory, and anticancer agents. ABX-1431, a first-in-class inhibitor of MAGL, is undergoing clinical studies for neurological disorders and other diseases (Cisar, J.S., Identification of ABX-1431, a Selective Inhibitor of Monoacylglycerol Lipase and Clinical Candidate for Treatment of Neurological Disorder, J. Med. Chem., 2018, Oct 25, 61(20), p 9062-9084).

MAGL is considered a therapeutic target in a number of disease areas, such as inflammation and neurological disorders, metabolic disorders, and cancer (Deng, H. and Li, W., Monoacylglycerol lipase inhibitors: Modulators for lipid metabolism in cancer malignancy, neurological and metabolic disorders, Acta Pharmaceutica Sinica B, https://doi.org/10.1016/i.apsb.2019.10.006). MAGL inhibition is applied in the area of pain and inflammation, metabolic disorders (such as obesity and diabetes), neurodegenerative pathologies (such as Alzheimer's disease), anxiety and epilepsy (Granchi, C. et al., Expert Opinion on Therapeutic Patents, 2017, 27(12), 1341-1351). MAGL inhibition is also considered a useful therapeutic approach in the treatment of many cancers and cancer associated symptoms, in particular in cancers such as breast cancer, ovarian cancer, melanoma, hepatocellular carcinoma, colorectal cancer, prostate cancer, or nasopharyngeal carcinoma. See, for example: Nomura D.K. et al., "Monoacylglycerol Lipase Regulates a Fatty Acid Network that Promotes Cancer Pathogenesis", Cell 140, 49-61, January 8, 2010; Zhu W. et al., "Monoacylglycerol lipase promotes progression of hepatocellular carcinoma via NF-κB-mediated epithelial-mesenchymal transition", Journal of Hematology & Oncology, 2016, 9, Article No. 127; Pagano E. et al., "Pharmacological inhibition of MAGL attenuates experimental colon carcinogenesis", Pharmacological Research, 2017, 119, 227-236; Nomura D.K. et al., "Monoacylglycerol Lipase Exerts Dual Control over Endocannabinoid and Fatty Acid Pathways to Support Prostate Cancer", Chemistry and Biology, 2011, 18(7), 846-856; Hu W.R etal., Monoacylglycerol lipase promotes metastases in nasopharyngeal carcinoma", Int. J. Clin. Exp. Pathol., 2014, 7(7), 3704-3713; Sticht M.A. et al., "Inhibition of monoacylglycerol lipase attenuates vomiting in Suncus murinus and 2-arachidonoyl glycerol attenuates nausea in rats", British Journal of Pharmacology, 2011, 165(8), https://doi.org/10.1111/j.1476-5381.2011.01407.x).

Accordingly, conditions that may be treated by the compounds of the invention include acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

The condition may be cancer. The cancer may be selected from Acute Lymphoblastic Leukemia (ALL), Acute Myeloid Leukemia (AML), Adrenocortical Carcinoma, Anal Cancer, Appendix Cancer, Atypical Teratoid/Rhabdoid Tumor, Basal Cell Carcinoma, Bile Duct Cancer, Biliary Cancer, Bladder Cancer, Bone Cancer, Brain Tumor, Astrocytoma, Brain and Spinal Cord Tumor, Brain Stem Glioma, Central Nervous System Atypical Teratoid/Rhabdoid Tumor, Central Nervous System Embryonal Tumor, Breast Cancer, Bronchial Tumor, Burkitt Lymphoma, Carcinoid Tumor, Cervical Cancer, Childhood Cancer, Chordoma, Chronic Lymphocytic Leukemia (CLL), Chronic Myelogenous Leukemia (CML), Chronic Myeloproliferative Disorder, Colon Cancer, Colorectal Cancer, Craniopharyngioma, Cutaneous T-Cell Lymphoma, Ductal Carcinoma In Situ (DCIS), Embryonal Tumors, Endometrial Cancer, Ependymoblastoma, Ependymoma, Esophageal Cancer, Esthesioneuroblastoma, Ewing Sarcoma, Extracranial Germ Cell Tumor, Extragonadal Germ Cell Tumor, Extrahepatic Bile Duct Cancer, Eye Cancer, Fibrous Histiocytoma of Bone, Gallbladder Cancer, Gastric Cancer, Gastrointestinal Carcinoid Tumor, Gastrointestinal Stromal Tumors (GIST), Extracranial Germ Cell Tumor, Extragonadal Germ Cell Tumor, Ovarian Germ Cell Tumor, Gestational Trophoblastic Tumor, Glioma, Hairy Cell Leukemia, Head and Neck Cancer, Heart Cancer, Hepatocellular Cancer, Hodgkin Lymphoma, Hypopharyngeal Cancer, Intraocular Melanoma, Islet Cell Tumors, Kaposi Sarcoma, Kidney Cancer, Langerhans Cell Histiocytosis, Laryngeal Cancer, Liver Cancer, Lobular Carcinoma In Situ (LCIS), Lung Cancer, Lymphoma, AIDS-Related Lymphoma, Male Breast Cancer, Medulloblastoma, Medulloepithelioma, Melanoma, Merkel Cell Carcinoma, Malignant Mesothelioma, Metastatic Squamous Neck Cancer with Occult Primary, Midline Tract Carcinoma Involving *NUT* Gene, Mouth Cancer, Multiple Endocrine Neoplasia Syndrome, Multiple Myeloma/Plasma Cell Neoplasm, Mycosis Fungoides, Myelodysplastic Syndrome, Myelodysplastic/Myeloproliferative Neoplasm, Multiple Myeloma, Nasal Cavity Cancer, Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip Cancer, Oropharyngeal Cancer, Osteosarcoma, Ovarian Cancer, Pancreatic Cancer, Papillomatosis, Paraganglioma, Parathyroid Cancer, Penile Cancer, Pharyngeal Cancer, Pheochromocytoma, Pineal Parenchymal Tumors of Intermediate Differentiation, Pineoblastoma, Pituitary Tumor, Plasma Cell Neoplasm, Pleuropulmonary Blastoma, Breast Cancer, Primary Central Nervous System (CNS) Lymphoma, Prostate Cancer, Rectal Cancer, Renal Cell Cancer, Renal Pelvis Cancer, Ureter Cancer, Retinoblastoma, Rhabdomyosarcoma, Salivary Gland Cancer, Sézary Syndrome, Skin Cancer, Small Cell Lung Cancer, Small Intestine Cancer, Soft Tissue Sarcoma, Squamous Cell Carcinom, Supratentorial Primitive Neuroectodermal Tumors, T-Cell Lymphoma, Testicular Cancer, Throat Cancer, Thymoma, Thymic Carcinoma, Thyroid Cancer, Transitional Cell Cancer of the Renal Pelvis and Ureter, Gestational Trophoblastic Tumor, Urethral Cancer, Uterine Cancer, Uterine Sarcoma, Waldenström Macroglobulinemia, or Wilms Tumor.

In an aspect of the invention, there is provided a compound of the invention or a pharmaceutical composition or formulation of the invention for use as a medicament.

In another aspect of the invention, there is provided a compound of the invention, or a pharmaceutical composition or formulation of the invention for the inhibition of monoacylglycerol lipase (MAGL).

In another aspect of the invention, there is provided a compound of the invention, or a pharmaceutical composition or formulation of the invention for use in the treatment of a condition which is modulated by monoacylglycerol lipase (MAGL).

Another aspect provides a compound of the invention, or a pharmaceutical composition or formulation of the invention for use in the treatment of a condition selected from: acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression. In embodiments, the condition is cancer. For example, the condition may be breast cancer, ovarian cancer, melanoma, hepatocellular carcinoma, colorectal cancer, prostate cancer, or nasopharyngeal carcinoma.

Another aspect provides a method for the treatment, in a mammal in need of said treatment, of a condition selected from: acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression. In embodiments, the condition is cancer. For example, the condition may be breast cancer, ovarian cancer, melanoma, hepatocellular carcinoma, colorectal cancer, prostate cancer, or nasopharyngeal carcinoma. The method comprises administering to the mammal a therapeutically effective amount of any of the compounds or any of the pharmaceutical compositions or formulations described herein.

Another aspect provides use of a compound of the invention or a pharmaceutical composition or formulation of the invention for the inhibition of monoacylglycerol lipase (MAGL).

Another aspect provides use of a compound of the invention in the manufacture of a medicament for use in the treatment of a condition selected from: acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

In embodiments the condition is cancer. In embodiments where the condition is cancer, the cancer may be selected from Acute Lymphoblastic Leukemia (ALL), Acute Myeloid Leukemia (AML), Adrenocortical Carcinoma, Anal Cancer, Appendix Cancer, Atypical Teratoid/Rhabdoid Tumor, Basal Cell Carcinoma, Bile Duct Cancer, Biliary Cancer, Bladder Cancer, Bone Cancer, Brain Tumor, Astrocytoma, Brain and Spinal Cord Tumor, Brain Stem Glioma, Central Nervous System Atypical Teratoid/Rhabdoid Tumor, Central Nervous System Embryonal Tumor, Breast Cancer, Bronchial Tumor, Burkitt Lymphoma, Carcinoid Tumor, Cervical Cancer, Childhood Cancer, Chordoma, Chronic Lymphocytic Leukemia (CLL), Chronic Myelogenous Leukemia (CML), Chronic Myeloproliferative Disorder, Colon Cancer, Colorectal Cancer, Craniopharyngioma, Cutaneous T-Cell Lymphoma, Ductal Carcinoma In Situ (DCIS), Embryonal Tumors, Endometrial Cancer, Ependymoblastoma, Ependymoma, Esophageal Cancer, Esthesioneuroblastoma, Ewing Sarcoma, Extracranial Germ Cell Tumor, Extragonadal Germ Cell Tumor, Extrahepatic Bile Duct Cancer, Eye Cancer, Fibrous Histiocytoma of Bone, Gallbladder Cancer, Gastric Cancer, Gastrointestinal Carcinoid Tumor, Gastrointestinal Stromal Tumors (GIST), Extracranial Germ Cell Tumor, Extragonadal Germ Cell Tumor, Ovarian Germ Cell Tumor, Gestational Trophoblastic Tumor, Glioma, Hairy Cell Leukemia, Head and Neck Cancer, Heart Cancer, Hepatocellular Cancer, Hodgkin Lymphoma, Hypopharyngeal Cancer, Intraocular Melanoma, Islet Cell Tumors, Kaposi Sarcoma, Kidney Cancer, Langerhans Cell Histiocytosis, Laryngeal Cancer, Liver Cancer, Lobular Carcinoma In Situ (LCIS), Lung Cancer, Lymphoma, AIDS-Related Lymphoma, Male Breast Cancer, Medulloblastoma, Medulloepithelioma, Melanoma, Merkel Cell Carcinoma, Malignant Mesothelioma, Metastatic Squamous Neck Cancer with Occult Primary, Midline Tract Carcinoma Involving *NUT* Gene, Mouth Cancer, Multiple Endocrine Neoplasia Syndrome, Multiple Myeloma/Plasma Cell Neoplasm, Mycosis Fungoides, Myelodysplastic Syndrome, Myelodysplastic/Myeloproliferative Neoplasm, Multiple Myeloma, Nasal Cavity Cancer, Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip Cancer, Oropharyngeal Cancer, Osteosarcoma, Ovarian Cancer, Pancreatic Cancer, Papillomatosis, Paraganglioma, Parathyroid Cancer, Penile Cancer, Pharyngeal Cancer, Pheochromocytoma, Pineal Parenchymal Tumors of Intermediate Differentiation, Pineoblastoma, Pituitary Tumor, Plasma Cell Neoplasm, Pleuropulmonary Blastoma, Breast Cancer, Primary Central Nervous System (CNS) Lymphoma, Prostate Cancer, Rectal Cancer, Renal Cell Cancer, Renal Pelvis Cancer, Ureter Cancer, Retinoblastoma, Rhabdomyosarcoma, Salivary Gland Cancer, Sézary Syndrome, Skin Cancer, Small Cell Lung Cancer, Small Intestine Cancer, Soft Tissue Sarcoma, Squamous Cell Carcinoma, Supratentorial Primitive Neuroectodermal Tumors, T-Cell Lymphoma, Testicular Cancer, Throat Cancer, Thymoma, Thymic Carcinoma, Thyroid Cancer, Transitional Cell Cancer of the Renal Pelvis and Ureter, Gestational Trophoblastic Tumor, Urethral Cancer, Uterine Cancer, Uterine Sarcoma, Waldenström Macroglobulinemia, or Wilms Tumor. In embodiments where the condition is cancer, the cancer may be selected from breast cancer, ovarian cancer, melanoma, hepatocellular carcinoma, colorectal cancer, prostate cancer, or nasopharyngeal carcinoma.

MAGL inhibition is also useful in other applications. For example, MAGL inhibitors may be used *in vitro,* in research and other settings where inhibition of one or more lipases and MAGL more specifically is desirable. Embodiments therefore provide use of a compound of the invention, or a pharmaceutical composition or formulation of the invention for the inhibition of MAGL.

### Synthesis of Compounds

Compounds of the invention may be synthesised according the synthetic schemes disclosed herein.

### General procedure A: Peptide coupling using EDC/HOBt

The appropriate amine (1 eq.), EDC (1.5 eq.), HOBt (1.5 eq.) and 2-chloro-4-*trans*-4-(3-chlorophenyl)-2,3-dimethylpiperazine-1-carbonyl)phenyl)sulfinyl)acetic acid (1 eq.) were dissolved in DCM (0.04 M), after which DiPEA (4 eq.) was added in a dropwise manner and the mixture was stirred for 16 h. Upon reaction completion, the solution was diluted with DCM and washed with brine, after which the aqueous layer was extracted with DCM (3x). The combined organic layers were dried (MgSO₄), filtered and concentrated under reduced pressure and the crude product was further purified by silica gel column chromatography to obtain the final compounds.

### General procedure B: Peptide coupling using PyAOP

The appropriate amine (1 eq.), PyAOP (2 eq.) and the appropriate carboxylic acid (1 eq.) were dissolved in DMF (0.4 M), after which DiPEA (4 eq.) was added in a dropwise manner and the mixture was stirred for 16 h. Upon reaction completion, the solution was diluted with EtOAc and washed with brine (3x), after which the aqueous layer was extracted with EtOAc (4x). The combined organic layers were dried (MgSO₄), filtered and concentrated under reduced pressure and the remaining oil was further purified by silica gel column chromatography to obtain the final compounds.

### General procedure C: Buchwald-Hartwig coupling

The appropriate bromo-benzene (1 eq.), the appropriate Boc-protected piperazine (1 or 1.1 eq.), potassium *tert*-butoxide (2 eq.) and BINAP (0.06 eq.) were dissolved in degassed 1,4-dioxane (0.4 M). Subsequently, the suspension was stirred and further degassed with argon for an additional 15 minutes, before Pd(Ac)₂ (0.04 eq.) was added and the mixture was heated to 85 °C under argon atmosphere overnight. After confirming product formation by TLC analysis, the mixture was diluted with DCM, filtered over Celite^{®} and washed with water and brine. Subsequently, the organic layer was dried (MgSO₄), filtered and concentrated under reduced pressure, after which the crude residue was purified by silica column chromatography to afford the tertiary amine.

### General procedure D: tert-Butyl deprotection

The appropriate *tert*-butyl protected benzoic acid was dissolved in a mixture of 20% (v/v) TFA/DCM (0.4 M) and stirred for 7 h, during which reaction progression was monitored by TLC analysis. Upon reaction completion, the mixture was concentrated under reduced pressure, before purifying the crude product by silica gel column chromatography to isolate the free carboxylic acid.

### General procedure E: Boc deprotection

The appropriate Boc-protected amine was dissolved in a mixture of 20% (v/v) TFA/DCM (0.4 M) and stirred for 1 h, during which reaction progression was monitored by TLC analysis. Upon reaction completion, the mixture was basified using 10 M NaOH, extracted with DCM (3x). The combined organic layers were dried (MgSO₄), filtered and concentrated under reduced pressure, before purifying the crude product by silica gel column chromatography to isolate the free amine.

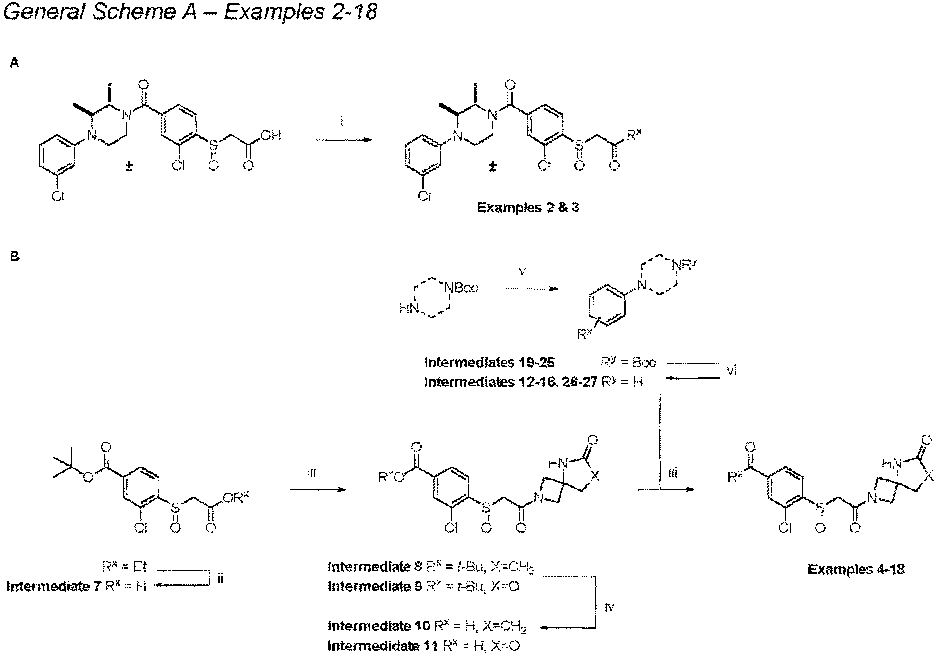

Reagents and conditions: i) Intermediate 1 or Intermediate 2, EDC, HOBt, DiPEA, DCM, 16 h, 14-35%; ii) MeOH/TEA/H₂O (1/1/1), 2.5 h, 93%; iii) Intermediate 1 or Intermediate 2, PyAOP, DiPEA, DMF, 16 h, 18-71%; iv) TFA, DCM, 6 h, 70-84%; v) the appropriate bromobenzene, KOtBu, BINAP, Pd(OAc)₂, 1,4-dioxane, 85 °C, 18 h, 28-78%; vi) TFA, DCM, 1 h, 64-92%.

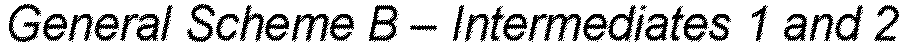

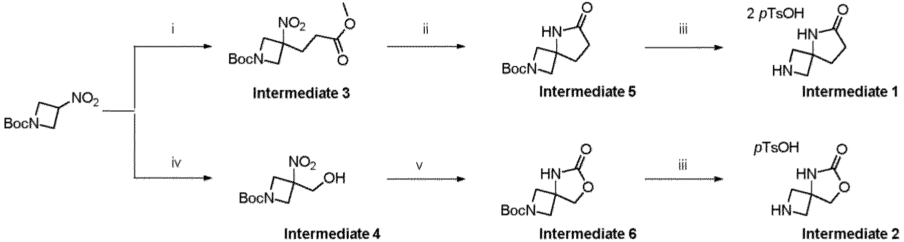

Reagents and conditions: i) methylacrylate, K₂CO₃, MeOH, 3 h 65%; ii) NiCl₂·6H₂O, NaBH₄, MeOH, 4 h, 90%; iii) pTsOH, EtOAc, 75 °C, 3 h, 79-95%; iv) formaldehyde, TEA, ACN, 1 h, 86%; v) Pd/C, H₂, THF, 72 h, then triphosgene, TEA, THF, 1 h, 46% over two steps.

### FURTHER EMBODIMENTS

The invention and disclosure further include the subject matter of the following numbered clauses:
1. A compound of formula (I), or a pharmaceutically acceptable salt or solvate thereof 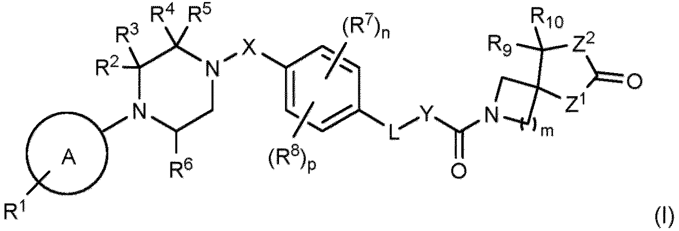 wherein
   A is a 6-membered aromatic ring, or a 5- or 6-membered heteroaromatic ring optionally comprising at least one N atom, wherein the 6-membered aromatic ring of the 5- or 6-membered heteroaromatic ring is optionally fused to a 5- or -6 membered heterocycloalkyl ring or a C₅ or C₆ cycloalkyl;
   X is selected from -C(O)- and -C(R^{a}R^{b})-;
   L is selected from S(=O)-, -SO₂-, -S-, -O-, -C(O)-, -CH₂-, -C(R^{c}R^{d})-;
   Y is -CH₂-, -CF₂-, -C(R^{c}R^{d})-;
   Z¹ is -NR^{e}-;
   Z² is selected from -CH₂-, -O-CH₂-, and -O-;
   R' is selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl;
   each R² and R⁴ is independently selected from H and C₁₋₄ alkyl;
   each R³, R⁵ and R⁶ is independently selected from H and C₁₋₄ alkyl; or R³ and R⁶ together form an alkyl or heteroalkyl bridge and R⁵ is selected from H and C₁₋₄ alkyl;
   each R⁷ and R⁸ is independently selected from H, halo, C₁₋₄ alkyl, C₁₋₄ haloalkyl, C₁₋₄ alkoxy, CN and NO₂;
   R⁹ is selected from H, C₁₋₆ haloalkyl, OR¹¹, C₁₋₆ alkyl, and C₂₋₈ alkyl interrupted by 1, 2 or 3 ether linkages, -(C₂₋₄ haloalkyl)-OR¹¹, and -(C₁₋₆ haloalkyl)-R¹¹;
   R¹⁰ is selected from H and C₁₋₆ alkyl;
   R¹¹ is selected from C₁₋₆ alkyl, C₂₋₈ alkyl interrupted by 1, 2 or 3 ether linkages, C₃₋₈ cycloalkyl, C₆ aryl, 5- or 6-membered heteroaryl, and 3- to 7-membered heterocycloalkyl;
   wherein each R⁹, R¹⁰ and R¹¹ is optionally independently substituted where chemically possible with one, two or three groups independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl;
   R^{a} and R^{b} are independently selected from H, and C₁₋₄ alkyl; or wherein R^{a} and R^{b} together form a 3- to 6-membered cycloalkyl or heterocycloalkyl ring system;
   R^{c} and R^{d} are independently selected at each occurrence from H, F, C₁₋₄ alkyl, and C₁₋₄ haloalkyl; or R^{c} and R^{d} together form a 3- to 6-membered cycloalkyl or heterocycloalkyl ring system;
   R^{e} is selected from H and C₁₋₄ alkyl;
   m is selected from 1, or 2;
   n is selected from 0, 1, or 2; and
   p is selected from 0, 1, or 2.
2. The compound of clause 1, wherein Z¹ is -NH-.
3. The compound of clause 1 or clause 2, wherein Z² is -CH₂- or -O-.
4. The compound of any preceding clause, wherein m is 1.
5. The compound of any preceding clause, wherein A is selected from optionally substituted phenyl, optionally substituted pyridyl and optionally substituted benzodioxole.
6. The compound of any of clauses 1 to 4, wherein the compound is a compound of formula (II) or a pharmaceutically acceptable salt or solvate thereof: 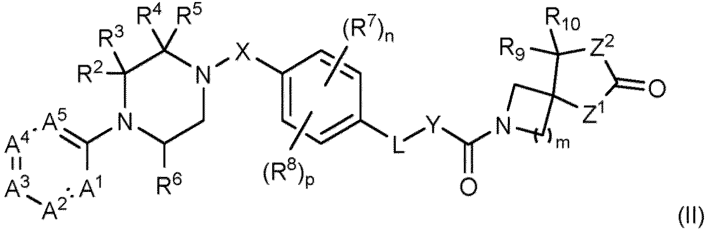
   wherein A¹, A³, A⁴ and A⁵ are each independently selected from CH, CR¹² or N, and A² is CR¹; or A¹, A⁴ and A⁵ are each independently selected from CH, CR¹² or N, and A² and A³ together form a 5- or 6-membered heterocycloalkyl ring;
   wherein each R¹² is independently selected from H, halo, CN, OCF₃, C₁₋₄ alkyl, C₁₋₄ haloalkyl, and C₁₋₄ alkoxy.
7. The compound of any preceding clause, wherein X is -C(O)-.
8. The compound of any preceding clause, wherein Y is -CH₂-.
9. The compound of any preceding clause, wherein L is selected from -S(=O)- and - O-, optionally wherein L is -S(=O)-.
10. The compound of any preceding clause, wherein R¹ is selected from H, halo, C₁₋₄ alkyl, C₁₋₄ haloalkyl, or O-(C₁₋₄ alkyl)ₐ-aryl; optionally wherein R¹ is selected from H, halo, C₁₋₄ alkyl, C₁₋₄ haloalkyl, or O-(C₁₋₄ alkyl)ₐ-aryl; further optionally wherein R¹ is halo.
11. The compound of any preceding clause, wherein a is selected from 1 and 2; optionally wherein a is 1.
12. The compound of any preceding clause, wherein b is selected from 1 and 2; optionally wherein b is 1.
13. The compound of any preceding clause, wherein at least one of R² and R⁴ is C₁₋₄ alkyl (e.g. CH₃); optionally wherein both of R² and R⁴ are C₁₋₄ alkyl (e.g. CH₃).
14. The compound of clause 13, wherein R² and R"are arranged *trans* to one another.
15. The compound of any preceding clause, wherein each of R³, R⁵ and R⁶ may be independently selected from H and C₁₋₄ alkyl; optionally wherein each of R³, R⁵ and R⁶ may be independently selected from H and CH₃.
16. The compound of any preceding clause, wherein each R⁷ and R⁸ may be independently selected from H, halo, C₁₋₄ haloalkyl, and NO₂; optionally wherein each R⁷ is H and each R⁸ is independently selected from halo, C₁₋₄ haloalkyl, and NO₂.
17. The compound of any preceding clause, wherein p is 0 or 1.
18. The compound of any preceding clause, wherein n is 0 or 1, optionally wherein n is 1.
19. The compound of clause 1, wherein the compound is selected from: 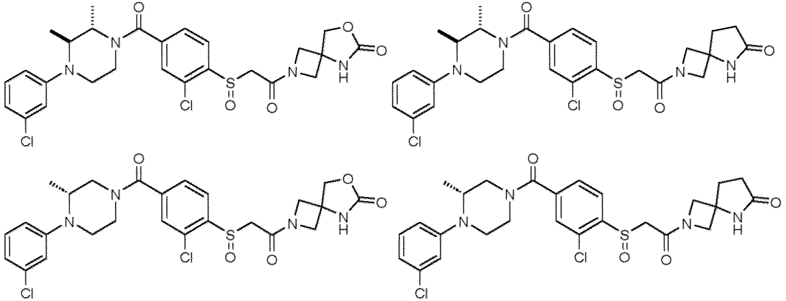 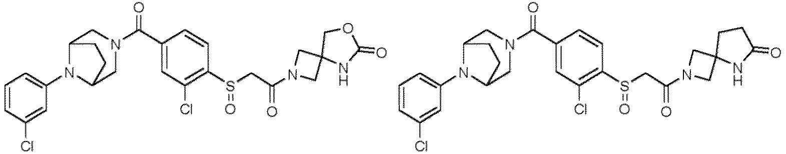 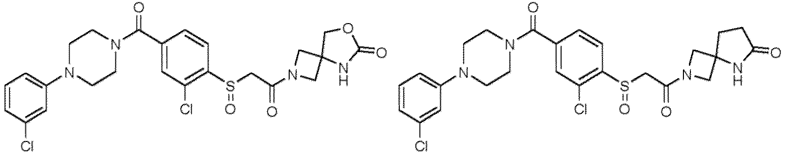 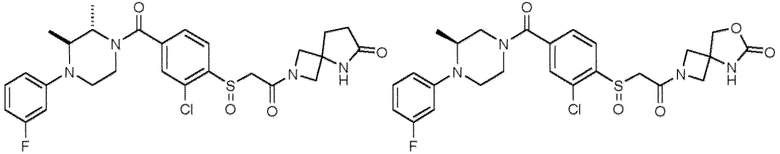 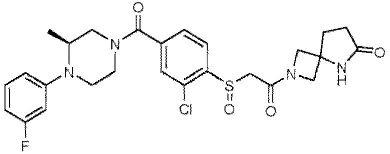 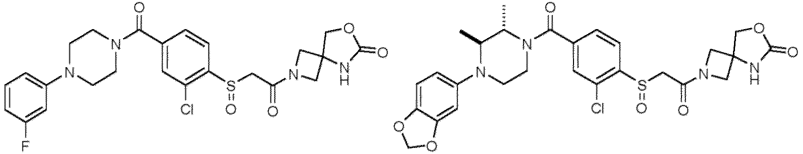 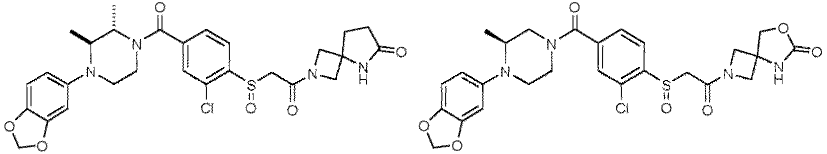 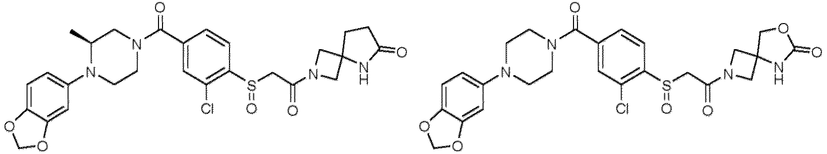 and 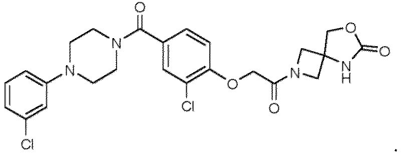
20. A pharmaceutical composition comprising a compound of any preceding clause.
21. The composition of clause 20, further comprising a pharmaceutically acceptable excipient.
22. The compound of any of clauses 1 to 19 or the pharmaceutical composition of clause 20 or clause 21 for use as a medicament.
23. The compound of any of clauses 1 to 19 or the pharmaceutical composition of claim 20 or claim 21 for use in the treatment of a condition which is modulated by monoacylglycerol lipase (MAGL).
24. The compound of any of clauses 1 to 19 or the pharmaceutical composition of clause 20 or clause 21 for use in the treatment of a condition selected from acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.
25. The compound for use according to clause 24, wherein the condition is cancer.
26. The compound for use according to clause 25, wherein the condition is breast cancer, ovarian cancer, melanoma, hepatocellular carcinoma, colorectal cancer, prostate cancer, or nasopharyngeal carcinoma.
27. Use of the compound of any of clauses 1 to 19 or the pharmaceutical composition of clause 20 or clause 21 for the inhibition of monoacylglycerol lipase (MAGL).
28. Use of a compound of any one of clauses 1 to 19 in the manufacture of a medicament for use in the treatment of a condition selected from: acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

### ASSAYS

### Cloning, overexpression, and membrane preparation

Full-length cDNA encoding human MAGL (GenBank ID: BC006230.2; obtained from Source Bioscience) was amplified by PCR and cloned into expression vector pcDNA3.1 in frame with a C-terminal FLAG-tag. All plasmids were isolated from transformed XL10-Gold competent cells (prepared using E. coli transformation buffer set; Zymo Research) using plasmid isolation kits following the supplier's protocol (Qiagen). Constructs were verified by Sanger sequencing (Macrogen).

HEK293T (human embryonic kidney) cells were obtained from ATCC and tested on regular basis for mycoplasma contamination. Cultures were discarded after 2-3 months of use. Cells were cultured at 37 °C under 7% CO₂ in high-glucose DMEM containing phenol red, stable glutamine (GlutaMAX^{™}), 10% (v/v) high iron newborn calf serum (Seradigm), penicillin and streptomycin (200 µg/mL each; Duchefa). Medium was refreshed every 2-3 days and cells were passaged two times a week at 80-90% confluence. One day prior to transfection, HEK293T cells were transferred from confluent 10 cm dishes to 15 cm dishes. Before transfection, medium was refreshed (13 mL). A 3:1 mixture of polyethyleneimine (PEI; 60 µg/dish) and plasmid DNA (20 µg/dish) was prepared in serum-free medium (2 mL) and incubated for 15 min at RT. The mixture was then added dropwise to the cells, after which the cells were grown to confluence in 72 h. Cells were then harvested by suspension in PBS, followed by centrifugation (200 g, 5 min). Cell pellets were flash-frozen in liquid nitrogen and stored at -80 °C.

Cell pellets were thawed on ice and resuspended in lysis buffer A (20 mM HEPES (pH 7.2), 2 mM DTT, 250 mM sucrose, 1 mM MgCl₂, and 25 U/mL benzonase). Suspensions were homogenized by polytron (3 × 7 s, 20,000 rpm, SilentCrusher S; Heidolph, Schwabach, Germany), incubated on ice for 30 min, and subsequently centrifuged at 93,000 g for 30 min at 4°C (Ti70 or Ti70.1 rotor; Beckman Coulter). Pellet was resuspended in storage buffer B (20 mM HEPES (pH 7.2), 2 mM DTT)]. Suspension was homogenized by polytron (1 × 10 s, 20,000 rpm). Protein concentrations were determined with Quick Start Bradford reagent (Bio-Rad, Hilversum, The Netherlands) or Qubit fluorometric quantitation (Life Technologies, Breda, The Netherlands). Membranes were diluted with storage buffer B to the desired concentration, aliquoted, frozen in liquid nitrogen, and stored at -80°C.

### Biochemical evaluation

Assays were performed in HEMNB buffer (50 mM HEPES pH 7.4, 1 mM EDTA, 5 mM MgCl₂, 100 mM NaCl, 0.5% (w/w) BSA) in black, flat-bottom 96-well plates (Greiner). Inhibitors were added from 40× concentrated stock solution in DMSO. MAGL-overexpressing membrane preparations (0.3 µg per well) were incubated with inhibitor for 20 min at RT in a total volume of 100 µL. Next, 100 µL assay mix containing glycerol kinase (GK), glycerol-3-phosphate oxidase (GPO), horse radish peroxidase (HRP), adenosine triphosphate (ATP), Amplifu^{™} Red and 2-arachidonoylglycerol (2-AG) was added. Fluorescence (λₑₓ = 535 nm, λₑₘ = 595 nm) was measured at RT in 5 min intervals for 60 min on a Clariostar (BMG Labtech) plate reader. Final assay concentrations: 1.5 ng/µL MAGL-overexpressing membranes, 0.2 U/mL GK, GPO and HRP, 125 µM ATP, 10 µM Amplifu^{™} Red, 25 µM 2-AG, 5% DMSO, 0.5% ACN in a total volume of 200 µL. For pIC₅₀ determinations, the assay was performed as described above, but with variable inhibitor concentrations. All measurements were performed in N = 2 (individual plates), n = 2 (technical replicates on same plate) or N = 2, n = 4 for controls. Fluorescence values were corrected for the average fluorescence of the negative control (mock-membranes + vehicle). Slopes of the corrected data were determined in the linear interval. The Z'-factor for each assay plate was calculated using the formula Z' = 1 - 3(σ_{pc} + σ_{nc})/(µ_{pc} - µ_{nc}) with σ = standard deviation, µ = mean, pc = positive control and nc = negative control, and plates with Z' ≥ 0.6 were accepted for further analysis. For pIC₅₀ determination, slopes were normalized to the positive control and analysed in a non-linear dose-response analysis with variable slope (GraphPad Prism 9.0). The pIC₅₀ values were converted into pKᵢ values following the Cheng-Prusoff equation²³ IC₅₀ = (([Kᵢ]/K_{M}) × [L]) + Kᵢ. Where K_{M} is the affinity constant of 2-AG (12.5 µM)²⁴ and [L] is the 2-AG concentration.

### Cellular target engagement evaluation

### Cell culture

U-87 MG (human glioblastoma) cells were obtained from ATCC (catalogue number: HBT-14) and were cultured in DMEM (Sigma, D6546), supplemented with 10% fetal calf serum (FCS, Biowest), stable glutamine (glutaMAX^{™}) and penicillin and streptomycin (100 µg/mL), at 37°C and 7% CO₂ in a humidified incubator. Cells were passaged through detachment with trypsin/EDTA in PBS for 3 minutes at RT, followed by the addition of fresh medium. After cell counting, cells were seeded in fresh medium at the appropriate confluency. Cell lines were regularly tested for mycoplasma contamination and consistently tested negative. Cultures were discarded after 2-3 months of use.

### In situ treatments

The *term in situ* is used to describe experiments in which live cell cultures are treated with inhibitors. U-87 MG cells were seeded at 20.000 cells/well in 24-wells plates, 24 h prior to treatment. Culture medium was aspirated and replaced with medium (DMEM supplemented with 2% FCS, glutaMAX, P/S) containing vehicle (0.1% DMSO) or inhibitor (final concentrations: 1 nM - 10 µM) was added. After incubation for 1 h, the MAGL specific fluorescent activity-based probe LEI463-Cy5 (Prokop, S. et al., Nat. Commun., 12, 6505 (2021)), was added as a 6× concentrated stock in medium (final concentration: 10 nM). After incubation for an additional 1 h, the medium was aspirated.

### Cell lysate preparation and gel based ABPP: in situ treatment

Cells were lysed in 30 µL SDS-lysis buffer (50 mM Tris buffer pH 7.0, 2 mM EDTA, 1% SDS). 10 µL 4× Laemmli-buffer (240 mM Tris (pH 6.8), 8% (w/v) SDS, 40% (v/v) glycerol, 5% (v/v) β-mercaptoethanol, 0.04% (v/v) bromophenol blue) was added to the samples and 20 µL of protein was resolved by SDS-PAGE (10% 29:1 acrylamide:bisacrylamide). In-gel fluorescence was detected in the Cy5-channel (700/50 nm) on a ChemiDoc MP imaging system (Bio-Rad). The gel was stained using Coomassie G250 blue staining as a protein loading control. Fluorescence and Coomassie intensities were quantified using ImageLab 6.0 software (Bio-Rad) and normalized log-transformed data was fitted using the variable slope dose-response function in GraphPad Prism 9.0.

### Targeted lipidomics

### Lipidomics sample preparation

2 × 10⁶ U-87 MG cells were seeded 24 h before treatment in 6 cm dishes. Before treatment, cells were washed twice with PBS and then treated with vehicle or compound in 2 mL medium without serum. After 1 h, the medium was aspirated and the cells were harvested in 1250 µL PBS of which 1000 µL was collected in 1.5 mL safe-lock tubes and centrifuged (5 min, 1,000 g). The supernatant was removed, the cell pellets were washed with PBS, centrifuged (5 min, 1,000 g) and the pellets were flash frozen with liquid nitrogen and stored at -80 °C. The remaining 250 µL cell suspension was lysed using sonication (Q700MPX (QSonica), 5 sec, 30% amplitude) and the protein concentration was determined using a Bradford assay (Bio-Rad) for normalization after lipid measurements.

### Lipid extraction

Lipid extraction was performed on ice. Samples were thawed on ice and spiked with 10 µL deuterium labelled internal standard mix and vortexed. Subsequently, 100 µL 0.5 % sodium chloride, 100 µL ammonium acetate buffer (0.1 M, pH 4) and 1 mL methyl tert-butyl ether (MTBE) were added and the samples were thoroughly mixed for 6 min using a Bullet Blender Blue (Next advance Inc., Averill Park, NY, USA) at speed 8, followed by a centrifugation step (16,000g, 11 min, 4 °C). Next, 925 µL of the upper MTBE layer was transferred into a clean 1.5 mL Safe-Lock Eppendorf tube. Samples were dried in a speedvac (Eppendorf, 45 min, 30 °C) and reconstituted in acetonitrile/water (30 µL, 90/10, v/v). The samples were thoroughly mixed for 4 min, followed by a centrifugation step (10,000 g, 4 min, 4 °C) and transferred to a LC-MS vial (9 mm, 1.5 mL, amber screw vial, KG 090188, Screening Devices) with insert (0.1 mL, tear drop with plastic spring, ME 060232, Screening devices). 5 µL of each sample was injected into the LC-MS/MS system.

### Selectivity evaluation

### Preparation of mouse brain proteome

Mouse tissues were isolated according to guidelines approved by the ethical committee of Leiden University. Isolated tissues were thawed on ice, dounce homogenized in lysis buffer A (20 mM HEPES pH 7.2, 2 mM DTT, 1 mM MgCl₂, 2 U/mL Benzonase) and incubated for 15 min on ice. Then, debris was removed by low-speed spin (2,500 g, 1 min, 4 °C) and the supernatant was subjected to ultracentrifugation (100,000 g, 45 min, 4 °C, Beckman Coulter, Type Ti70 rotor) to yield the membrane fraction as a pellet and the cytosolic fraction in the supernatant. The membrane fraction was resuspended in lysis buffer B (20 mM HEPES pH 7.2, 2 mM DTT). The total protein concentration was determined with Quick Start Bradford assay. The obtained membranes and supernatant were flash frozen in liquid nitrogen and stored in small aliquots at -80 °C until use.

### Gel based competitive ABPP

The competitive ABPP assay on mouse brain proteome was performed as previously reported (van Esbroeck, A. C. M. et al., Science (1979) 356, 1084-1087 (2017)). To 19 µL of mouse brain proteome or HSL overexpression membranes (2 mg/mL) was added 0.5 µL of the inhibitor or pure DMSO and incubated for 30 min at RT. Subsequently, 0.5 µL MB064 (final concentration: 250 nM) was added. After 10 minutes of incubation at RT, 0.5 µL FP-Bodipy (final concentration: 100 nM) was added to the proteome sample and incubated for 10 min. The reaction was quenched by adding 10 µL of 4× Laemmli-buffer and 10 µL of quenched reaction mixture was resolved on 10 % acrylamide SDS-PAGE (180V, 75 min). Fluorescence was measured using a Biorad ChemiDoc MP system. Gels were then stained using Coomassie staining and imaged for protein loading control.

### Chemical proteomics

Mouse liver proteome (100 µL, 1.0 mg/mL) was incubated with vehicle (DMSO) or inhibitor (1 µM) in DMSO for 30 minutes at 37 °C. The proteome was labelled with a probe cocktail (2.5 µM MB108 and 5 µM FP-Biotin, 30 minutes, 37 °C). The protein content was isolated by methanol/chloroform precipitation. Precipitation was done by addition of 247 µL water, 465 µL MeOH and 120 µL chloroform with briefly vortexing after MeOH and chloroform addition. The precipitated protein was pelleted by centrifugation (1,500 *g,* 10 min, RT). The upper and lower layer were removed without disturbing the floating pellet. Thereafter, the pellet was resuspended in 500 µL of MeOH and sonicated (Q700MPX (QSonica), 10% amplitude, 2× 10 s). The protein was pelleted by centrifugation (20,000 *g,* 5 min, RT) and the supernatant was removed. The protein pellet was resuspended in 250 µL PBS containing 0.5% SDS and 5 mM DTT, sonicated (Q700MPX (QSonica)10% amplitude, 2× 10 sec), incubated for 15 min at 65 °C while shaking (1000 rpm) and spinned down briefly (600 *g,* 10 sec, RT). After the samples were cooled down to RT, iodoacetamide (15 µL, final concentration: 30 mM) was added, vortexed briefly and incubated for 30 min at RT in the dark. For 24 samples, 240 µL of slurry streptavidin (Thermo Scientific, cat. no. 20361) and 720 µL control agarose bead slurry (Thermo Scientific, cat. no. 26150) was washed with PBS containing 0.5% SDS (2× 6 mL) and PBS (1 × 6 mL) by vortexing and spinning down (3,000 *g*, 2 min). The beads were resuspended in PBS containing 20 mM DTT and divided over 1.5 mL epps, 250 µL per sample. The pulldown sample was transferred to the beads resulting in a volume of 500 µL (final SDS concentration: 0.25%). The samples were rotated with an overhead shaker at room temperature for 3 h.

After centrifugation (3,500 *g*, 2 min) and removal of the supernatant, the beads were consecutively washed with 0.5% SDS in PBS (4× 1 mL) and PBS (5× 1 mL), each time centrifuging (3,500 *g*, 2 min) and pouring out the supernatant. Per sample, 100 µL on-bead digestion buffer (100 mM Tris (pH 8.0), 100 mM NaCl, 1 mM CaCl₂, 2% v/v acetonitrile) containing 0.25 µg trypsin (0.5 µg/µL trypsin (Promega, V5111), 0.1 mM HCl) was added. Proteins were digested at 37 °C with shaking (1000 rpm) overnight. Formic acid (10%, 100 µL) was added to each sample and spinned down (3,000 *g*, 2 min). Samples were purified using StageTips. Each StageTip was conditioned with MeOH (50 µL, centrifugation: 2 min, 300 g), followed by StageTip solution B (50 µL, 80% v/v acetonitrile, 0.5% v/v formic acid in MilliQ, centrifugation for 2 min, 300 *g*) and StageTip solution A (50 µL, 0.5% v/v formic acid in MilliQ, centrifugation for 2 min, 300 *g*). Next, the samples were loaded, centrifuged (2 min, 600 *g*) and the peptides on the StageTip were washed with StageTip solution A (100 µL, centrifugation for 2 min, 600 *g*). The StageTips were transferred to a new 1.5 mL low binding epp and the peptides were eluted with StageTip solution B (100 µL, centrifugation for 2 min, 600 g). The solvents were evaporated to dryness in a SpeedVac concentrator at 45 °C for 3 h. Samples were reconstituted in LCMS solution (50 µL, 3% v/v acetonitrile, 0.1% v/v formic acid, 1 µM yeast enolase peptide digest (Waters, 186002325) in MilliQ).

The peptides were separated on an UltiMate 3000 RSLCnano system set in a trap-elute configuration with a nanoEase M/Z Symmetry C18 100 Å, 5 µm, 180 µm × 20 mm (Waters) trap column for peptide loading/retention and nanoEase M/Z HSS C18 T3 100 Å, 1.8 µm, 75 µm × 250 mm (Waters) analytical column for peptide separation. The column was kept at 40 °C in a column oven. Samples (5 µL/sample) were injected on the trap column at a flow rate of 15 µL/min for 2 min with 99% mobile phase A (0.1% FA in ULC-MS grade water (Biosolve)), 1% mobile phase B (0.1% FA in ULC-MS grade acetonitrile (Biosolve)) eluent. The 85 min LC method, using mobile phase A and mobile phase B controlled by a flow sensor at 0.3 µL/min with average pressure of 400-500 bar (5500-7000 psi), was programmed as gradient with linear increment to 1% B from 0 to 2 min, 5% B at 5 min, 22% B at 55 min, 40% B at 64 min, 90% B at 65 to 74 min and 1% B at 75 to 85 min. The eluent was introduced by electro-spray ionization (ESI) via the nanoESI source (Thermo) using stainless steel Nano-bore emitters (40 mm, OD 1/32", ES542, Thermo Scientific). The QExactive HF was operated in positive mode with data dependent acquisition without the use of lock mass, default charge of 2+ and external calibration with LTQ Velos ESI positive ion calibration solution (88323, Pierce, Thermo) every 5 days to less than 2 ppm. The tune file for the survey scan was set to scan range of 350 - 1400 m/z, 120,000 resolution (m/z 200), 1 microscan, automatic gain control (AGC) of 3e6, max injection time of 100 ms, no sheath, aux or sweep gas, spray voltage ranging from 1.7 to 3.0 kV, capillary temp of 250 °C and an S-lens value of 80. Data was analysed with MaxQuant 2.0 (unique peptides ≥2), KNIME and GraphPad Prism 9.0. Statistical analysis: multiple unpaired t-test with false discovery rate (FDR) 10%.

### EXAMPLES

### Materials and Methods

Reagents were either acquired from Sigma-Aldrich, Acros and Merck or produced in earlier research projects and used without further purification unless specified otherwise. Moisture sensitive reactions were performed under a nitrogen atmosphere using anhydrous solvents dried over activated molecular sieves (3 or 4 Å). Traces of water were removed from starting materials through co-evaporation with toluene.

Thin layer chromatography (TLC) was performed using TLC Silica gel 60 F₂₄₅ on aluminium sheets (Merck). Compounds were visualized using an ultraviolet lamp (λmax = 254 nm), KMnO₄ staining (K₂CO₃ (25 g), KMnO₄ (5 g), H₂O (1 L)) or ninhydrin staining (ninhydrin (200 mg), AcOH (5 mL) and EtOH (100 mL).

The crude compounds were purified by either flash column chromatography from Screening Devices silica gel 60, or automated flash column chromatography from Biotage Isolera Flash Chromatography Systems and pre-packed cartridges of Screening Devices UltraPure Irregular Silica Gel (40 - 63 µm, 60 Å).

Preparative HPLC was performed on a Waters AutoPurification HPLC/MS apparatus, equipped with a Gemini prep column 5 µm 18C 110 Å (150 × 21.1 mm), Waters 2767 Sample manager, Waters 2545 Binary gradient module, Waters SFO System Fluidics Organizer, Waters 515 HPLC pump M, Waters 515 HPLC pump L attached to a Waters SQ detector Acquity Ultra Performance LC.

LC-MS measurements were performed on a Thermo Finnigan LCQ Advantage Max ion-trap mass spectrometer (ESI+), coupled to a Surveyor HPLC system (Thermo Finnigan) or a Thermo Vanquish Focused UHPLC⁺ system, equipped with a C18 column and coupled to a Thermo LCQ Fleet ion-trap mass-spectrometer. Both LC-MS systems were equipped with a standard C18 (Gemini, 4.6 × 50 mm, 3 µm particle size, Macherey-Nagel) analytical column. Eluents A: H₂O, B: ACN, C: 1% aq. TFA, gradients: 10-90% or a 0-50% gradient of ACN in water with 0.1% TFA. All compounds used for biological experiments were determined to be >95% pure by integrating UV intensity of spectra generated by either of the LCMS instruments.

High-resolution mass spectra (HRMS) were recorded on a Thermo Scientific LTQ Orbitrap XL. HRMS spectra were recorded on a Thermo Fisher Q exactive HF orbitrap spectrometer, coupled to an Ultimate 3000 Nano LCEN ESI probe. Calculated and observed m/z values refer to the exact mass of the [M+H] species.

A Bruker AV-(300, 400, 500 or 600) Cryomagnet was used to obtain proton (¹H)-NMR and carbon (¹³C)-NMR. Chemical shifts (δ) are reported in parts per million (ppm) downfield of tetramethylsilane (TMS) or solvent resonance as the internal standard (CDCl₃: δ 7.26 for ¹H, δ 77.16 for ¹³C; MeOD: δ 3.31 for ¹H, δ 49.00 for ¹³C; DMSO-d₆: 2.50 for ¹H, 39.52 for ¹³C). Splitting patterns reported in an abbreviated manner (s = singlet, d = doublet, t = triplet, q = quartet, and m = multiplet, br = broad), coupling constants (*J*) are quoted in Hertz (Hz). Peak assignments were aided by 2D COSY, HSQC, and HMBC experiments. MestReNova software (version 14.0.1) was used for the analysis of the NMR spectra.

PerkinElmer ChemDraw Professional (version 22.2) was used to draw molecular structures presented in this work.

### Example 1: Synthesis of Intermediates

### Intermediate 1: 2,5-Diazaspiro[3.4]octan-6-one bis(4-methylbenzenesulfonate)

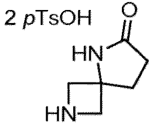

A solution of Intermediate 5 (1.27 g, 5.61 mmol, 1 eq.) and *p*TsOH.H₂O (2.42 g, 12.7 mmol, 2 eq.) in EtOAc (55 mL) was heated to 75 °C for 3 h. After being cooled to RT, the resulting precipitates were collected, washed with Et₂O, and dried in vacuo to afford the product as a white solid (2.08 g, 4.42 mmol, 79%). ¹H-NMR (400 MHz, MeOD) δ 7.72-7.74 (d, *J* = 8.2 Hz, 4H), 7.23-7.25 (d, *J* = 8.2 Hz, 4H), 5.32 (s, 4H), 4.13-4.49 (m, 4H), 2.38-2.62 (m, 4H), 2.35 (s, 6H). ¹³C NMR (101 MHz, MeOD) δ 180.10, 142.64, 142.04, 129.94, 126.76, 59.92, 59.81, 59.44, 33.06, 30.49, 21.30.

### Intermediate 2: 7-Oxa-2,5-diazaspiro[3.4]octan-6-one 4-methylbenzenesulfonate

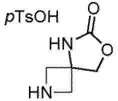

A solution of Intermediate 6 (925 mg, 4.05 mmol, 1 eq.) and *p*TsOH·H₂O (1.54 g, 8.11 mmol, 2 eq.) in EtOAc (40 mL) was heated to 75 °C for 3 h. The mixture was cooled to RT and the resulting precipitates were collected, washed with Et₂O, and dried in vacuo to afford the product as a white solid (960 mg, 3.20 mmol, 79%). ¹H NMR (400 MHz, MeOD) δ 7.77 - 7.67 (m, 2H), 7.26 (d, *J* = 8.0 Hz, 2H), 4.70 - 4.48 (m, 3H), 4.41 - 4.22 (m, 3H), 2.38 (s, 3H). ¹³C NMR (101 MHz, MeOD) δ 161.34, 142.62, 141.98, 129.95, 126.93, 75.17, 70.35, 59.81, 54.62, 21.31.

### Intermediate 3: tert-Butyl 3-(3-methoxy-3-oxopropyl)-3-nitroazetidine-1-carboxylate

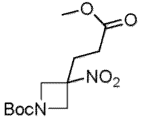

To a cooled (0 °C) solution of *tert*-butyl 3-nitroazetidine-1-carboxylate (2.00 g, 9.89 mmol, 1 eq.) in MeOH (20 mL) were added methyl acrylate (1.00 mL, 11.9 mmol, 1.2 eq.) and K₂CO₃ (1.64 g, 11.9 mmol, 1.2 eq.) and the resulting mixture was stirred at the same temperature for 3 h. After concentration in vacuo, the residue was quenched with sat. NH₄Cl and extracted with EtOAc. The organic layer was washed with brine, dried (Na₂SO₄), filtered, and concentrated in vacuo. The crude compound was purified by column chromatography (25% EtOAc in pentane) to afford the product as a colourless oil (1.86 g, 6.45 mmol, 65%). ¹H NMR (400 MHz, CDCl₃) δ 4.45 (d, *J* = 10.1 Hz, 2H), 4.07 (d, *J* = 2.6 Hz, 2H), 3.69 (s, 3H), 2.64 - 2.45 (m, 2H), 2.45 - 2.31 (m, 2H), 1.45 (s, 9H). ¹³C NMR (101 MHz, CDCl₃) δ 171.58, 155.47, 82.02, 80.58, 59.11, 51.91 (br), 31.24, 28.06.

### Intermediate 4: tert-Butyl 3-(hydroxymethyl)-3-nitroazetidine-1-carboxylate

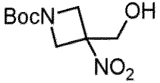

To a solution of *tert*-butyl 3-nitroazetidine-1-carboxylate (2.00 g, 9.89 mmol, 1 eq.) in ACN (20 mL) were added formaldehyde solution (30% (w/v), 1.89 mL, 19.78 mmol, 2 eq.) and TEA (1.52 mL, 10.9 mmol, 1.1 eq.) and the resulting mixture was stirred for 1 h. After concentration in vacuo, the crude compound was purified by column chromatography (80% EtOAc in pentane) to afford the product as a white solid (2.05 g, 8.81 mmol, 89%). ¹H NMR (400 MHz, CDCl₃) δ 4.41 (d, *J* = 10.2 Hz, 2H), 4.20 - 4.08 (m, 4H), 3.97 (t, *J* = *6.2* Hz, 1H), 1.45 (s, 9H). ¹³C NMR (101 MHz, CDCl₃) δ 156.14, 89.67, 82.47, 81.33, 64.55, 28.27.

### Intermediate 5: tert-Butyl 6-oxo-2,5-diazaspiro[3.4]octane-2-carboxylate

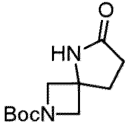

To a cooled (-10 °C) solution of Intermediate 3 (1.86 g, 6.45 mmol, 1 eq.) and nickel(II) chloride.6H₂O (1.50 g, 6.45 mmol, 1 eq.) in MeOH (65 mL) was added sodium borohydride (1.20 g, 32.3 mmol, 5 eq.) and the reaction mixture was stirred at the same temperature for 2 h. Aq. K₂CO₃ (1 mL) was added, and the resulting mixture was stirred at RT for 2 h. The reaction mixture was filtered through a Celite^{®} pad, and the filtrate was concentrated in vacuo. The residue was diluted with water and extracted with EtOAc. The organic layer was dried (MgSO₄), filtered, and concentrated in vacuo to afford the product as a white solid (1.27 g, 5.63 mol, 87%). ¹H NMR (400 MHz, CDCl₃) δ 7.81 (br, 1H), 4.01 (dd, *J* = 9.7, 8.6 Hz, 4H), 2.38 (h, *J* = 6.7 Hz, 4H), 1.44 (s, 9H). ¹³C NMR (101 MHz, CDCl₃) δ 177.74, 156.09, 80.00, 62.41 (br), 55.47, 33.37, 30.26, 28.36.

### Intermediate 6: tert-Butyl 6-oxo-7-oxa-2,5-diazaspiro[3.4]octane-2-carboxylate

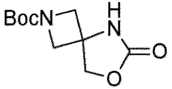

A solution of Intermediate 4 (2.05 g, 8.81 mmol, 1 eq.) and 10% Pd-C (411 mg, 2.84 mmol, 0.3 eq.) in THF (16 mL) was hydrogenated under balloon pressure for 72 h. The catalyst was removed by filtration, and the filtrate was concentrated in vacuo to give the amine product as a clear oil. The amine was subjected to the next reaction without further purification. To a cooled (-10 °C) solution of the crude amine (2.01 g, 8.81 mmol) and DIPEA (2.27 mL, 17.62 mmol, 2 eq.) in THF (33 mL) was added triphosgene (1.31 g, 4.41 mmol, 0.5 eq.) and the mixture was stirred at the same temperature for 30 min. The mixture was diluted with water and extracted with EtOAc. The organic layer was separated, washed with brine, dried (MgSO₄), and concentrated in vacuo. The residue was purified by column chromatography (60-80% EtOAc in hexane) to give the product as a clear oil (0.93 g, 4.1 mmol, 2 steps 46% from Intermediate 4). NMR spectra show a mixture of rotamers. ¹H NMR (400 MHz, CDCl₃) δ 4.58 - 4.42 (m, 3H), 4.24 - 3.99 (m, 2H), 3.94 (d, *J =* 9.7 Hz, 1H), 1.43 (s, 9H). ¹³C NMR (101 MHz, CDCl₃) δ 158.68, 157.23, 156.14, 156.07, 80.60, 80.53, 74.80, 70.38, 58.59, 53.92, 28.27.

### Intermediate 7: 2-((4-(tert-Butoxycarbonyl)-2-chlorophenyl)sulfinyl)acetic acid

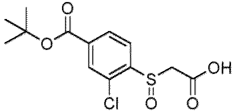

To a solution of *tert*-butyl 3-chloro-4-((2-ethoxy-2-oxoethyl)sulfinyl)- benzoate (654 mg, 1.89 mmol) in MeOH (10.5 mL) were added TEA (10.5 mL) and water (10.5 mL). The reaction mixture was stirred for 2.5 h, after which TLC analysis confirmed reaction completion and product formation. Subsequently, the solution was acidified to pH 2 with a 3 M HCl solution and extracted with EtOAc (5x), after which all organic layers were dried (MgSO₄), filtered and concentrated under reduced pressure. The remaining oil was purified by silica gel column chromatography (1-5% MeOH in DCM), to afford the product as a viscous oil (561 mg, 1.76 mmol, 93%). ¹H NMR (400 MHz, CDCl₃) δ 8.14 (dd, *J* = 8.2, 1.6 Hz, 1H), 8.03 (d, *J* = 1.5 Hz, 1H), 8.00 (d, *J =* 8.3 Hz, 1H), 4.13 (d, *J* = 14.4 Hz, 1H), 3.77 (d, *J =* 14.8 Hz, 1H), 1.61 (s, 9H). ¹³C NMR (101 MHz, CDCl₃) δ 166.80, 163.49, 143.36, 136.86, 131.05, 130.11, 129.14, 126.66, 82.92, 55.94, 28.21.

### Intermediate 8: tert-Butyl 3-chloro-4-((2-oxo-2-(6-oxo-2,5-diazaspiro[3.4]octan-2-yl)ethyl)sulfinyl)benzoate

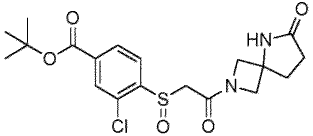

The title compound was synthesised from Intermediate 7 (1.61 g, 5.30 mmol, 1.2 eq.) and Intermediate 1 (2.08 g, 4.42 mmol, 1 eq.) according to general procedure B. Column chromatography (3% MeOH in DCM) yielded the product as a white solid (1.00 g, 2.43 mmol, 55%). NMR spectra show a mixture of rotamers. ¹H NMR (400 MHz, CDCl₃) δ 8.14 - 8.09 (m, 1H), 8.01 - 7.92 (m, 2H), 4.47 (ddd, *J* = 9.1, 5.3, 1.1 Hz, 1H), 4.36 - 4.17 (m, 2H), 4.11 (ddd, *J* = 14.2, 10.5, 1.2 Hz, 1H), 3.94 (d, *J* = 13.6 Hz, 2H), 3.59 (dd, *J* = 23.3, 13.5 Hz, 1H), 2.40 (d, *J* = 2.7 Hz, 4H), 1.61 (s, 9H). ¹³C NMR (101 MHz, CDCl₃) δ 177.21, 176.98, 163.95, 163.37, 163.30, 163.14, 145.14, 144.94, 144.54, 136.24, 136.22, 130.63, 130.61, 129.86, 129.83, 128.80, 128.73, 128.54, 126.22, 126.19, 82.51, 63.88, 63.14, 63.00, 61.75, 61.66, 56.18, 56.09, 55.46, 55.43, 33.08, 30.37, 30.13, 30.11, 28.03.

### Intermediate 9: tert-Butyl 3-chloro-4-((2-oxo-2-(6-oxo-7-oxa-2,5-diazaspiro[3.4]octan-2-yl)ethyl)sulfinyl)benzoate

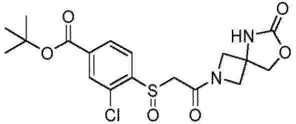

The title compound was synthesised from Intermediate 7 (904 mg, 2.98 mmol, 1.2 eq.) and Intermediate 2 (747 mg, 2.49 mmol, 1 eq.) according to general procedure B. Column chromatography (10-50% acetone in toluene) obtained the product as an off-white solid (849 mg, 2.06 mmol, 83%). NMR spectra show a mixture of rotamers. ¹H NMR (400 MHz, acetone-d₆, 298 K) δ 8.16 (dt, *J* = 8.1, 1.6 Hz, 1H), 8.02 - 7.96 (m, 2H), 7.48 (br s, 1H) 4.64 - 4.57 (m, 2H), 4.57 - 4.47 (m, 2H), 4.25 - 4.13 (m, 2H), 4.04 (dd, *J* = 14.0, 8.2 Hz, 1H), 3.62 (dd, *J* = 14.0, 6.6 Hz, 1H), 1.61 (s, 9H). ¹H NMR (500 MHz, acetone-d₆, 325.6 K) δ 8.16 (dd, *J* = 8.1, 1.5 Hz, 1H), 8.01 (d, *J* = 6.5 Hz, 1H), 8.00 (s, 1H), 7.41 (br s, 1H), 4.60 (s, 2H), 4.55 (d, *J* = 6.3 Hz, 2H), 4.22 (s, 2H), 4.05 (d, *J* = 10.8 Hz, 1H), 3.65 (d, *J* = 13.9 Hz, 1H), 1.63 (s, 9H). ¹³C NMR (126 MHz, acetone-d₆) δ 163.29, 163.26, 157.19, 146.78, 146.72, 136.06, 130.10, 129.76, 128.65, 128.60, 126.54, 126.52, 82.05, 73.74, 73.73, 63.37, 63.33, 63.30, 63.27, 60.85, 60.83, 56.96, 56.64, 42.93, 27.26.

### Intermediate 10: 3-Chloro-4-((2-oxo-2-(6-oxo-2,5-diazaspiro[3.4]octan-2-yl)ethyl)sulfinyl)benzoic acid

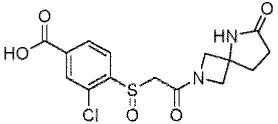

The title compound was synthesised from Intermediate 8 (242 mg, 0.57 mmol) according to general procedure D. Column chromatography (10% MeOH in DCM, 1% AcOH) obtained the product as a white solid (177 mg, 0.480 mmol, 84%). ¹H NMR (400 MHz, MeOD) δ 8.19 (dd, *J* = 8.1, 1.5 Hz, 1H), 8.06 (dd, *J* = 3.2, 1.4 Hz, 1H), 7.97 (dd, *J* = 8.1, 2.8 Hz, 1H), 4.43 - 4.31 (m, 2H), 4.20 - 4.09 (m, 3H), 3.77 (dd, *J* = 14.2, 6.7 Hz, 1H), 2.46 -2.35 (m, 4H). ¹³C NMR (101 MHz, MeOD) δ 179.90, 167.20, 165.41, 146.24, 146.12, 136.77, 131.90, 131.30, 131.27, 130.24, 127.60, 127.52, 64.98, 62.47, 57.16, 56.99, 56.95, 56.68, 33.62, 33.58, 30.96.

### Intermediate 11: 3-Chloro-4-((2-oxo-2-(6-oxo-7-oxa-2,5-diazaspiro[3.4]octan-2-yl)ethyl)sulfinyl)benzoic acid

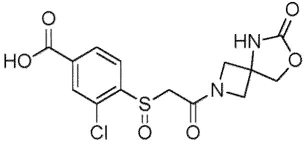

The title compound was synthesised from Intermediate 9 (260 mg, 0.610 mmol) according to general procedure D. Column chromatography (10% MeOH in DCM, 1% AcOH) obtained the compound as a white solid (158 mg, 0.430 mmol, 70%). ¹H NMR (600 MHz, DMSO-d₆) δ 8.49 (d, *J* = 15.4 Hz, 1H), 8.14 (dt, *J* = 8.1, 1.8 Hz, 1H), 7.99 (br s, *J* = 1.6 Hz, 1H), 7.93 (dd, *J* = 8.0, 1.8 Hz, 1H), 4.50 (dd, *J* = 9.4, 2.1 Hz, 1H), 4.45 (t, *J* = 9.6 Hz, 1H), 4.38 - 4.24 (m, 3H), 4.12 (ddd, *J* = 10.8, 5.9, 1.4 Hz, 1H), 3.99 (dt, *J* = 10.8, 1.6 Hz, 1H), 3.67 (dd, *J* = 19.4, 16.9 Hz, 1H). ¹³C NMR (151 MHz, DMSO-d₆) δ 172.01, 165.68, 163.55, 157.34, 136.92, 130.08, 129.26, 128.86, 126.56, 73.10, 62.85, 60.78, 53.52, 40.04.

### Intermediate 12: (R)-4-(3-Chlorophenyl)-3-methylpiperazine

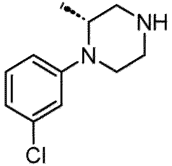

The title compound was synthesised from Intermediate 19 (63 mg, 0.20 mmol) according to general procedure E. Column chromatography (5% MeOH in DCM, 1% TEA) yielded the product as a yellow oil (39 mg, 0.18 mmol, 90%). ¹H NMR (300 MHz, CDCl₃) δ 7.13-7.18 (t, *J =* 8.1 Hz, 1H), 6.85 (t, *J* =2.2 Hz, 1H), 6.77 (td, *J* = 7.3, 1.8 Hz, 2H), 3.77-3.86 (m, 1H), 2.85-3.19 (m, 6H), 2.14 (s, 1H), 1.08 (d, *J* = 6.6 Hz, 3H). ¹³C NMR (300 MHz, CDCl₃) δ : 151.75, 135.09, 128.93, 118.67, 116.45, 113.99, 53.04, 50.82, 45.73, 44.34, 12.26.

### Intermediate 13: 8-(3-Chlorophenyl)-3,8-diazabicyclo[3.2.1]octane

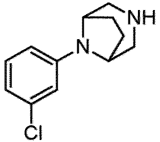

The title compound was synthesised from Intermediate 20 (93 mg, 0.29 mmol) according to general procedure E. The product was obtained as an orange oil and used directly in the next step without further purification (53 mg, 0.24 mmol, 83%). ¹H NMR (400 MHz, CDCl₃) δ 7.12 (t, *J* = 8.1 Hz, 1H), 6.73 (t, *J* = 2.2 Hz, 1H), 6.61-6.68 (m, 2H), 4.07 (br s, 2H), 3.19 (d, *J* = 11.9 Hz, 2H), 2.58 (d, *J* = 11.8 Hz, 2H), 1.94-2.09 (m, 4H). ¹³C NMR (400 MHz, CDCl₃) δ 148.16, 135.41, 130.61, 117.14, 115.07, 113.34, 56.00, 47.77, 27.27.

### Intermediate 14: 4-(3-Chlorophenyl)piperazine

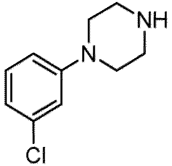

The title compound was synthesised from Intermediate 21 (79 mg, 0.27 mmol) according to general procedure E. Column chromatography (5% MeOH in DCM, 1% TEA) yielded the product as a yellow oil (48 mg, 0.25 mmol, 92%). ¹H-NMR (400 MHz, CDCl₃) *δ* 7.17 (t, *J* = 8.2 Hz, 1H), 6.78-6.88 (m, 3H), 3.05-3.18 (m, 8H). ¹³C-NMR (400 MHz, CDCl₃) δ 152.28, 138.42, 130.17, 119.68, 116.07, 114.20, 49.58, 45.75.

### Intermediate 15: (R)-1-(3-Fluorophenyl)-2-methylpiperazine

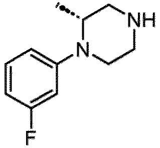

The title compound was synthesised from Intermediate 22 (126 mg, 0.430 mmol) according to general procedure E. Column chromatography (0-10% MeOH in DCM, 1% TEA) yielded the product as a pale-yellow oil (68 mg, 0.35 mmol, 82%). ¹H NMR (400 MHz, CDCl₃) δ 7.19 (td, *J* = 8.2, 7.0 Hz, 1H), 6.66 (ddd, *J* = 8.3, 2.4, 0.8 Hz, 1H), 6.62 - 6.50 (m, 2H), 3.84 (qt, *J* = 6.7, 3.4 Hz, 1H), 3.66 (br s, 1H), 3.25-2.88 (m, 6H), 1.10 (d, *J* = 6.7 Hz, 3H). ¹³C NMR (101 MHz, CDCl₃) δ 164.05 (d, *J* = 243.2 Hz), 152.12 (d, *J* = 9.7 Hz, 130.26 (d, *J* = 10.0 Hz), 112.20 (d, *J* = 2.4 Hz), 106.12 (d, *J* = 21.4 Hz), 103.73 (d, *J* = 24.7 Hz), 50.90, 50.66, 45.81, 44.06, 12.43.

### Intermediate 16: 1-(3-Fluorophenyl)piperazine

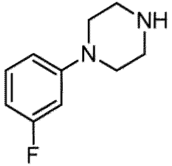

The title compound was synthesised from Intermediate 23 (367 mg, 1.31 mmol) according to general procedure E. Column chromatography (0-10% MeOH in DCM, 1% TEA) partially isolated the free amine as a pale-yellow oil (74 mg, 0.41 mmol, 32%). ¹H NMR (400 MHz, CDCl₃) δ 7.19 (td, *J* = 8.2, 7.0 Hz, 1H), 6.67 (dd, *J* = 8.2, 2.4 Hz, 1H), 6.59 (dt, *J* = 12.4, 2.4 Hz, 1H), 6.53 (ddt, *J* = 9.9, 2.9, 0.7 Hz, 1H), 3.21 - 3.12 (m, 4H), 3.06 - 2.96 (m, 4H). ¹³C NMR (101 MHz, CDCl₃) 163.94 (d, *J* = 243.1 Hz), 153.45 (d, *J* = 9.7 Hz), 130.19 (d, *J* = 10.0 Hz), 111.27 (d, *J* = 2.5 Hz), 105.99 (d, *J* = 21.5 Hz), 102.79 (d, *J* = 25.0 Hz), 77.47, 49.79, 45.97.

### Intermediate 17: (R)-1-(Benzo[d][1,3]dioxol-5-yl)-2-methylpiperazine

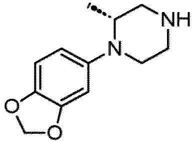

The title compound was synthesised from Intermediate 24 (157 mg, 0.490 mmol) according to general procedure E. Column chromatography (0-10% MeOH in DCM, 1% TEA) yielded the product as a pink oil (98 mg, 0.44 mmol, 90%). NMR spectra show a mixture of rotamers. ¹H NMR (400 MHz, CDCl₃) δ 6.73 (d, *J* = 8.3 Hz, 1H), 6.62 (d, *J* = 2.2 Hz, 1H), 6.48 (dd, *J* = 8.3, 2.3 Hz, 1H), 5.96 - 5.88 (m, 2H), 3.91 (br s, 1H), 3.32 (qt, *J =* 6.4, 3.3 Hz, 1H), 3.15 (dd, *J* = 12.2, 3.3 Hz, 1H), 3.10 - 2.89 (m, 4H), 2.77 (dd, *J* = 12.2, 6.6 Hz, 1H), 0.93 (d, *J =* 6.4 Hz, 3H). ¹³C NMR (101 MHz, CDCl₃) δ 148.13, 146.25, 143.10, 114.19, 108.18, 103.41, 101.04, 53.87, 52.06, 50.48, 46.13, 14.68.

### Intermediate 18: 1-(Benzo[d][1,3]dioxol-5-yl)piperazine

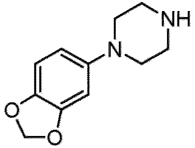

The title compound was synthesised from Intermediate 25 (211 mg, 0.690 mmol) according to general procedure E. Column chromatography (0-10% MeOH in DCM, 1% TEA) yielded the product as a pink oil (91 mg, 0.44 mmol, 64%). ¹H NMR (400 MHz, CDCl₃) δ 6.73 (d, *J* = 8.4 Hz, 1H), 6.57 (d, *J* = 2.4 Hz, 1H), 6.37 (dd, *J* = 8.4, 2.4 Hz, 1H), 5.90 (s, 2H), 3.05 (s, 8H), 2.94 (br s, 1H). ¹³C NMR (101 MHz, CDCl₃) δ 148.27, 147.84, 141.75, 109.27, 108.23, 100.96, 100.17, 51.88, 46.03.

### Intermediate 19: tert-Butyl (R)-4-(3-chlorophenyl)-3-methylpiperazine-1-carboxylate

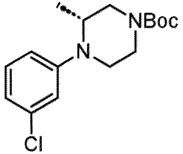

The title compound was synthesised from *tert*-butyl (*R*)-3-methylpiperazine-1-carboxylate (104 mg, 0.520 mmol) and 1-bromo-3-chlorobenzene (100 mg, 0.520 mmol) according to general procedure C. Column chromatography (10% Et₂O in pentane) yielded the product as a yellow oil (63 mg, 0.20 mmol, 39%). ¹H NMR (400 MHz, CDCl₃) *δ* 7.16 (t, *J=* 8.5 Hz, 1H), 6.73-6.84 (m, 3H), 3.83-4.10 (m, 3H), 3.03-3.30 (m, 4H), 1.47 (s, 9H), 1.02 (d, *J=* 6.4 Hz, 3H). ¹³C NMR (400 MHz, CDCl₃) *δ* 155.67, 151.23, 135.72, 128.27, 118.70, 116.00, 114.17, 80.93, 51.31, 43.46, 42.46, 30.41, 28.52.

### Intermediate 20: tert-Butyl 8-(3-chlorophenyl)-3,8-diazabicyclo[3.2.1]octane-3-carboxylate

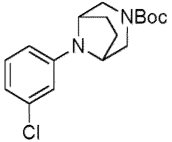

The title compound was synthesised from *tert*-butyl 3,8-diazabicyclo[3.2.1]octane-3-carboxylate (110 mg, 0.520 mmol) and 1-bromo-3-chlorobenzene (100 mg, 0.520 mmol) according to general procedure C. Column chromatography (10% Et₂O in pentane) yielded the compound as clear crystals (131 mg, 0.410 mmol, 78%). ¹H NMR (400 MHz, CDCl₃) δ 7.13 (t, *J* = 8.2 Hz, 1H), 6.75 (t, *J* = 2.5 Hz, 1H), 6.69-6.71 (m, 1H), 6.63-6.66 (m, 1H), 4.14 (d, *J* = 21.1 Hz, 2H), 3.73 (d, *J =* 12.7 Hz, 1H), 3.59 (d, *J =* 12.9 Hz, 1H), 3.27 (d, J = 12.7 Hz, 1H), 3.17 (d, *J* = 12.8 Hz, 1H), 1.97-2.06 (m, 2H), 1.79-1.89 (m, 2H), 1.44 (s, 9H). ¹³C NMR (400 MHz, CDCl₃) δ 156.18, 147.61, 135.49, 130.70, 117.78, 115.45, 113.67, 79.86, 54.63, 54.00, 46.67, 45.46, 28.50, 27.11.

### Intermediate 21: tert-Butyl 4-(3-chlorophenyl)piperazine-1-carboxylate

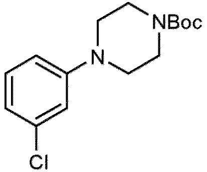

The title compound was synthesised from tert-butyl piperzine-1-carboxylate (97 mg, 0.52 mmol) and 1-bromo-3-chlorobenzene (100 mg, 0.620 mmol) according to general procedure C. Column chromatography (10% Et₂O in pentane) yielded the compound as clear crystals (68 mg, 0.23 mmol, 44%). ¹H NMR (400 MHz, CDCl₃) δ 7.16 (t, J = 8.1 Hz, 1H), 8.87 (t, J = 2.3 Hz, 1H), 6.81-6.84 (m, 1H), 6.76-6.79 (m, 1H), 3.56 (t, J = 5.2 Hz, 4H), 3.12 (t, J = 5.2 Hz, 4H), 1.48 (s, 9H). ¹³C NMR (400 MHz, CDCl₃) δ 154.70, 152.34, 135.03, 130.17, 119.29, 117.75, 113.88, 80.53, 48.92, 29.76, 28.85

### Intermediate 22: tert-Butyl (R)-4-(3-fluorophenyl)-3-methylpiperazine-1-carboxylate

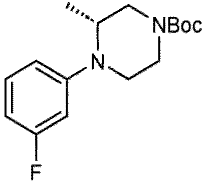

The title compound was synthesised from *tert*-butyl (*R*)-3-methylpiperazine-1-carboxylate (338 mg, 1.69 mmol) and 1-chloro-3-fluorobenzene (200 mg, 1.53 mmol) according to general procedure C. Column chromatography (0-30% Et₂O in pentane) yielded the product as a yellow oil (125 mg, 0.430 mmol, 28%). ¹H NMR (300 MHz, CDCl₃) δ 7.18 (td, *J* = 8.3, 7.1 Hz, 1H), 6.62 (ddd, *J* = 8.4, 2.4, 0.9 Hz, 1H), 6.59 - 6.40 (m, 2H), 3.96 - 3.73 (m, 2H), 3.37 - 2.97 (m, 5H), 1.49 (s, 9H), 1.03 (d, *J* = 6.4 Hz, 3H). ¹³C NMR (75 MHz, CDCl₃) δ 164.07 (d, *J* = 243.1 Hz), 155.16, 151.80 (d, *J* = 9.8 Hz), 130.31 (d, *J* = 10.1 Hz), 111.60, 105.81 (d, *J* = 21.5 Hz), 103.17 (d, *J* = 25.0 Hz), 79.93, 51.23, 42.75, 28.50, 12.32.

### Intermediate 23: tert-Butyl 4-(3-fluorophenyl)piperazine-1-carboxylate

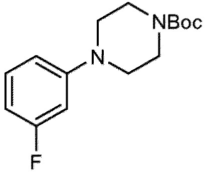

The title compound was synthesised from *tert*-butyl piperazine-1-carboxylate (471 mg, 2.53 mmol) and 1-chloro-3-fluorobenzene (300 mg, 2.30 mmol), according to general procedure C. Column chromatography (0-20% Et₂O in pentane) yielded the product as an orange oil (397 mg, 1.42 mmol, 62%). ¹H NMR (400 MHz, CDCl₃) δ 7.20 (td, *J* = 8.2, 6.9 Hz, 1H), 6.67 (ddd, *J* = 8.4, 2.4, 0.8 Hz, 1H), 6.58 (dt, *J* = 12.2, 2.8 Hz, 1H), 6.54 (ddd, *J* = 8.2, 2.4, 0.8 Hz, 1H), 3.57 (t, *J* = 5.2 Hz, 4H), 3.14 (t, *J =* 5.2 Hz, 4H), 1.48 (s, 9H). ¹³C NMR (101 MHz, CDCl₃) δ 163.93 (d, *J* = 243.7 Hz), 154.80, 152.98 (d, *J* = 9.8 Hz), 130.34 (d, *J* = 9.9 Hz), 111.74 (d, *J* = 2.4 Hz), 106.52 (d, *J* = 21.5 Hz), 103.31 (d, *J =* 25.0 Hz), 80.14, 48.96, 28.54.

### Intermediate 24: tert-Butyl-(R)-4-(benzo[d][1,3]dioxol-5-yl)-3-methylpiperazine-1-carboxylate

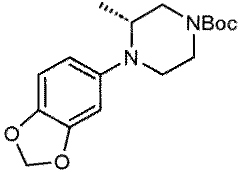

The title compound was synthesised from tert-butyl (R)-3-methylpiperazine-1-carboxylate (329 mg, 1.64 mmol) and 1-bromo-3,4-(methylenedioxy) benzene (300 mg, 1.49 mmol) according to general procedure C. Column chromatography (5-30% Et₂O in pentane) yielded the product as a pale-yellow oil (157 mg, 0.49 mmol, 33%). NMR spectra show a mixture of rotamers. 1H NMR (500 MHz, CDCl₃) δ 6.72 (d, J = 8.3 Hz, 1H), 6.57 (s, 1H), 6.41 (d, J = 7.8 Hz, 1H), 5.92-5.91 (m, 2H), 3.47 (m, 5H), 2.99-2.89 (m, 2H), 1.48 (s, 9H), 0.91 (d, J = 5.9 Hz, 3H). 13C NMR (126 MHz, CDCl₃) δ 155.06, 148.29, 146.11, 112.77, 108.29, 102.49, 101.11, 79.86, 53.86, 50.08, 49.00, 47.59, 44.56, 43.40*, 28.57, 13.42. *C NMR Peaks of piperazine broadened and split up.

### Intermediate 25: tert-Butyl 4-(benzo[d][1,3]dioxol-5-yl)piperazine-1-carboxylate

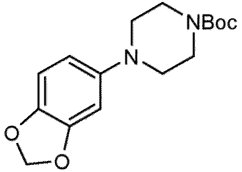

The title compound was synthesised from *tert*-butyl piperazine-1-carboxylate (306 mg, 1.64 mmol) and 1-bromo-3,4-(methylenedioxy) benzene (300 mg, 1.49 mmol) according to general procedure C. Column chromatography (0-30% Et₂O in pentane) yielded the product as a pale-yellow oil (229 mg, 0.75 mmol, 50%). ¹H NMR (300 MHz, CDCl₃) δ 6.72 (d, *J* = 8.4 Hz, 1H), 6.55 (d, *J* = 2.4 Hz, 1H), 6.36 (dd, *J* = 8.4, 2.4 Hz, 1H), 5.90 (s, 2H), 3.56 (t, *J* = 5.1 Hz, 4H), 2.98 (t, *J* = 5.1 Hz, 4H), 1.48 (s, 9H). ¹³C NMR (75 MHz, CDCl₃) δ 155.29, 147.51, 143.06, 109.80, 108.32, 101.08, 100.67, 79.99, 51.28, 28.57.

### Intermediate 26: ± trans-4-(3-Fluorophenyl)-2,3-dimethylpiperazine

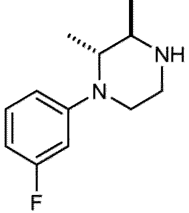

To a cooled (10 °C) solution of *trans*-2,3-dimethylpiperazine (0.50 g, 4.4 mmol, 1 eq.) in dioxane (12 mL) was added 1-chloro-3-fluorobenzene (0.47 mL, 4.4 mmol, 1 eq.) and KHMDS (5.7 mL 1 M in THF, 5.7 mmol, 1.3 eq.) in a dropwise manner. The suspension was allowed to warm to RT and stirred for 4 h. After conversion of the starting material, as indicated by LC-MS/TLC the suspension was diluted with DCM, washed with water (2x), brine. The organic layer was dried (Na₂SO₄), filtered and concentrated *in vacuo.* Flash column chromatography (1 - 3% MeOH in DCM, 1% TEA) afforded the product as a brown oil. ¹H NMR (400 MHz, CDCl₃) δ 7.19 (td, *J* = 8.1, 6.9 Hz, 1H), 6.73 (ddd, *J* = 8.3, 2.2, 0.9 Hz, 1H), 6.65 (dt, *J* = 12.0, 2.3 Hz, 1H), 6.59 (tdd, *J* = 8.3, 2.5, 0.9 Hz, 1H), 3.21 (qd, *J =* 6.4, 4.6 Hz, 1H), 3.16 - 3.07 (m, 1H), 3.08 - 3.01 (m, 2H), 3.00 - 2.90 (m, 1H), 2.84 (qd, J = 6.6, 4.6 Hz, 1H), 2.17 (s, 1H), 1.29 (d, *J* = 6.6 Hz, 3H), 1.05 (d, *J* = 6.4 Hz, 3H). ¹³C NMR (101 MHz, CDCl₃) δ 163.72 (d, *J* = 243.8 Hz), 153.55 (d, *J* = 9.4 Hz), 129.97 (d, *J* = 9.9 Hz), 114.90 (d, *J* = 2.6 Hz), 107.22 (d, *J* = 21.4 Hz), 106.23 (d, *J* = 23.5 Hz), 57.51, 54.22, 48.21, 42.23, 19.07, 14.63.

### Intermediate 27: ± trans-4-(Benzo[d][1,3]dioxol-5-yl)-2,3-dimethylpiperazine

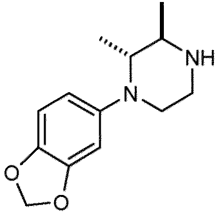

To a solution of 2,3-*trans*-dimethylpiperazine (50 mg, 0.44 mmol, 1 eq.) in 1,4-dioxane (5 mL) were added 5-bromo-1,3-benzodioxole (89 mg, 0.44 mmol, 1 eq.) and KHMDS (88 mg, 0.44 mmol, 1 eq.). After 2 h, TLC analysis indicated full consumption of the starting material and the mixture was diluted with DCM, washed with water, dried (MgSO₄), filtered and concentrated under reduced pressure. Purification of the crude product by flash column chromatography (1-10% MeOH in DCM, 1% TEA) yielded the product as an orange solid (29 mg, 0.12 mmol, 27%). ¹H NMR (400 MHz, CDCl₃) δ 6.68-6.72 (m, 1H), 6.50-6.60 (m, 1H), 6.15-6.31 (m, 1H), 5.87-5.97 (m, 2H), 2.64-3.15 (m, 6H), 1.07-1.26 (m, 6H). ¹³C NMR (400 MHz, CDCl₃) δ 134.78, 118.48, 108.15, 105.44, 101.16, 61.46, 56.76, 55.56, 50.85, 45.55, 29.83, 19.02, 16.38.

### Intermediate 28: tert-Butyl 3-chloro-4-(2-ethoxy-2-oxoethoxy)benzoate

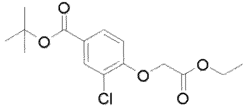

Sodium hydride (60%, 676 mg, 17 mmol, 1.3 eq.) is suspended in dry DMF (9 mL) under a nitrogen atmosphere and the resulting mixture is cooled in ice water. To above suspension is added dropwise over 20 minutes ethyl 2-hydroxyacetate (1.49 g, 14.3 mmol, 1.1 eq.) and *tert*-butyl 3-chloro-4-fluorobenzoate (3.0 g, 13 mmol, 1 eq.) in dry DMF (18 mL). Upon the end of addition the cold bath is removed and the reaction mixture is stirred for another 1 h. Water (40 mL) was added slowly to the reaction mixture, and a yellow precipitate resulted. The resultant solid is isolated by suction filtration, washed with water (40 mL), and air dried. The crude product was purified by silica column chromatography (30% Et₂O in pentane) to yield 868 mg (2.8 mmol, 21%) as a colourless oil.

¹H NMR (400 MHz, CDCl₃) δ 8.01 (d, *J* = 2.1 Hz, 1H), 7.85 (dd, *J* = 8.7, 2.1 Hz, 1H), 6.82 (d, *J=* 8.7 Hz, 1H), 4.76 (s, 2H), 4.27 (q, *J* = 7.1 Hz, 2H), 1.58 (s, 9H), 1.29 (t, *J* = 7.1 Hz, 3H). ¹³C NMR (101 MHz, CDCl₃) δ 167.85, 164.32, 156.66, 131.89, 129.45, 126.34, 122.90, 112.32, 81.39, 65.96, 61.74, 28.22, 14.19.

### Intermediate 29: 3-Chloro-4-(2-ethoxy-2-oxoethoxy)benzoic acid

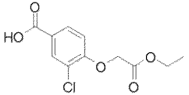

Intermediate 28 (350 mg, 1.11 mmol) was dissolved in TFA in DCM (20%, v/v, 11 mL), stirred for 7h. The mixture was diluted with toluene, concentrated, and purified by silica column chromatography (3-10% MeOH in DCM with 1% AcOH) to yield 256 mg (0.99 mmol, 89%) as a white solid.

### Intermediate 30: Ethyl 2-(2-chloro-4-(4-(3-chlorophenyl)piperazine-1-carbonyl)phenoxy)acetate

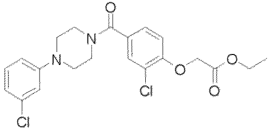

To a solution of Intermediate 29 (100 mg, 0.39 mmol, 1 eq.), PyAOP (404 mg, 0.77 mmol, 2 eq.) and Intermediate 14 (84 mg, 0.43 mmol, 1.1 eq.) in DCM (4 mL) was added DiPEA (150 mg, 1.16 mmol, 3 eq.) the resulting mixture was stirred over the weekend before water was added and the mixture was extracted with DCM (3x). The organic layer was, dried (MgSO₄), filtrated and concentrated in vacuo. The crude product was purified using silica column chromatography (eluent = 50% EtOAc in pentane) to yield the product as a transparent oil (155 mg, 0.35 mmol, 92%).

¹H NMR (400 MHz, CDCl₃) δ 7.51 (d, *J* = 2.1 Hz, 1H), 7.32 (dd, *J* = 8.4, 2.1 Hz, 1H), 7.19 (t, *J =* 8.1 Hz, 1H), 6.91 - 6.83 (m, 3H), 6.79 (ddd, *J* = 8.4, 2.4, 1.0 Hz, 1H), 4.75 (s, 2H), 4.28 (q, *J =* 7.1 Hz, 2H), 4.05 - 3.50 (m, 4H), 3.37 - 3.00 (m, 4H), 1.31 (t, *J* = 7.2 Hz, 3H). ¹³C NMR (101 MHz, CDCl₃) δ 168.75, 168.03, 154.85, 151.97, 135.11, 130.28, 129.95, 129.57, 129.38, 127.22, 123.48, 120.36, 116.55, 114.64, 113.28, 66.12, 61.77, 49.25, 14.23.

### Intermediate 31: 2-(2-Chloro-4-(4-(3-chlorophenyl)piperazine-1-carbonyl)phenoxy)acetic acid

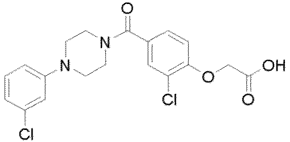

Ethyl 2-(2-chloro-4-(4-(3-chlorophenyl)piperazine-1-carbonyl)phenoxy)acetate (1 eq) was dissolved in MeOH (2.5 mL), before adding TEA (2.5 mL) and water (2.5 mL). The reaction mixture was stirred for 2.5 h, after which TLC analysis confirmed reaction completion and product formation (TLC: 5% MeOH in DCM, 10 drops acetic acid). Subsequently, the solution was acidified to pH 2 with a 6 M HCl solution and rapidly extracted with EtOAc, after which all organic layers were dried (MgSO₄), filtered and concentrated under reduced pressure to yield the title product as a white solid (90 mg, 0.22 mmol, 96%). Used in the next step without purification.

### Example 2: ± 2-(2-((2-Chloro-4-(trans-4-(3-chlorophenyl)-2,3-dimethylpiperazine-1-carbonyl)phenyl)sulfinyl)acetyl)-7-oxa-2,5-diazaspiro[3.4]octan-6-one

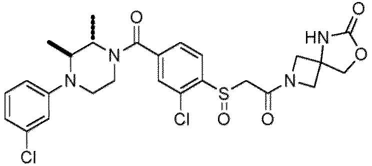

The title compound was synthesised from Intermediate 2 (7.8 mg, 61 µmol) according to general procedure A. Column chromatography (5% MeOH in DCM) yielded the product as a white solid (4 mg, 7 µmol, 12%). NMR spectra show a mixture of rotamers. ¹H NMR (400 MHz, CDCl₃) δ 8.00 (d, *J* = 7.6 Hz, 1H), 7.67 - 7.45 (m, 2H), 7.20 (t, *J =* 8.0 Hz, 1H), 6.87 - 6.81 (m, 2H), 6.79 - 6.70 (m, 1H), 4.83 (s, 1H), 4.72 - 4.12 (m, 6H), 3.99 - 3.83 (m, 1H), 3.77 - 3.64 (m, 1H), 3.64 - 3.50 (m, 1H), 3.49 - 3.10 (m, 3H), 1.59 - 1.41 (m, 5H), 1.20 - 1.00 (m, 3H). HRMS: calculated for [C₂₆H₂₈Cl₂N₄O₅S + H]⁺ 579.12302; found 579.12289.

### Example 3: ± 2-(2-((2-Chloro-4-(trans-4-(3-chlorophenyl)-2,3-dimethylpiperazine-1-carbonyl)phenyl)sulfinyl)acetyl)-2,5-diazaspiro[3.4]octan-6-one

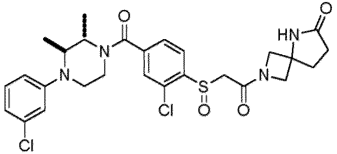

The title compound was synthesised from Intermediate 1 (9.7 mg, 77 µmol) according to general procedure A. Column chromatography (5% MeOH in DCM) yielded the product as a white solid (12 mg, 21 µmol, 33%). NMR spectra show a mixture of rotamers. ¹H NMR (500 MHz, CDCl₃) δ 8.05 - 7.97 (m, 1H), 7.62 - 7.53 (m, 1H), 7.52 - 7.44 (m, 1H), 7.18 (t, *J* = 7.9 Hz, 1H), 6.86 - 6.79 (m, 2H), 6.72 (t, *J* = 6.8 Hz, 1H), 4.82 (t, J = 6.9 Hz, 1H), 4.63 (d, *J* = 12.1 Hz, 1H), 4.45 - 4.30 (m, 2H), 4.29 - 4.07 (m, 3H), 3.97 - 3.84 (m, 1H), 3.75 - 3.63 (m, 1H), 3.60 - 3.49 (m, 2H), 3.40 - 3.07 (m, 3H), 2.50 - 2.32 (m, 3H), 1.55 - 1.46 (m, 3H), 1.18 - 0.99 (m, 3H). ¹³C NMR (126 MHz, CDCl₃) δ 176.98, 176.81, 163.24, 151.35, 142.42, 135.98, 135.30, 130.36, 129.06, 128.66, 128.63, 128.26, 126.89, 119.51, 116.25, 114.24, 79.23, 64.02, 61.85, 56.17, 55.48, 55.33, 49.70, 42.38, 41.34, 40.49, 36.56, 33.32, 33.23, 30.05, 29.77, 17.84, 16.79, 12.86, 12.57. HRMS: calculated for [C₂₇H₃₀Cl₂N₄O₄S + H]⁺ 577.14376; found 577.14353.

### Example 4: 2-(2-((2-Chloro-4-((R)-4-(3-chlorophenyl)-3-methylpiperazine-1-carbonyl)phenyl)sulfinyl)acetyl)- 7-oxa-2,5-diazaspiro[3.4]octan-6-one

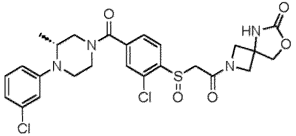

The title compound was synthesised from Intermediate 12 (10 mg, 49 µmol) and Intermediate 11 (15 mg, 40 µmol) according to general procedure B. Column chromatography (50% acetone in toluene) yielded the compound as an off-white solid (11 mg, 20 µmol, 52%). NMR spectra show a mixture of rotamers. ¹H NMR (600 MHz, CDCl₃) δ 8.04 - 7.96 (m, 1H), 7.66 - 7.58 (m, 1H), 7.57 - 7.50 (m, 1H), 7.22 (t, *J* = 8.0 Hz, 1H), 6.93 - 6.86 (m, 2H), 6.84 - 6.75 (m, 1H), 4.62 - 4.48 (m, 3H), 4.48 - 4.38 (m, 1H), 4.38 - 4.24 (m, 2H), 4.24 - 4.13 (m, 1H), 4.08 - 3.77 (m, 2H), 3.70 - 3.58 (m, 2H), 3.58 - 3.30 (m, 3H), 3.30 - 3.05 (m, 2H), 1.21 - 0.94 (m, 3H). ¹³C NMR (151 MHz, CDCl₃) δ 168.41, 168.10, 163.37, 163.20, 157.65, 157.53, 150.78, 142.56, 142.34, 140.51, 140.40, 135.34, 130.81, 130.45, 129.11, 128.74, 127.01, 126.90, 126.67, 126.52, 125.68, 120.66, 120.43, 117.61, 117.16, 115.66, 115.16, 74.28, 74.19, 67.25, 63.69, 63.64, 63.60, 61.25, 61.22, 56.36, 55.87, 55.78, 54.18, 54.06, 54.04, 51.88, 50.04, 47.60, 47.35, 44.23, 42.83, 42.33, 12.74. HRMS: calculated for [C₂₆H₂₈Cl₂N₄O₅S + H]⁺ 565.1074; found 565.1072.

### Example 5: 2-(2-((2-Chloro-4-((R)-4-(3-chlorophenyl)-3-methylpiperazine-1-carbonyl)phenyl)sulfinyl)acetyl)-2,5-diazaspiro[3.4]octan-6-one

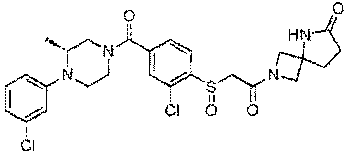

The title compound was synthesised from Intermediate 12 (10 mg, 49 µmol) and Intermediate 10 (15 mg, 40 µmol) according to general procedure B. Column chromatography (100% acetone) yielded the product as a white solid (7 mg, 12 µmol, 30%). NMR spectra show a mixture of rotamers. ¹H NMR (400 MHz, CDCl₃) δ 8.01 (dd, *J* = 8.1, 1.0 Hz, 1H), 7.62 (dt, *J* = 7.9, 1.6 Hz, 1H), 7.57 - 7.49 (m, 1H), 7.22 (t, *J* = 8.9, 7.5 Hz, 1H), 6.92 - 6.85 (m, 2H), 6.85 - 6.75 (m, 1H), 4.49 - 4.09 (m, 5H), 4.06 - 3.67 (m, 3H), 3.62 - 3.53 (m, 2H), 3.50 - 3.04 (m, 4H), 2.47 - 2.38 (m, 4H), 1.18 - 0.96 (m, 3H). ¹³C NMR (101 MHz, Acetone) δ 176.70, 176.63, 164.32, 152.11, 144.19, 144.10, 141.45, 135.42, 131.27, 130.82, 129.72, 129.20, 129.01, 127.67, 127.44, 119.39, 116.43, 115.16, 64.62, 64.56, 62.24, 62.21, 58.09, 58.03, 55.98, 55.91, 55.47, 51.64, 48.05, 47.04, 43.75, 42.50, 33.80, 12.39. HRMS: calculated for [C₂₆H₂₈Cl₂N₄O₄S + H]⁺ 563.12811; found 563.12807.

### Example 6: 2-(2-((2-Chloro-4-(8-(3-chlorophenyl)-3,8-diazabicyclo[3.2.1]octane-3-carbonyl)phenyl)sulfinyl)- acetyl)-7-oxa-2,5-diazaspiro[3.4]octan-6-one

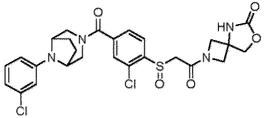

The title compound was synthesised from Intermediate 13 (11 mg, 49 µmol) and Intermediate 11 (15 mg, 40 µmol) according to general procedure B. Column chromatography (50% acetone in toluene) yielded the compound as an off-white solid (4.9 mg, 8.0 µmol, 21%). NMR spectra show a mixture of rotamers. ¹H NMR (600 MHz, CDCl₃) δ 7.99 - 7.91 (m, 1H), 7.61 - 7.35 (m, 2H), 7.18 (t, *J* = 8.0 Hz, 1H), 6.80 - 6.74 (m, 2H), 6.69 - 6.63 (m, 1H), 4.61 - 4.47 (m, 2H), 4.46 - 4.36 (m, 2H), 4.35 - 4.09 (m, 6H), 3.98 - 3.76 (m, 1H), 3.68 - 3.51 (m, 2H), 3.32 - 3.20 (m, 2H), 2.16 - 2.03 (m, 2H), 2.03 - 1.91 (m, 1H), 1.77 - 1.66 (m, 1H). ¹³C NMR (151 MHz, CDCl₃) δ 169.26, 163.17, 157.43, 147.03, 147.01, 142.32, 142.18, 140.96, 135.79, 131.00, 130.80, 128.69, 128.20, 126.91, 126.39, 125.67, 118.60, 113.79, 74.20, 63.64, 63.55, 62.30, 61.24, 61.17, 56.06, 55.84, 55.48, 54.92, 54.74, 54.71, 54.10, 53.93, 50.36, 50.28, 44.58, 29.85, 27.25. HRMS calculated for [C₂₆H₂₈Cl₂N₄O₅S + H]⁺ 577.1074; found: 577.1072.

### Example 7: 2-(2-((2-Chloro-4-(8-(3-chlorophenyl)-3,8-diazabicyclo[3.2.1]octane-3-carbonyl)phenyl)sulfinyl)acetyl)-2,5-diazaspiro[3.4]octan-6-one

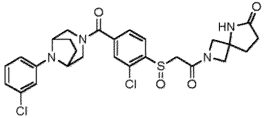

The title compound was synthesised from Intermediate 13 (11 mg, 40 µmol) and Intermediate 10 (15 mg, 40 µmol) according to general procedure B. The obtained crude was further purified by silica gel column chromatography eluting with 50% acetone in toluene to yield the title compound as an off-white solid (4.3 mg, 7.7 µmol, 18%). NMR spectra show a mixture of rotamers. ¹H NMR (400 MHz, CDCl₃) δ 8.03 - 7.94 (m, 1H), 7.61 - 7.37 (m, 2H), 7.21 (t, *J* = 8.3 Hz, 1H), 6.82 - 6.75 (m, 2H), 6.73 - 6.65 (m, 1H), 4.52 - 4.09 (m, 8H), 3.97 - 3.86 (m, 1H), 3.72 - 3.63 (m, 1H), 3.62 - 3.53 (m, 1H), 3.36 - 3.24 (m, 1H), 2.48 - 2.37 (m, 4H), 2.27 - 1.92 (m, 4H), 1.87 - 1.64 (m, 1H). ¹³C NMR (101 MHz, CDCl₃) δ 176.47, 173.64, 172.83, 146.91, 141.95, 138.95, 135.64, 130.86, 127.79, 126.73, 118.44, 115.49, 114.88, 113.66, 112.57, 63.44, 61.79, 56.84, 55.56, 54.03, 50.53, 47.01, 44.67, 32.46, 29.93, 27.22, 26.24. HRMS: calculated for [C₂₇H₂₈Cl₂N₄O₄S + H]⁺ 575.12811; found 575.12826.

### Example 8: 2-(2-((2-Chloro-4-(4-(3-chlorophenyl)piperazine-1-carbonyl)phenyl)sulfinyl)acetyl)-7-oxa-2,5- diazaspiro[3.4]octan-6-one

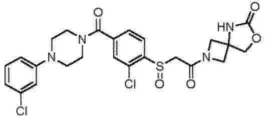

The title compound was synthesised from Intermediate 14 (9.5 mg, 49 µmol) and Intermediate 11 (15 mg, 40 µmol) according to general procedure B. Column chromatography (50% acetone in toluene) yielded the compound as an off-white solid (5.0 mg, 9.0 µmol, 23%). NMR spectra show a mixture of rotamers. ¹H NMR (600 MHz, CDCl₃) δ 7.97 (dd, J = 8.0, 5.5 Hz, 1H), 7.63 - 7.55 (m, 1H), 7.51 (d, *J* = 1.5 Hz, 1H), 7.20 (t, *J* = 8.3 Hz, 1H), 6.92 - 6.87 (m, 2H), 6.82 - 6.76 (m, 1H), 4.58 - 4.48 (m, 3H), 4.45 - 4.37 (m, 1H), 4.33 - 4.22 (m, 2H), 4.22 - 4.11 (m, 1H), 3.99 - 3.86 (m, 4H), 3.62 - 3.53 (m, 2H), 3.34 - 3.12 (m, 4H). ¹³C NMR (151 MHz, CDCl₃) δ 167.67, 167.65, 163.74, 163.34, 163.14, 157.64, 157.53, 151.88, 151.86, 142.53, 142.40, 140.43, 140.30, 135.29, 130.83, 130.77, 130.43, 130.09, 128.81, 128.72, 127.01, 126.92, 126.69, 126.57, 120.81, 120.77, 116.86, 116.83, 114.90, 114.88, 74.29, 74.25, 74.17, 67.24, 63.69, 63.62, 61.25, 61.23, 56.41, 56.13, 55.75, 54.16, 54.12, 54.04. HRMS: calculated for [C₂₄H₂₄Cl₂N₄O₅S + H]⁺ 551.09172; found 551.09137.

### Example 9: 2-(2-((2-Chloro-4-(4-(3-chlorophenyl)piperazine-1-carbonyl)phenyl)sulfinyl)acetyl)-2,5-diazaspiro[3.4]octan-6-one

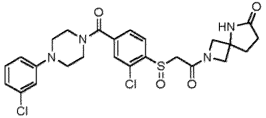

The title compound was synthesised from Intermediate 14 (9.7 mg, 49 µmol) and Intermediate 10 (15 mg, 40 µmol) according to general procedure B. Column chromatography (100% acetone) yielded the compound as an off-white solid (9.0 mg, 16 µmol, 40%). NMR spectra show a mixture of rotamers. ¹H NMR (400 MHz, MeOD) δ 8.00 (dd, *J* = 8.0, 1.3 Hz, 1H), 7.80 - 7.71 (m, 1H), 7.72 - 7.67 (m, 1H), 7.29 - 7.20 (m, 1H), 7.05 - 6.98 (m, 1H), 6.99 - 6.91 (m, 1H), 6.88 - 6.82 (m, 1H), 4.90 - 4.60 (m, 8H), 4.60 - 4.37 (m, 2H), 4.32 - 4.16 (m, 2H), 4.01 - 3.86 (m, 2H), 3.70 - 3.55 (m, 2H), 3.29 - 3.18 (m, 2H). ¹³C NMR (101 MHz, MeOD) δ 169.33, 164.68, 160.29, 153.60, 143.84, 141.61, 135.88, 131.64, 131.41, 129.72, 127.99, 127.81, 120.72, 117.19, 115.76, 75.31, 64.81, 64.74, 62.09, 55.98, 55.30, 50.13, 43.17. HRMS calculated for [CzsHzsCIzNaOaS + H]⁺ 549.11246; found: 549.11254.

### Example 10: ± 2-(2-((2-Chloro-4-(trans-4-(3-fluorophenyl)-2,3-dimethylpiperazine-1-carbonyl)phenyl)sulfinyl)acetyl)-2,5-diazaspiro[3.4]octan-6-one

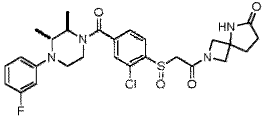

The title compound was synthesised from Intermediate 26 (5.6 mg, 27 µmol) and Intermediate 10 (9 mg, 24 µmol) according to general procedure B. Column chromatography (100% acetone) yielded the compound as an off-white solid (5.0 mg, 8.9 µmol, 37%). NMR spectra show a mixture of rotamers. ¹H NMR (500 MHz, CDCl₃) δ 8.21 (dd, *J* = 8.1, 1.5 Hz, 1H), 8.10 (d, *J* = 1.5 Hz, 1H), 8.00 (d, *J* = 8.2 Hz, 1H), 7.65 - 7.41 (m, 1H), 7.22 (q, *J* = 7.8 Hz, 1H), 6.68 - 6.49 (m, 2H), 4.89 - 4.59 (m, 1H), 4.50 - 4.34 (m, 2H), 4.25 - 4.11 (m, 3H), 4.02 - 3.88 (m, 5H), 3.67 - 3.52 (m, 2H), 2.50 - 2.37 (m, 4H), 1.58 - 1.48 (m, 3H), 1.23 - 1.02 (m, 3H). ¹³C NMR (126 MHz, CDCl₃) δ 176.77, 176.70, 164.11 (d, *J* = 239.4 Hz), 162.97, 145.76, 134.57, 131.03, 131.01, 130.55 (d, *J* = 10.2 Hz), 130.34, 129.08, 128.31, 126.96, 126.67, 126.54, 111.50, 106.13 (d, *J* = 20.5 Hz), 103.19 (d, *J* = 25.3 Hz), 64.17, 61.92, 56.11, 56.01, 55.84, 55.61, 55.51, 55.40, 52.97, 42.44, 41.45, 40.60, 33.35, 33.27, 30.02, 30.01. HRMS calculated for [C₂₇H₃₀ClFN₄O₄S + H]⁺ 561.17331; found: 561.17357.

### Example 11: 2-(2-((2-Chloro-4-((R)-4-(3-fluorophenyl)-3-methylpiperazine-1-carbonyl)phenyl)sulfinyl)acetyl)-7-oxa-2,5-diazaspiro[3.4]octan-6-one

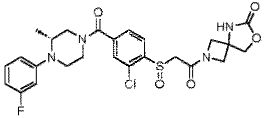

The title compound was synthesised from Intermediate 15 (10 mg, 54 µmol) and Intermediate 11 (20 mg, 54 µmol) according to general procedure B. Column chromatography (50% acetone in toluene) yielded the compound as a white solid (21 mg, 38 µmol, 71%). NMR spectra show a mixture of rotamers. ¹H NMR (400 MHz, CDCl₃) δ 8.01 - 7.94 (m, 1H), 7.65 - 7.57 (m, 1H), 7.54 - 7.49 (m, 1H), 7.21 (q, *J* = 7.9 Hz, 1H), 6.71 - 6.61 (m, 1H), 6.61 -6.51 (m, 2H), 4.59 -4.50 (m, 3H), 4.50 - 4.35 (m, 1H), 4.29 (d, *J* = 9.0 Hz, 2H), 4.16 (t, *J* = 11.7 Hz, 1H), 4.05 - 3.79 (m, 2H), 3.76 - 3.52 (m, 2H), 3.51 - 3.00 (m, 5H), 1.17 - 0.91 (m, 3H).¹³C NMR (101 MHz, CDCl₃) δ 164.00 (d, *J* = 244.4 Hz), 163.66, 163.43, 158.03, 157.86, 151.30 (d, *J* = 9.1 Hz), 142.42, 142.26, 140.35, 140.29, 130.71, 130.54 (d, *J* = 9.9 Hz), 128.68, 126.91, 126.72, 112.47, 112.05, 106.81, 103.92, 74.31, 74.16, 63.59, 61.27, 56.26, 54.15, 54.12, 51.62, 47.51, 47.22, 42.25, 12.49. HRMS: Calculated for [C₂₅H₂₆ClFN₄O₅S + H]⁺ 549.13692; found 549.13717.

### Example 12: 2-(2-((2-Chloro-4-((R)-4-(3-fluorophenyl)-3-methylpiperazine-1-carbonyl)phenyl)sulfinyl)acetyl)-2,5-diazaspiro[3.4]octan-6-one

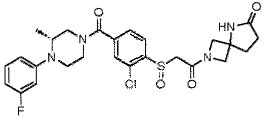

The title compound was synthesised from Intermediate 15 (11 mg, 54 µmol) and Intermediate 10 (20 mg, 54 µmol) according to general procedure B. Column chromatography (100% acetone) yielded the compound as a white solid (12 mg, 22 µmol, 41%). NMR spectra show a mixture of rotamers. ¹H NMR (400 MHz, CDCl₃) δ 8.05 - 7.97 (m, 1H), 7.61 (dt, *J* = 8.0, 1.6 Hz, 1H), 7.55 - 7.51 (m, 1H), 7.27 - 7.09 (m, 2H), 6.71 - 6.52 (m, 3H), 4.70 - 4.31 (m, 3H), 4.30 - 4.08 (m, 3H), 4.08 - 3.80 (m, 2H), 3.78 - 3.53 (m, 1H), 3.53 - 3.02 (m, 4H), 2.49 - 2.36 (m, 4H), 1.18 - 0.95 (m, 3H). ¹³C NMR (101 MHz, CDCl₃) δ 177.00, 176.85, 163.42 (d, *J =* 244.4 Hz), 163.25, 163.09, 151.25 (d, J = 10.1 Hz), 142.69, 142.60, 140.25, 140.18, 130.52 (d, *J=* 9.9 Hz), 128.67, 126.86, 126.59, 112.06, 106.85, 64.05, 61.87, 56.18, 55.49, 55.36, 51.64, 33.31, 33.23, 30.08, 29.80, 12.47. HRMS: Calculated for [C₂₆H₂₈ClFN₄O₄S + H]⁺ 547.15766; found 547.15808.

### Example 13: 2-(2-((2-Chloro-4-(4-(3-fluorophenyl)piperazine-1-carbonyl)phenyl)sulfinyl)acetyl)-7-oxa-2,5-diazaspiro[3.4]octan-6-one

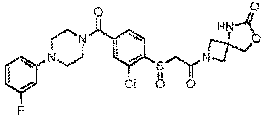

The title compound was synthesised from Intermediate 16 (13 mg, 70 µmol), and Intermediate 11 (20 mg, 54 µmol) according to general procedure B. Column chromatography (50% acetone in toluene) yielded the compound as a white solid (18 mg, 34 µmol, 63%). NMR spectra show a mixture of rotamers. ¹H NMR (400 MHz, CDCl₃) δ 7.98 (dd, *J* = 8.0, 2.2 Hz, 1H), 7.61 (ddd, *J* = 8.0, 3.2, 1.5 Hz, 1H), 7.53 (d, *J* = 1.5 Hz, 1H), 7.36 (d, *J* = 31.9 Hz, 1H), 7.28 - 7.19 (m, 1H), 6.70 (ddd, *J* = 8.4, 2.1, 1.0 Hz, 1H), 6.66 - 6.56 (m, 2H), 4.59 - 4.51 (m, 2H), 4.50 - 4.38 (m, 1H), 4.36 - 4.25 (m, 2H), 4.23 - 4.12 (m, 1H), 4.01 - 3.87 (m, 3H), 3.65 - 3.54 (m, 3H), 3.37 - 3.09 (m, 4H). ¹³C NMR (101 MHz, CDCl₃) δ 167.62, 167.60, 163.86 (d, *J* = 245.4 Hz), 163.62, 163.38, 157.99, 157.81, 152.39 (d, *J* = 9.6 Hz), 142.51, 142.35, 140.27, 140.21, 130.68, 130.53 (d, *J* = 9.9 Hz), 128.74, 128.70, 126.92, 126.88, 126.74, 126.67, 111.99, 107.22 (d, *J* = 21.3 Hz), 103.64 (d, *J* = 24.9 Hz), 74.29, 74.15, 63.65, 63.59, 61.27, 56.38, 56.26, 54.15, 54.12, 49.51, 48.98, 47.50, 42.19. HRMS: Calculated for [C₂₄H₂₄ClFN₄O₅S + H]⁺ 535.12127; found 535.12165.

### Example 14: ± 2-(2-((4-(trans-4-(Benzo[d][1,3]dioxol-5-yl)-2,3-dimethylpiperazine-1-carbonyl)-2-chlorophenyl) sulfinyl)acetyl)-7-oxa-2,5-diazaspiro[3.4]octan-6-one

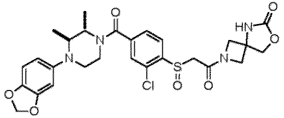

The title compound was synthesised from Intermediate 27 (11 mg, 49 µmol) and Intermediate 11 (15 mg, 40 µmol) according to general procedure B. Column chromatography (50% acetone in toluene) yielded the compound as an off-white solid (4.0 mg, 6.8 µmol, 17%). NMR spectra show a mixture of rotamers. ¹H NMR (400 MHz, CDCl₃) δ 8.01 (d, *J* = 8.0 Hz, 1H), 7.67 - 7.52 (m, 1H), 7.52 - 7.42 (m, 1H), 6.74 (d, *J* = 8.4 Hz, 1H), 6.49 (d, *J=* 7.7 Hz, 1H), 6.28 (dd, *J* = 8.4, 2.4 Hz, 1H), 5.99 - 5.83 (m, 2H), 4.90 - 4.53 (m, 1H), 4.45 - 4.31 (m, 2H), 4.30 - 4.05 (m, 2H), 4.01 - 3.83 (m, 1H), 3.76 - 3.45 (m, 2H), 3.35 - 3.23 (m, 1H), 3.16 - 2.92 (m, 1H), 2.51 - 2.33 (m, 5H), 1.61 - 1.48 (m, 3H), 1.13-0.89 (m, 3H). ¹³C NMR (214 MHz, CDCl₃) δ 168.51, 162.94, 157.21, 148.45, 145.90, 142.04, 141.88, 141.54, 141.32, 130.72, 130.61, 128.13, 127.48, 126.78, 126.03, 125.53, 109.77, 109.68, 108.28, 100.99, 100.34, 74.10, 74.05, 63.51, 63.45, 61.10, 61.00, 58.14, 58.03, 55.70, 55.32, 55.18, 53.96, 53.79, 49.78, 46.32, 46.30, 42.66, 42.17, 41.34, 36.80. HRMS: calculated for [C₂₇H₂₉ClN₄O₇S + H]⁺ 589.15182; found 589.15141.

### Example 15: ± 2-(2-((4-(trans-4-(benzo[d][1,3]dioxol-5-yl)-2,3-dimethylpiperazine-1-carbonyl)-2-chlorophenyl)sulfinyl)acetyl)-2,5-diazaspiro[3.4]octan-6-one

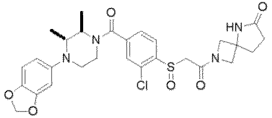

The title compound was synthesised from Intermediate 27 (12 mg, 49 µmol) and Intermediate 10 (15 mg, 40 µmol) according to general procedure B. Column chromatography (100% acetone) yielded the compound as a white solid (7.0 mg, 12 µmol, 29%). NMR spectra show a mixture of rotamers. ¹H NMR (400 MHz, CDCl₃) δ 8.01 (d, *J* = 8.0 Hz, 1H), 7.67 - 7.41 (m, 1H), 7.13 - 6.87 (m, 1H), 6.74 (d, *J =* 8.4 Hz, 1H), 6.59 - 6.42 (m, 1H), 6.28 (dd, *J* = 8.4, 2.4 Hz, 1H), 5.99 - 5.83 (m, 2H), 4.90 - 4.53 (m, 1H), 4.45 - 4.31 (m, 2H), 4.30 - 4.05 (m, 3H), 4.01 - 3.83 (m, 1H), 3.76 - 3.45 (m, 3H), 3.35 - 3.23 (m, 1H), 3.16 - 2.92 (m, 1H), 2.51 - 2.33 (m, 5H), 1.61 - 1.48 (m, 3H), 1.13 - 0.89 (m, 3H). ¹³C NMR (101 MHz, CDCl₃) δ 176.64, 163.12, 148.44, 145.95, 141.52, 126.77, 109.72, 108.28, 100.98, 100.32, 63.95, 61.78, 58.11, 56.04, 55.36, 46.33, 46.28, 33.28, 33.18, 29.94, 29.29, 26.48, 26.40. HRMS: Calculated for [C₂₈H₃₁ClN₄O₆S + H]⁺ 587.17179; 587.17175 found.

### Example 16: 2-(2-((4-((R)-4-(Benzo[d][1,3]dioxol-5-yl)-3-methylpiperazine-1-carbonyl)-2-chlorophenyl)sulfinyl) acetyl)-7-oxa-2,5-diazaspiro[3.4]octan-6-one

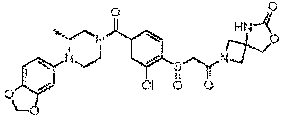

The title compound was synthesised from Intermediate 17 (12 mg, 54 µmol) and Intermediate 11 (20 mg, 54 µmol) according to general procedure B. Column chromatography (50% acetone in toluene) yielded the compound as a white solid (18 mg, 32 µmol, 59%). NMR spectra show a mixture of rotamers. ¹H NMR (400 MHz, CDCl₃) δ 7.97 (dd, *J* = 7.9, 1.5 Hz, 1H), 7.60 (dd, *J* = 8.6, 1.2 Hz, 1H), 7.53 - 7.48 (m, 1H), 6.73 (d, *J* = 8.3 Hz, 1H), 6.64 - 6.38 (m, 2H), 5.99 - 5.88 (m, 2H), 4.60 - 4.48 (m, 3H), 4.48 - 4.34 (m, 1H), 4.33 - 4.24 (m, 2H), 4.16 (dd, *J* = 15.1, 11.2 Hz, 1H), 4.04 - 3.75 (m, 3H), 3.67 - 3.52 (m, 3H), 3.53 - 2.76 (m, 3H), 1.00 - 0.83 (m, 3H). ¹³C NMR (101 MHz, CDCl₃) δ 167.93, 163.61, 163.38, 157.97, 157.79, 148.34, 145.26, 142.30, 142.11, 140.59, 140.51, 130.65, 128.67, 126.87, 126.71, 126.60, 114.27, 113.68, 108.35, 103.51, 103.04, 101.25, 74.29, 74.15, 63.63, 63.57, 61.25, 56.42, 56.18, 54.42, 54.13, 54.09, 53.53, 48.12, 42.72, 14.39, 13.83. HRMS: Calculated for [C₂₆H₂₇ClN₄O₇S + H]⁺575.13617; found 575.13649.

### Example 17: 2-(2-((4-((R)-4-(Benzo[d][1,3]dioxol-5-yl)-3-methylpiperazine-1-carbonyl)-2-chlorophenyl) sulfinyl)acetyl)-2,5-diazaspiro[3.4]octan-6-one

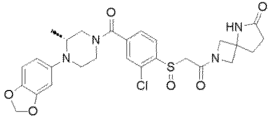

The title compound was synthesised from Intermediate 17 (12 mg, 54 µmol) and Intermediate 10 (20 mg, 54 µmol) according to general procedure B. Column chromatography (100% acetone) yielded the compound as a white solid (14 mg, 25 µmol, 46%). NMR spectra show a mixture of rotamers. ¹H NMR (400 MHz, CDCl₃) δ 7.98 (dd, *J* = 8.0, 1.8 Hz, 1H), 7.58 (d, *J* = 7.9 Hz, 1H), 7.50 (d, *J* = 2.5 Hz, 1H), 6.74 (d, *J* = 8.3 Hz, 1H), 6.66 - 6.35 (m, 2H), 5.97 - 5.88 (m, 2H), 4.43 - 4.30 (m, 2H), 4.28 - 4.05 (m, 3H), 4.01 (d, *J* = 7.9 Hz, 2H), 3.95 - 3.77 (m, 2H), 3.77 - 3.46 (m, 3H), 3.46 - 3.22 (m, 1H), 3.22 - 2.82 (m, 1H), 2.46 - 2.36 (m, 4H), 1.05 - 0.76 (m, 3H). ¹³C NMR (101 MHz, CDCl₃) δ 177.15, 176.96, 163.29, 163.14, 148.33, 145.25, 142.53, 142.41, 140.47, 140.39, 130.68, 128.67, 127.87, 126.80, 126.61, 126.53, 114.26, 114.00, 113.62, 108.33, 103.53, 103.03, 101.23, 70.53, 64.02, 61.85, 56.21, 55.51, 55.39, 54.35, 48.08, 42.64, 33.28, 33.21, 30.10, 29.79. HRMS: Calculated for [C₂₇H₂₉ClN₄O₆S + H]⁺ 573.15691; found 573.15736.

### Example 18: 2-(2-((4-(4-(Benzo[d][1,3]dioxol-5-yl)piperazine-1-carbonyl)-2-chlorophenyl)sulfinyl)acetyl)-7-oxa-2,5-diazaspiro[3.4]octan-6-one

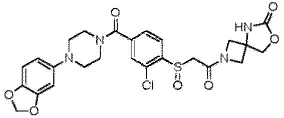

The title compound was synthesised from Intermediate 18 (11 mg, 54 µmol) and Intermediate 11 (20 mg, 54 µmol) according to general procedure B. Column chromatography (50% acetone in toluene) yielded the compound as a white solid (12 mg, 22 µmol, 41%). NMR spectra show a mixture of rotamers. ¹H NMR (400 MHz, CDCl₃) δ 7.96 (d, *J* = 8.0 Hz, 1H), 7.59 (ddd, *J* = 8.0, 3.4, 1.5 Hz, 1H), 7.51 (d, *J* = 1.5 Hz, 1H), 6.73 (d, *J* = 8.4 Hz, 1H), 6.55 (d, *J* = 2.4 Hz, 1H), 6.37 (dd, *J* = 8.4, 2.4 Hz, 1H), 5.93 (s, 2H), 4.58 - 4.51 (m, 3H), 4.48 - 4.40 (m, 1H), 4.32 - 4.28 (m, 2H), 4.21 - 4.14 (m, 1H), 3.96 - 3.89 (m, 3H), 3.62 - 3.56 (m, 3H), 3.15 - 3.02 (m, 4H). ¹³C NMR (101 MHz, CDCl₃) δ 167.55, 167.53, 163.53, 163.28, 157.88, 157.72, 148.44, 146.71, 142.60, 142.34, 142.17, 140.47, 140.38, 130.66, 130.63, 128.70, 128.66, 126.88, 126.83, 126.70, 126.61, 110.08, 108.36, 101.19, 100.88, 74.25, 74.12, 63.63, 63.56, 61.22, 56.28, 56.08, 54.11, 54.06, 51.75, 51.25, 47.87, 42.49. HRMS: Calculated for [C₂₅H₂₅ClN₄O₇S + H]⁺ 561.12052; found 561.12100.

### Example 19: 2-(2-(2-Chloro-4-(4-(3-chlorophenyl)piperazine-1-carbonyl)phenoxy)acetyl)-7-oxa-2,5-diazaspiro[3.4]octan-6-one

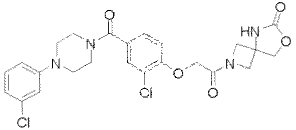

To a solution of Intermediate 31 (23 mg, 55 µmol, 1 eq.), PyAOP (57 mg, 110 µmol, 2 eq.) and 7-oxa-2,5-diazaspiro[3.4]octan-6-one hydrochloride (10 mg, 60 pmol 1.1 eq.). in DCM (1 mL) was added DiPEA (28 mg, 220 µmol, 3 eq.) the resulting mixture was stirred over the weekend before water was added and the mixture was extracted with DCM (3x). The organic layer was, dried (MgSO₄), filtrated and concentrated in vacuo. The crude product was purified using flash column chromatography (eluent = 50% acetone in pentane) to yield the product as a white solid (18 mg, 35 µmol, 63%).

¹H NMR (400 MHz, CDCl₃) δ 7.51 (d, *J* = 2.1 Hz, 1H), 7.34 (dd, *J* = 8.5, 2.1 Hz, 1H), 7.19 (t, *J* = 8.0 Hz, 1H), 6.94 (d, *J* = 8.6 Hz, 1H), 6.89 - 6.83 (m, 2H), 6.81 - 6.76 (m, 1H), 4.73 - 4.47 (m, 6H), 4.27 (d, *J* = 11.2 Hz, 1H), 4.18 (d, *J* = 11.1 Hz, 1H), 3.95 - 3.51 (m, 4H), 3.33 - 3.13 (m, 4H).

¹³C NMR (101 MHz, CDCl₃) δ 168.76, 167.08, 158.02, 154.21, 151.94, 135.16, 130.36, 129.93, 129.70, 127.63, 122.77, 120.46, 116.61, 114.72, 112.81, 74.50, 68.14, 64.43, 61.68, 55.26.

### Example 20: Biological Data

### Cellular activity

The MAGL activity assay is based on the production of glycerol from 2-arachidonoylglycerol (2-AG) hydrolysis by MAGL-overexpressing membrane preparations *f*rom transiently transfected HEK293T cells, as previously reported in van der Wel T et al., "A natural substrate-based fluorescence assay for inhibitor screening on diacylglycerol lipase", J Lipid Res., 2015, 56(4), 927-35. The produced glycerol is coupled to the oxidation of commercially available Amplifu^{™}Red (Sigma-Aldrich Chemie N.V., Zwijndrecht, Netherlands) via a multi-enzyme cascade, resulting in a fluorescent signal from the dye resorufin.

Standard assays were performed in HEMNB buffer (50 mM HEPES pH 7.4, 1 mM EDTA, 5 mM MgCl₂, 100 mM NaCl, 0.5% (w/v) BSA) in black, flat bottom 96-wells plates. Final protein concentration of membrane preparations from overexpressing hMAGL HEK293T cells was 1.5 µg/mL (0.3 µg per well). Inhibitors were added from 40x concentrated DMSO stocks. After 20 min. incubation, 100 µL assay mix containing glycerol kinase (GK), glycerol-3-phosphate oxidase (GPO), horse radish peroxidase (HRP), adenosine triphosphate (ATP), Amplifu^{™}Red and 2-arachidonoylglycerol (2-AG) was added and fluorescence was measured in 5 min. intervals for 60 min. on a plate reader. Final assay concentrations: 0.2 U/mL GK, GPO and HRP, 0.125 mM ATP, 10 µM Amplifu^{™}Red, 25 µM 2-AG, 5% DMSO, 0.5% ACN in a total volume of 200 µL. All measurements were performed in N = 2, n = 2 or N = 2, n = 4 for controls, with Z' ≥ 0.6.

For IC₅₀ determination, the MAGL-overexpressing membranes were incubated with different inhibitor concentrations. Slopes of corrected fluorescence in time were determined in the linear interval of t = 10 to t = 35 min and then scaled to the corrected positive control of hMAGL-overexpressing membranes treated with vehicle (DMSO) as a 100% activity reference point. The data was exported to GraphPad Prism 5.0 and analysed in a non-linear dose-response analysis with variable slope. The plCso data is provided in Table 1 below. The cellular activity of a comparator compound, LEI-515 (reported in WO 2021/175913 and depicted below), is also provided in Table 1.

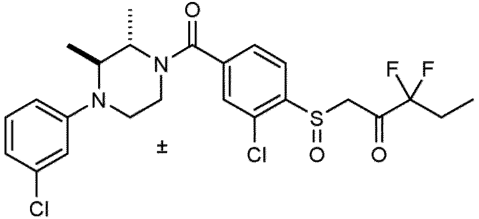

Table 1 also includes a clogP value for each compound (calculated using ChemDraw 22.2) and a lipophilic efficiency (LipE). LipE is calculated by subtracting the logP value from the plCso value.

**Table 1**

| **Example** | **pKᵢ** | **pKᵢ Standard deviation** | **plC₅₀** | **plC₅₀ Standard deviation** | **clogP** | **LipE** |
|---|---|---|---|---|---|---|
| LEI-515 (comparative) | 9.78 | 0.04 | 6.79 | 0.10 | 5.49 | 4.29 |
| 2 | 8.43 | 0.07 | 8.11 | 0.04 | 3.26 | 5.17 |
| 3 | 8.89 | 0.04 | 7.99 | 0.05 | 3.28 | 5.61 |
| 4 | 8.78 | 0.08 | 7.98 | 0.07 | 2.74 | 6.04 |
| 5 | 8.81 | 0.05 | 7.65 | 0.09 | 2.76 | 6.05 |
| 6 | 8.58 | 0.08 | 7.44 | 0.07 | 2.53 | 6.05 |
| 7 | 8.24 | 0.06 | 7.24 | 0.07 | 2.55 | 5.69 |
| 8 | 8.66 | 0.09 | 7.30 | 0.07 | 2.22 | 6.44 |
| 9 | 8.21 | 0.07 | 6.69 | 0.06 | 2.24 | 5.97 |
| 10 | 8.28 | 0.15 | 7.27 | 0.03 | 2.71 | 5.57 |
| 11 | 8.64 | 0.05 | 7.21 | 0.08 | 2.17 | 6.47 |
| 12 | 8.74 | 0.10 | 6.90 | 0.05 | 2.19 | 6.55 |
| 13 | 8.40 | 0.08 | 6.74 | 0.05 | 1.65 | 6.75 |
| 14 | 8.40 | 0.09 | 7.95 | 0.05 | 2.36 | 6.04 |
| 15 | 8.27 | 0.08 | 6.58 | 0.07 | 2.38 | 5.89 |
| 16 | 7.78 | 0.07 | 6.67 | 0.07 | 1.84 | 5.94 |
| 17 | 7.29 | 0.11 | 6.17 | 0.09 | 1.86 | 5.43 |
| 18 | 7.53 | 0.07 | 6.22 | 0.07 | 1.32 | 6.21 |
| 19 | 8.24 | 0.07 | - | - | 2.9 | 5.30 |

As indicated in Table 1, the compounds of the present invention achieve improved LipE compared to LEI-515, as well as comparable or better cellular activity (pICso). A decrease in logP was also achieved for all of the exemplified compounds relative to LEI-515.

### Targeted lipidomics

Compounds of the examples were tested in a lipidomics assessment by incubating each compound with U-87 MG cells. Subsequently, the lipid content was extracted and measured by LC-MS/MS, and the levels of 2-AG, arachidonic acid (AA) and anandamide (AEA) were quantified. The results of this assessment are depicted in Figure 1.

As indicated in Figure 1, for the compounds of the invention, an increase in 2-AG levels (Fig. 1A) and a decrease in AA levels (Fig. 1B) was observed relative to LEI-515. No notable change in AEA levels was observed.

### Selectivity Evaluation

The selectivity of compounds of the invention was assessed using competitive activity-based protein profiling (ABPP). Gel-based ABPP, using activity-based probes (ABPs) FP-Bodipy and MB064, revealed that **LEI-515** and compounds of the invention prevented the labelling of MAGL in mouse brain proteome (Figures 2A and 2B). Other serine hydrolases visualized by the broad spectrum ABPs were not inhibited by the compounds.

However, hormone-sensitive lipase (HSL) - which was previously confirmed as the main off-target of **LEI-515** in lung, liver and brain lysate (Jiang, M. et al., Nat. Commun. 14, 8039 (2023)) - was not inhibited by compounds of the invention in lysates of HEK293T cells overexpressing recombinant mouse HSL (Figure 2C). The gel-based ABPP findings were supported by chemical proteomics experiments using mouse liver lysates and the ABPs FP-biotin and MB108 (Figure 3). Accordingly, the compounds of the invention achieved improved MAGL selectivity relative to LEI-515, inhibiting only MAGL with no off-target inhibition detected in the pool of 62 identified serine hydrolases (Figure 3A and 3B). Without wishing to be bound by theory, it is thought that the reduced lipophilicity in the compounds of the invention gives rise to their enhanced selectivity.

## Claims

1. A compound of formula (I), or a pharmaceutically acceptable salt or solvate thereof 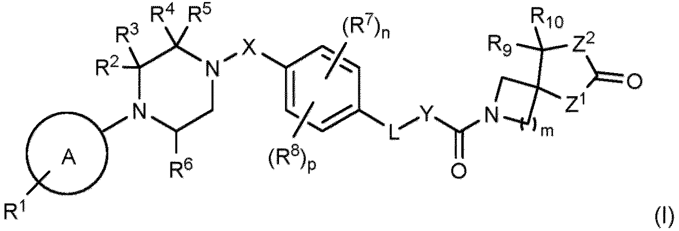 wherein
A is a 6-membered aromatic ring, or a 5- or 6-membered heteroaromatic ring optionally comprising at least one N atom, wherein the 6-membered aromatic ring of the 5- or 6-membered heteroaromatic ring is optionally fused to a 5- or -6 membered heterocycloalkyl ring or a C₅ or C₆ cycloalkyl;
X is selected from -C(O)- and -C(R^{a}R^{b})-;
L is selected from S(=O)-, -SO₂-, -S-, -O-, -C(O)-, -CH₂-, -C(R^{c}R^{d})-;
Y is -CH₂-, -CF₂-, -C(R^{c}R^{d})-;
Z' is -NR^{e}-;
Z² is selected from -CH₂-, -O-CH₂-, and -O-;
R¹ is selected from H, halo, C₁₋₄ alkyl, C₁₋₄ haloalkyl, C₁₋₄ alkoxy, NO₂, CN, substituted or unsubstituted aryl, substituted or unsubstituted heteroaryl, O-(C₁₋₄ alkyl)ₐ-aryl, or O-(C₁₋₄ alkyl)_{b}-heteroaryl;
each R² and R⁴ is independently selected from H and C₁₋₄ alkyl;
each R³, R⁵ and R⁶ is independently selected from H and C₁₋₄ alkyl; or R³ and R⁶ together form an alkyl or heteroalkyl bridge and R⁵ is selected from H and C₁₋₄ alkyl;
each R⁷ and R⁸ is independently selected from H, halo, C₁₋₄ alkyl, C₁₋₄ haloalkyl, C₁₋₄ alkoxy, CN and NO₂;
R⁹ is selected from H, C₁₋₆ haloalkyl, OR¹¹, C₁₋₆ alkyl, and C₂₋₈ alkyl interrupted by 1, 2 or 3 ether linkages, -(C₂₋₄ haloalkyl)-OR¹¹, and -(C₁₋₆ haloalkyl)-R¹¹;
R¹⁰ is selected from H and C₁₋₆ alkyl;
R¹¹ is selected from C₁₋₆ alkyl, C₂₋₈ alkyl interrupted by 1, 2 or 3 ether linkages, C₃₋₈ cycloalkyl, C₆ aryl, 5- or 6-membered heteroaryl, and 3- to 7-membered heterocycloalkyl;
wherein each R⁹, R¹⁰ and R¹¹ is optionally independently substituted where chemically possible with one, two or three groups independently selected at each occurrence from: H, OH, =O, CN, halo, C₁₋₄ haloalkyl, C₁₋₄ alkyl, C₁₋₄ alkoxy, OCF₃, C₆ aryl, and C₅₋₆ heteroaryl;
R^{a} and R^{b} are independently selected from H, and C₁₋₄ alkyl; or wherein R^{a} and R^{b} together form a 3- to 6-membered cycloalkyl or heterocycloalkyl ring system;
R^{c} and R^{d} are independently selected at each occurrence from H, F, C₁₋₄ alkyl, and C₁₋₄ haloalkyl; or R^{c} and R^{d} together form a 3- to 6-membered cycloalkyl or heterocycloalkyl ring system;
R^{e} is selected from H and C₁₋₄ alkyl;
m is selected from 1, or 2;
n is selected from 0, 1, or 2; and
p is selected from 0, 1, or 2.

2. The compound of claim 1, wherein Z' is -NH-; and/or
wherein Z² is -CH₂- or -O-; and/or
wherein m is 1.

3. The compound of any of claims 1 to 4, wherein the compound is a compound of formula (II) or a pharmaceutically acceptable salt or solvate thereof: 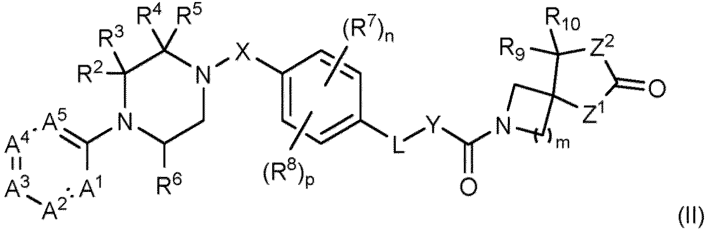
wherein A¹, A³, A⁴ and A⁵ are each independently selected from CH, CR¹² or N, and A² is CR¹; or A¹, A⁴ and A⁵ are each independently selected from CH, CR¹² or N, and A² and A³ together form a 5- or 6-membered heterocycloalkyl ring;
wherein each R¹² is independently selected from H, halo, CN, OCF₃, C₁₋₄ alkyl, C₁₋₄ haloalkyl, and C₁₋₄ alkoxy.

4. The compound of any preceding claim, wherein X is -C(O)-; and/or
wherein Y is -CH₂-; and/or
wherein L is selected from -S(=O)- and -O-, optionally wherein L is -S(=O)-.

5. The compound of any preceding claim, wherein R¹ is selected from H, halo, C₁₋₄ alkyl, C₁₋₄ haloalkyl, or O-(C₁₋₄ alkyl)ₐ-aryl; optionally wherein R¹ is selected from H, halo, C₁₋₄ alkyl, C₁₋₄ haloalkyl, or O-(C₁₋₄ alkyl)ₐ-aryl; further optionally wherein R¹ is halo; and/or
wherein at least one of R² and R⁴ is C₁₋₄ alkyl (e.g. CH₃); optionally wherein both of R² and R⁴ are C₁₋₄ alkyl (e.g. CH₃); and/or
wherein R² and R⁴ are arranged *trans* to one another; and/or
wherein R⁹ is selected from H, or C₁₋₆ alkyl.

6. The compound of any preceding claim, wherein a is selected from 1 and 2; optionally wherein a is 1; and/or
wherein b is selected from 1 and 2; optionally wherein b is 1.

7. The compound of any preceding claim, wherein each of R³, R⁵ and R⁶ may be independently selected from H and C₁₋₄ alkyl; optionally wherein each of R³, R⁵ and R⁶ may be independently selected from H and CH₃; and/or
wherein each R⁷ and R⁸ may be independently selected from H, halo, C₁₋₄ haloalkyl, and NO₂; optionally wherein each R⁷ is H and each R⁸ is independently selected from halo, C₁₋₄ haloalkyl, and NO₂.

8. The compound of any preceding claim, wherein p is 0 or 1; and/or
wherein n is 0 or 1, optionally wherein n is 1.

9. The compound of claim 1, wherein the compound is selected from: 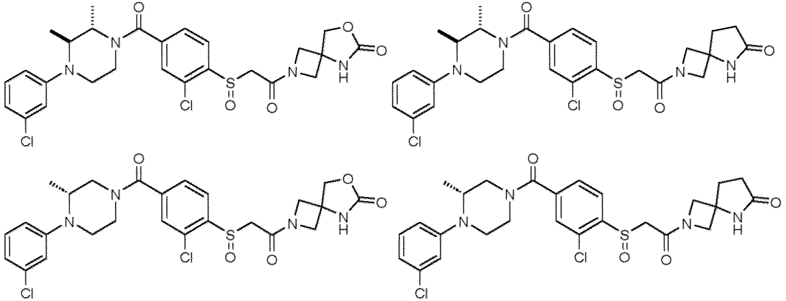 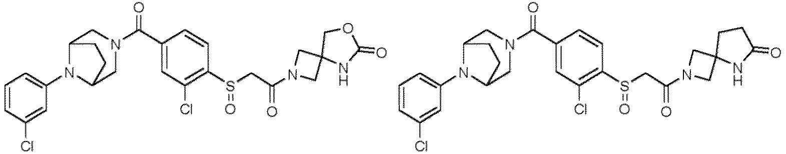 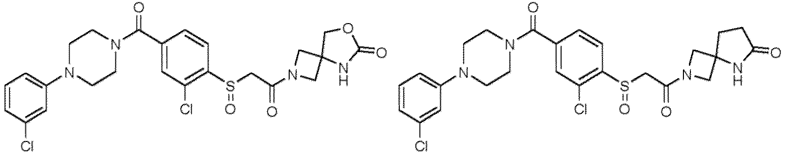 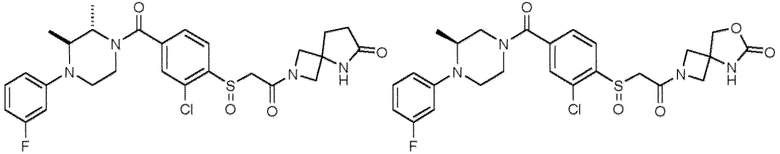 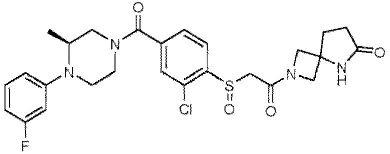 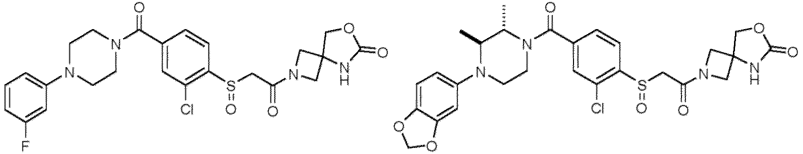 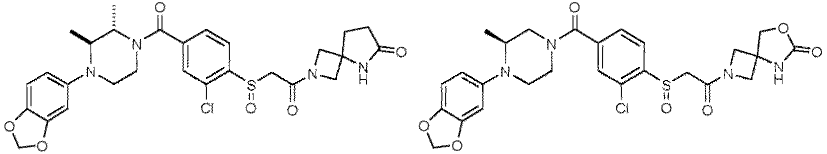 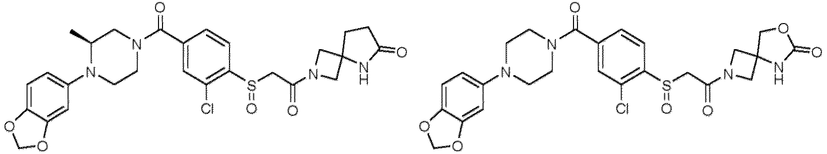 and

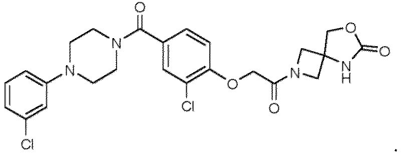

10. A pharmaceutical composition comprising a compound of any preceding claim;
optionally further comprising a pharmaceutically acceptable excipient.

11. The compound of any of claims 1 to 9 or the pharmaceutical composition of claim 10 for use as a medicament.

12. The compound of any of claims 1 to 9 or the pharmaceutical composition of claim 10 for use in the treatment of a condition which is modulated by monoacylglycerol lipase (MAGL).

13. The compound of any of claims 1 to 9 or the pharmaceutical composition of claim 10 for use in the treatment of a condition selected from acute pain, inflammatory pain, cancer pain, pain caused by peripheral neuropathy, central pain, incisional pain, fibromyalgia, migraine, chemotherapy-induced neuropathy, spinal nerve injury, vaso-occlusive painful crises in sickle cell disease, spasticity or pain associated with multiple sclerosis, functional chest pain, abdominal pain associated with irritable bowel syndrome, rheumatoid arthritis, osteoarthritis, nausea, fever, neuroinflammation, Tourette syndrome, Parkinson's disease, Alzheimer's disease (AD), multiple sclerosis, amyotrophic lateral sclerosis, traumatic brain injury, neurotoxicity, stroke, epilepsy, neuromyelitis optica, anxiety, cancer, inflammatory hypersensitivity conditions, mental disorders, neuropathic cold allodynia, food consumption and/or weight gain, opioid dependency, nicotine dependency, metabolic disease, obesity, diabetes, and depression.

14. The compound for use according to claim 13, wherein the condition is cancer;
optionally wherein the condition is breast cancer, ovarian cancer, melanoma, hepatocellular carcinoma, colorectal cancer, prostate cancer, or nasopharyngeal carcinoma.

15. Use of the compound of any of claims 1 to 9 or the pharmaceutical composition of claim 10 for the inhibition of monoacylglycerol lipase (MAGL).
